# EUROPEAN PATENT APPLICATION

(11) **EP 4 503 048 A1**
(43) Date of publication of application: **05.02.2025**
(21) Application number: 24190069.5
(22) Date of filing: 22.07.2024
(51) Int. Cl.: G16H 20/70, G16H 40/63

(54) **PRESENTING MULTIMODAL, MULTISTABLE STIMULI TO REDUCE SYMPTOMS ASSOCIATED WITH ATTENTION BIASES DUE TO IMPAIRMENT OF COGNITIVE FUNCTIONS IN USERS**

(30) Priority: 03.08.2023 US 202363530640 P; 06.11.2023 US 202363547456 P; 27.06.2024 US 202418757253
(71) Applicant: Click Therapeutics, Inc., New York, NY 10013 (US)
(72) Inventor: Mursky-Fuller, Jesse, New York, 10013 (US); Adler, Samantha, New York, 10013 (US)
(74) Representative: Marks & Clerk LLP

(57) **Abstract**

Provided herein are systems and methods for presenting multimodal stimulus to address symptoms associated with conditions. A server may identify a first visual stimulus associated with a condition and a second visual stimulus. The server may present the first visual stimulus at least partially overlapped with the second visual stimulus, to direct the user to interact with the display. The service may detect a response identifying an interaction associated with one of the first or second visual stimulus. The server may generate an auditory stimulus to include one or more portions indicating feedback based on the response.

## Description

### CROSS REFERENCE TO RELATED APPLICATIONS

The present application claims priority to U.S. Non-Provisional Application No. 18/757,253, filed June 27, 2024, which claims priority to U.S. Provisional Application Nos. 63/547,456, filed November 6, 2023 and 63/530,640, filed August 3, 2023, each of which are incorporated herein by reference in their entireties.

### BACKGROUND

Certain conditions may cause a subject to direct attention to certain biases which may ultimately exacerbate their symptoms. Subjects may simultaneously suffer from chronic pain, fear, and mood symptoms, such as in the case of patients experiencing multiple sclerosis (MS) or cancer. The desire to give attention to pain, fear, or other biases is innate, and further, subjects can become hyper-aware of biases corresponding to related stimuli. For example, a subject experiencing MS may pay more attention to pain-related stimuli than a patient without chronic pain. In a similar manner, hypersensitivity to negative or threat-related stimuli can exacerbate fear in a subject. For example, a patient experiencing hypersensitivity may seek out signs of threat within a social interaction, unlike a patient without such conditions. This attention to negative stimuli can cause worsened symptoms for the patient. For example, a bias towards pain can lead towards hypersensitization for the patient. Furthermore, these hypersensitivities can worsen the condition through fear-avoidance. In fear avoidance, individuals can avoid stimuli or activities which are perceived to potentially cause pain or threat, further weakening physical or mental conditions from the lack of activity.

Treating attentional biases in patients with chronic conditions can prove difficult due to the general reduction in cognitive function often caused by these conditions. The therapeutic exercises to mitigate the effects of these biases may reduce the severity of symptoms. Certain behavioral therapies may aid an individual in reducing or removing bias to direct attention towards or away from certain stimuli. Access to these behavioral therapies, however, can be difficult to obtain, especially for a patient experiencing a chronic condition such as MS or cancer. Furthermore, the therapies may not prove useful for the individual without consistent adherence, which may be difficult to guarantee due to the nature of pain and fear. Lastly, it can be difficult to ascertain which therapies would be the most beneficial to an individual, or if a combination of therapies would be the most beneficial.

Addressing such attentional biases related to chronic conditions in subjects digitally through a device to present these therapeutic exercises can present a multitude of problems. For one, the user may be unable or have extreme difficulty refraining from paying mind to negative stimuli, thereby ignoring any attempts at intervention from a clinician or through a device. The user may thus find it difficult to adhere to treatments through digital means due to the nature of the chronic condition, leading to ineffective clinical outcomes. For another, when the subject is experiencing an instance of pain, it may be physically difficult for the subject to access the device to access digital therapeutic treatment, let alone contact a clinician to receive treatment.

### SUMMARY

To resolve these and other technical challenges, digital therapeutic treatment using a multimodal, multistable modification of biases (or multimodal, multistable bias modification or MMBM) may be provided to users who have symptoms associated with an attention bias caused by a condition that is affecting the user's cognitive functions. By linking two different senses (e.g., visual multistable perception using a set of visual stimuli together with auditory multistable perception using auditory stimuli for multimodal perception), the resulting synergy in this multimodal multistable approach can provide enhanced training to the user to re-orient attention towards positive stimuli and resolve symptoms related to the attention biases of the user. In addition, the digital therapeutic treatment presented herein may provide an auditory stimulus (e.g., binaural beats) to address auxiliary symptoms (e.g., fatigue) related to the user's condition. In this manner, the user can be conditioned to pay less mind to negative stimuli, such as pain- or stress-related stimuli, at a speed, frequency, and efficacy available through the digital therapeutic application.

Through a multimodal multistable perception modification approach including visual and auditory stimuli, the user's ability to redirect attention from negative stimuli may be increased. Controlling the bias towards negative stimuli can be a facet of remediating or resolving symptoms of a condition such as MS or cancer. A multimodal approach can refer to therapy which applies to two or more of the user's senses, such as hearing and sight. Multistable perception can refer to the superimposing of two media to be detected by the same sense of a user. For example, multistable perception can include two images presented to a user, each image eliciting the user's attention or standing out individually to the user at different times of the user's viewing of the images.

Visual multistable perception can include monocular rivalry and binocular rivalry. During monocular rivalry, a different image (or word) can be presented to each eye of the subject. The subject then alters her view between the image presented to the left eye and the image presented to the right eye. In binocular rivalry, the two or more images can be superimposed on one another in such a way that the subject is able to discern either image at different times using both eyes. As a therapeutic approach, two stimuli presented as images or words can be presented to a subject. The first stimulus can be a negative stimulus, or a stimulus associated with the condition. For example, the first stimulus can include the words "pain," "disease," or "tired." The second stimulus can be a positive or neutral stimulus unassociated with the condition. For example, the second stimulus can include the words "love," "good," or "happy." The two stimuli can be presented to the subject as a superimposed image. Through cognitive bias training, the subject can be trained to see more clearly, pay more attention to, and otherwise be more inclined to notice the positive or neutral stimulus over the negative stimulus.

In addition, binaural beats are a unique type of auditory multistable perception for this approach. The system can present binaural beats as a combination of two or more different tones or frequencies of sound. A binaural beat can include a first sound presented to the first ear of a subject and a second sound concurrently presented to the second ear of the subject. For example, presenting a frequency of 415 Hz to the left ear of a patient while simultaneously presenting a frequency of 400 Hz to the right ear of a patient can cause the patient to hear a "beat" of 15 Hz. In some cases, this presentation of a beat can elicit a beneficial therapeutic response.

By using a combination of binaural beats and binocular rivalry, the user's ability to resist a bias towards negative stimuli may be synergistically increased through a multimodal multistable modification of biases. Resisting the bias towards negative stimuli can be a facet of remediating or resolving symptoms of a chronic condition such as MS or an oncological condition, as examples. The multimodal multistable modification of biases can include interventions such as binaural beats, in which the user receives two or more different but similar tones in each ear. Binaural beats can modulate brain activity based on the different frequency beat provided. For example, beats in the range of 14-30 Hz have been shown to modulate brain activity related to concentration, alertness, and cognition. Therefore, providing a tone in the left ear of a patient of 415 Hz and a tone in the right ear of a patient of 400 Hz can create a beat of 15 Hz which may stimulate brain activity related to concentration, alertness, and cognition.

The user may adapt to pay less attention to negative biases related to pain, anxiety, or depressed mood, among others. Performing an action using binocular rivalry can condition the user to focus on a positive stimulus or neutral stimulus over a negative stimulus. By presenting two superimposed images, the user can perform a task such as selecting the image associated with the positive or neutral stimulus. Upon choosing the correct (e.g., positive or neutral) stimulus, the user can receive positive feedback to bias the user towards a positive or neutral stimulus and away from the negative stimulus. Through this method, the user can be trained to focus on the image associated with the positive or neutral stimulus.

A combination of these therapies can greatly reduce the bias towards negative stimuli. Combining these therapies can create a multimodal enrichment which better approximates a real-world environment due to integrating multiple types of sensory information, such as visual and audio. As an illustrative example, the combination can include the service providing a game to the user through the user's mobile device. The service may also be coupled with a set of loudspeakers (e.g., headphones or earphones) to play a tone for each ear. The service may present two overlaid words to the user on a screen of a mobile device. The user may select the word depicting a positive or neutral stimulus. This illustrative example shows binocular rivalry to promote the user focusing on positive or neutral stimulus over a negative stimulus.

Together with the presentation of the visual stimuli, the service may provide one or more different tones to each ear. Each tone of an ear may have a counterpart tone presented in the other ear. The tone and the counterpart tone may each be a different frequency such that the difference of frequencies creates a beat. The beat may modulate brain activity to treat several impairments due to chronic conditions, such as fatigue, low cognitive acuity, anxiety, or pain perception, among others. This multimodal approach can lead to crossmodal interactions between each stimuli's relevant brain regions. In this manner, each brain region can be cross activated upon subsequent unisensory stimuli. Furthermore, this approach can increase functional connectivity of the sensory-specific regions. This combination of therapies provided by the service can reduce symptomology of chronic conditions.

To provide these therapies, the service may select two or more visual stimuli including at least two words or images and associated actions for the user to transmit to the end user device. The service may select a stimulus including at least two tones to be played to the user via two or more loudspeakers. The service may also time the delivery or presentation of the stimuli to the user to avoid the user from having to actively access the digital therapeutics application on their device while suffering from the chronic condition. The service may time the delivery or presentation of the stimuli to the user subsequent to the completion of a prior action related to the stimuli. Furthermore, as the service acquires additional data about the user, the service may be able to select stimuli more targeted toward the specific user and their condition and may store this data in a profile of the user. The service may receive preferences from the user, such as a preferred list of words or preferred tones to hear. The service may select a subsequent stimulus based on at least the prior stimuli, the completion of the prior action, the profile of the user, or an evaluation of the user's performance with prior stimuli, among others.

In this manner, the user can receive stimuli relating to the chronic condition with ease to help retrain a bias towards negative stimuli relating to the condition as documented herein. This may reduce or eliminate barriers to the user from physically accessing their device while combating the chronic condition. By selecting the stimuli sent to the user to address the subject's bias towards negative stimuli, the quality of a human computer interactions (HCI) between the user and the device may be improved. In addition, since the stimuli are more related to the user's condition, unnecessary consumption of computational resources (e.g., processing and memory) of the service and the user device and the network bandwidth may be reduced, relative to sending ineffective messages. Furthermore, in the context of a digital therapeutics application, the individualized selection of such stimuli may result in the delivery of user-specific interventions to improve subject's adherence to the treatment. This may result in not only higher adherence to the therapeutic interventions but also potential improvements to the subject's bias towards negative stimuli.

Since the digital therapeutics application operates on the subject's device, or at least a device that the user can access easily and reliably (e.g., according to the predetermined frequency such as once per day), the application can provide real-time support to the subject. For example, upon receiving a request from the user to initiate a session, the application initiates a session in near-real time. Such prompt guidance cannot be achieved via in-person visits, phone calls, video conferences or even text messages between the user and health care providers examining the user for multiple sclerosis or cancer. Due to this accessibility, the application is able to provide and customize tasks for the user based on the performance of the user. This can create an iteratively improving service for the user wherein overall bandwidth and data communications are minimized due to the increasing usefulness of each session.

Aspects of the present disclosure relate to systems and methods for presenting multimodal stimuli to address symptoms associated with conditions. The system may include a computing system having one or more processors coupled with memory. The computing system may identify, for addressing a condition of a user, (i) a first visual stimulus associated with the condition and (ii) a second visual stimulus. The computing system may present, via a display, the first visual stimulus at least partially overlapped with the second visual stimulus, to direct the user to interact with the display. The system may detect a response identifying an interaction. The interaction may be associated with one of the first visual stimulus or the second visual stimulus. The computing system may generate an auditory stimulus to include one or more portions. The one or more portions may indicate feedback based on the response. The computing system may play, via a transducer, the auditory stimulus to provide the feedback on the response to the user.

In some embodiments, the computing system may determine that the response is correct based on the interaction associated with the second visual stimulus. The second visual stimulus may disassociate the user from the condition. The computing system may add at least one portion to the auditory stimulus to provide positive reinforcement, responsive to determining that the response is correct. In some embodiments, the computing system may determine that the response is incorrect based on the interaction associated with the first visual stimulus associated with the condition. The computing system may remove at least one portion from the auditory stimulus to provide negative punishment, responsive to determining that the response is incorrect. In some embodiments, the computing system may assign (i) the first visual stimulus to a first type of interaction and (ii) the second visual stimulus to a second type of interaction. The computing system may present (i) the first visual stimulus with a first indicator to identify the first type of interaction and (ii) the second visual stimulus with a second indicator to identify the second type of interaction.

In some embodiments, the computing system may select, based on a rate of correct responses from previous presentations of visual stimuli, a difficulty level from a set of difficulty levels. Each of the set of difficulty levels may define one or more visual characteristics. The computing system may modify a presentation of the first visual stimulus and the second visual stimulus using the one or more visual characteristics in accordance with the difficulty level. In some embodiments, the computing system may generate the auditory stimulus to include (i) a first element to be played to a first ear of the user and (ii) a second element to be played to a second ear of the user. The computing system may provide the first element to the first ear of the user via a first loudspeaker and the second element to the second ear via a second loudspeaker. In some embodiments, the interaction may include an orientation of the display toward a side corresponding to one of a first side associated with the first visual stimulus or a second side associated with the second visual stimulus.

In some embodiments, the computing system may modify a visual characteristic of one of the first visual stimulus or the second visual stimulus based on the response. In some embodiments, the computing system may play at one portion of the one or more portions of the auditory stimulus. The computing system may play the one or more portions of the auditory stimulus at least in partial concurrence with the presentation of the first visual stimulus and the second visual stimulus. In some embodiments, the computing system may receive, via an interface, a selection by the user of at least one property from a set of properties for auditory stimuli. The computing system may generate the auditory stimulus in accordance with the at least one property. In some embodiments, the computing system may receive, via an interface, a list identifying a plurality of words comprising a first subset of words related to the condition and a second subset of words. The computing system may select the first visual stimulus from the first subset of words and the second visual stimulus from the second subset of words. In some embodiments, the user may be on a medication to address the condition associated with at least one of cancer or multiple sclerosis, at least in partial concurrence with the session.

Aspects of the present disclosure relate to systems and methods for reducing a symptom associated with an attention bias of a user suffering from a condition affecting a cognitive function in need thereof. A computing system may obtain a first metric associated with the user prior to a plurality of sessions. The computing system may repeat, during each session of the plurality of sessions, (i) presentation, via a display to a user, of a respective set of visual stimuli comprising (a) a first visual stimulus associated with the symptom and (b) a second visual stimulus at least partially overlapped with the first visual stimulus, to direct the user to perform an interaction, and (ii) playing, via a transducer to the user, of a respective auditory stimulus to provide feedback based on the interaction with one of the respective set of visual stimuli via the display. The computing system may obtain a second metric associated with the user subsequent to at least one of the plurality of sessions. A reduction in the symptom associated with the attention bias may occur in the user, when the second metric is (i) decreased from the first metric by a first predetermined margin or (ii) increased from the first metric by a second predetermined margin. In some embodiments, the attention bias may include one or more of (i) avoidance of stimuli or an activity related to the symptom; (ii) anxiety induced by stimuli associated with the symptom, multiple sclerosis, or cancer; or (iii) depressed mood.

In some embodiments, the condition of the user may include multiple sclerosis (MS) with or without mobility limitations. In some embodiments, the reduction in the symptom may occur when the second metric increased from the first metric by the second predetermined margin, and the first metric and the second metrics may be Symbol Digit Modalities Test (SDMT) values. In some embodiments, the user may be in relapse and steroid-free for at least a predefined period of time prior to the plurality of sessions. In some embodiments, the user may be on medication, at least in partial concurrence with the plurality of sessions, and the medication may include at least one of beta interferons, glatiramer, cladribine, dimethyl fumarate, diroximel fumarate, fingolimod, monomethyl fumarate, ofatumumab, ozanimod, ponesimod, siponimod, teriflunomide, alemtuzumab, mitoxantrone, or natalizumab.

In some embodiments, the condition of the user may include at least one of lung cancer, colorectal cancer, skin cancer, breast cancer, ovarian cancer, leukemia, pancreatic cancer, or gastric cancer. In some embodiments, the reduction in the symptom may occur when the second metric is increased from the first metric by the second predetermined margin, and the first metric and the second metric may be functional assessment of cancer therapy-cognitive function (FACT-Cog) values. In some embodiments, the user may have completed cancer chemotherapy within a predefined period of time prior to the plurality of sessions.

In some embodiments, the reduction in the symptom may occur when the second metric is decreased from the first metric by the first predetermined margin, and the first metric and the second metric may be patient reported outcomes measurement information system (PROMIS) values. In some embodiments, the reduction in the symptom may occur when the second metric is decreased from the first metric by the first predetermined margin, and the first metric and the second metric may be brief pain inventory (BPI) values. In some embodiments, the reduction in the symptom may occur when the second metric is decreased from the first metric by the first predetermined margin, and the first metric and the second metric may be pain catastrophizing scale (PCS) scores. In some embodiments, the first metric and the second metric may be computerized cognitive assessment values.

In some embodiments, the user may experience an improvement in fatigue associated with the condition, in response to binaural beats of the respective auditory stimulus in at least one of the plurality of sessions. In some embodiments, the respective set of visual stimuli for each session of the plurality of sessions may be selected from a plurality of visual stimuli comprising (i) a pain-related visual stimulus, (ii) a threat-related visual stimulus, (iii) a condition-related visual stimulus, (iv) a negative visual stimulus, or (v) a neutral visual stimulus.

In some embodiments, in at least one of the plurality of sessions, the respective auditory stimulus may be played to provide positive reinforcement when the interaction with the respective set of visual stimuli is correct. In some embodiments, in at least one of the plurality of sessions, the respective auditory stimulus may be played to provide negative punishment, when the interaction with the respective set of visual stimuli is incorrect. In some embodiments, in at least one of the plurality of sessions, the presentation of the respective visual stimuli may be at least partially concurrent with the playing of a portion of the respective auditory stimulus.

In some embodiments, in at least one of the plurality of sessions, the presentation of the respective set of visual stimuli may be modified in accordance with a difficulty level selected based on a response in a prior session. In some embodiments, the interaction comprises an orientation of the display toward a side corresponding to one of a first side associated with the first visual stimulus or a second side associated with the second visual stimulus.

### BRIEF DESCRIPTION OF THE DRAWINGS

The foregoing and other objects, aspects, features, and advantages of the disclosure will become more apparent and better understood by referring to the following description taken in conjunction with the accompanying drawings, in which:
FIG. 1 depicts a block diagram of a system for presenting multimodal stimuli to address symptoms associated with conditions in accordance with an illustrative embodiment;
FIG. 2 depicts a block diagram for a process to present the stimuli and detect a response identifying an interaction associated with the stimuli in accordance with an illustrative embodiment;
FIG. 3 depicts a block diagram for a process to generate an auditory stimulus and to play the auditory stimulus to provide feedback in accordance with an illustrative embodiment;
FIG. 4 depicts a flow diagram of a method for presenting multimodal stimuli to address symptoms associated with conditions in accordance with an illustrative embodiment;
FIG. 5 depicts a screenshot of an example user interface for a system for presenting multimodal stimuli to address symptoms associated with conditions in accordance with an illustrative embodiment;
FIG. 6 depicts a screenshot of an example user interface for a system for presenting multimodal stimuli to address symptoms associated with conditions in accordance with an illustrative embodiment;
FIG. 7 depicts a flow diagram of a method of reducing a symptom as associated with an attention bias of a user suffering from a condition affecting a cognitive function in need thereof in accordance with an illustrative embodiment;
FIG. 8 depicts a timeline of a randomized, controlled, exploratory basket study to evaluate multimodal multistable modification of biases for the normalization of attentional biases and improving cognition in adults with multiple sclerosis, breast cancer, and lung cancer in accordance with an illustrative embodiment;
FIG. 9 is a block diagram of a server system and a client computer system in accordance with an illustrative embodiment;
FIG. 10 depicts the results of the PROMIS-fatigue study. In PROMIS-Fatigue, a lower score is improvement (the patient is experiencing less fatigue). Clinically important change: 2 to 6 points;
FIG. 11 depicts the results of the PROMIS-depression study. In PROMIS-Depression, a lower score is improvement (the patient is experiencing less depression). Clinically important change: 2 to 6 points;
FIG. 12 depicts results of the PROMIS-anxiety study. In PROMIS-Anxiety, a lower score is improvement (the patient is experiencing less anxiety). Clinically important change: 2 to 6 points;
FIG. 13 depicts results of the PROMIS-pain interference study. In PROMIS-Pain Interference, a lower score is improvement (the patient is experiencing less pain interference). Clinically important change: 2 to 6 points;
FIG. 14 depicts results of the PROMIS-pain score study. In PROMIS-Pain Interference, a lower score is improvement (the patient is experiencing less pain interference). Clinically important change: >2;
FIG. 15 depicts results of the PROMIS-sleep disturbance study. In PROMIS-Sleep Disturbance, a lower score is improvement (the patient is experiencing less sleep disturbance). Clinically important change: 2 to 6 points;
FIG. 16 depicts results of the PROMIS-physical functioning study. In PROMIS-Physical Functioning, a higher score is improvement (the patient is experiencing better physical functioning). Clinically important change: 2 to 6 points;
FIG. 17 depicts results of the PROMIS-cognitive functioning study. In PROMIS-Cognitive Functioning, a higher score is improvement (the patient is experiencing better cognitive functioning). Clinically important change: 2 to 6 points;
FIG. 18 depicts results of the PROMIS-social functioning study. In PROMIS-Social Functioning, a higher score is improvement (the patient is experiencing better social functioning). Clinically important change: 2 to 6 points;
FIG. 19 depicts results of the brief pain inventory study for interference. Lower scores represent improvement (less pain interference). Clinically important change: ≥1 point;
FIG. 20 depicts results of the brief pain inventory study for worst pain severity. Lower scores represent improvement (less pain). Clinically important change: ≥1 point;
FIG. 21 depicts results of the brief pain inventory study for least pain severity. Lower scores represent improvement (less pain). Clinically important change: ≥1 point;
FIG. 22 depicts results of the brief pain inventory study for average pain severity. Lower scores represent improvement (less pain). Clinically important change: ≥1 point;
FIG. 23 depicts results of the brief pain inventory study for current pain severity. Lower scores represent improvement (less pain). Clinically important change: ≥1 point;
FIG. 24 depicts results of the pain catastrophizing scale study. Lower number = less pain catastrophizing (better condition). Minimum detectable change: 9.1 points;
FIG. 25 depicts results for patient reported outcomes for cognition in MS. Higher scores represent better cognitive functioning. Clinically important change: 4 points;
FIG. 26 depicts results for the global rating of change (GRC) between DiNaMo and control;
FIG. 27 depicts results of the attention bias index study for depression. Attention Bias Index: Goal is to approach 0 (no bias) or lower (biased away from triggering stimuli);
FIG. 28 depicts results of the attention bias index study for anxiety. Attention Bias Index: Goal is to approach 0 (no bias) or lower (biased away from triggering stimuli); and

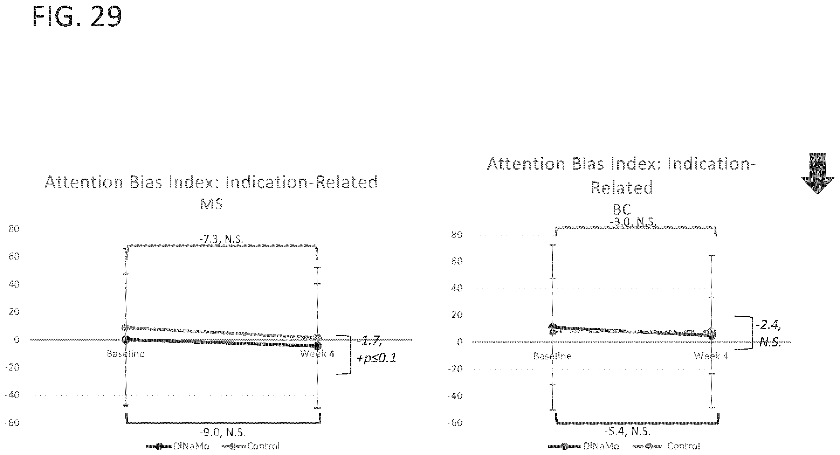

FIG. 29 depicts results of the attention bias index study for indication. Attention Bias Index: Goal is to approach 0 (no bias) or lower (biased away from triggering stimuli).

### DETAILED DESCRIPTION

For purposes of reading the description of the various embodiments below, the following enumeration of the sections of the specification and their respective contents may be helpful:
Section A describes systems and methods for presenting multimodal stimuli to address symptoms associated with conditions;
Section B describes methods of reducing symptoms associated with attention bias of users suffering from conditions affecting cognitive function in need thereof; and
Section C describes a network and computing environment which may be useful for practicing embodiments described herein.

### A. Systems and Methods for Presenting Multimodal Stimuli to Address Symptoms Associated with Conditions

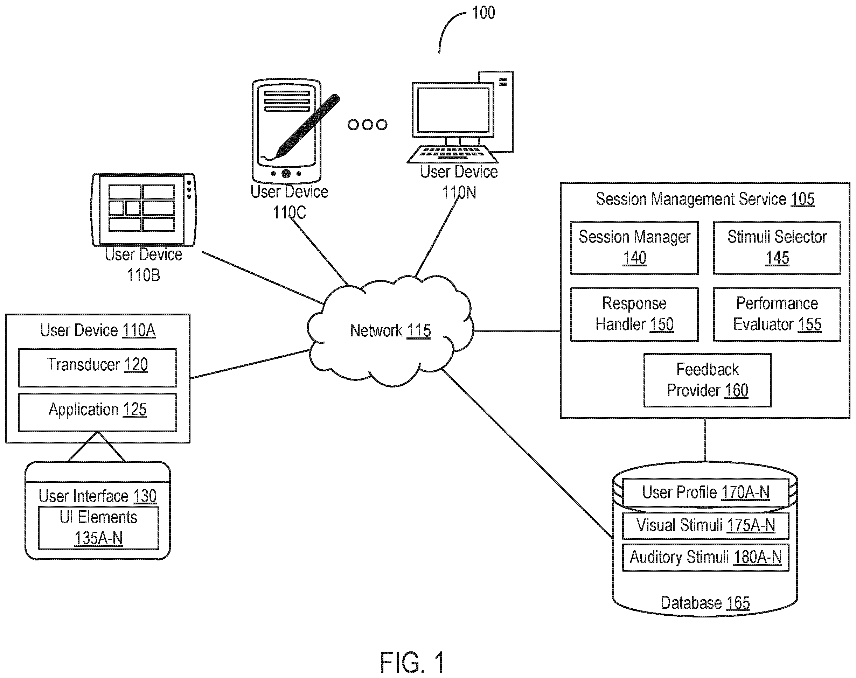

Referring now to FIG. 1, depicted is a block diagram of a system 100 for presenting multimodal stimuli to address symptoms associated with conditions. In an overview, the system 100 may include at least one session management service 105 and a set of user devices 1 10A-N (hereinafter generally referred to as user devices 110 or client devices), communicatively coupled with one another via at least one network 115. At least one user device 110 (e.g., the first user device 110A as depicted) may include at least one transducer 120 and at least one application 125. The application 125 may include or provide at least one user interface 130 with one or more user interface (UI) elements 135A-N (hereinafter generally referred to as UI elements 135). The session management service 105 may include at least one session manager 140, one stimuli selector 145, one response handler 150, one performance evaluator 155, or at least one feedback provider 160, among others. The session management service 105 may include or have access to at least one database 165. The database 165 may store, maintain, or otherwise include one or more user profiles 170A-N (hereinafter generally referred to as user profiles 170), one or more visual stimuli 175A-N (hereinafter generally referred to as visual stimuli 175) or one or more auditory stimuli 180AN (hereinafter generally referred to as auditory stimuli 180), among others. The functionality of the application 125 may be performed in part on the session management service 105. The functionality of the application 125 may also incorporate operations performed on the session management service 105.

In further detail, the session management service 105 may (sometimes herein generally referred to as a computing system or a service) be any computing device comprising one or more processors coupled with memory and software and capable of performing the various processes and tasks described herein. The session management service 105 may be in communication with the one or more user devices 110 and the database 165 via the network 115. The session management service 105 may be situated, located, or otherwise associated with at least one server group. The server group may correspond to a data center, a branch office, or a site at which one or more servers corresponding to the session management service 105 is situated. The session management service 105 may be situated, located, or otherwise associated with one or more of the user devices 110. Some components of the session management service 105 may be located within the server group, and some may be located within the client device. For example, the session manager 140 may operate or be situated on the user device 110A, and the stimuli selector 145 may operate or be situated on the server group.

Within the session management service 105, the session manager 140 may identify a session including a set of visual stimuli 175 to present to a user by the application 125 on respective user devices 110. The session manager 140 may identify a first visual stimulus and a second visual stimulus for addressing a condition of the user. The stimuli selector 145 may present the first visual stimulus at least partially overlapped with the second visual stimulus to direct the user to interact with via a display, such as the user interface 130. The response handler 150 may detect a response identifying an interaction associated with the first visual stimulus or the second visual stimulus. The performance evaluator 155 may determine a score based on the response. The feedback provider 160 may generate an auditory stimulus including one or more portions to indicate feedback based on the response to the user via the transducer 120. The feedback provider 160 may generate an auditory stimulus that plays one or more portions at least partially concurrently with the presentation of the visual stimuli by the stimuli selector 145.

The user device 110 (sometimes herein referred to as an end user computing device or client device) may be any computing device comprising one or more processors coupled with memory and software and capable of performing the various processes and tasks described herein. The user device 110 may be in communication with the session management service 105 and the database 165 via the network 115. The user device 110 may be a smartphone, other mobile phone, tablet computer, wearable computing device (e.g., smart watch, eyeglasses), or laptop computer. The user device 110 may be used to access the application 125. In some embodiments, the application 125 may be downloaded and installed on the user device 110 (e.g., via a digital distribution platform). In some embodiments, the application 125 may be a web application with resources accessible via the network 115.

In some embodiments, the user device 110 may be coupled with at least one transducer 120. The transducer 120 may convert an electric signal provided by the session management service 105 into an audio signal. The transducer 120 may generate one or more audio signals for output by one or more loudspeakers coupled with the transducer 120. The transducer 120 may generate an audio signal such as a tone, beep, note, voice, or other sound for playing by the loudspeakers. The transducer 120 can generate instructions for the loudspeaker to play one or more sounds. For example, the transducer 120 may generate the audio signal in a Musical Instrument Digital Interface (MIDI) format for output by the loudspeakers. The transducer can generate instructions including a prerecorded sound for the loudspeakers to play. For example, the transducer may generate the audio signal according to a recorded audio format, such as a Moving Picture Experts Group (MPEG), Audio Layer 3 (MP3), or Waveform Audio File (WAV). The audio signal may contain instructions for playback of the sounds, such as volume, pitch, speed, tempo, compression, pan, bit rate, distortion, or timbre.

Continuing on, the transducer 120 may transmit the audio signal or instructions to one or more loudspeakers. The loudspeakers can be any device designed or configured to emit audio. The loudspeakers can include a magnet, cone, audio amplifier, or other such devices to produce mechanical vibrations to output sound from a signal. In some embodiments, the loudspeaker can be coupled with or included in the transducer 120. For example, the loudspeaker and the transducer 120 may be part of the same speakers, headphones, earbuds, or other device to transform an electrical signal to audio and produce the output audio. In some embodiments, the transducer 120 may be configured to produce two or more audio signals for two or more loudspeakers. For example, a first transducer 120 may produce a first audio signal for a first loudspeaker for playback in the left ear of a subject and a second transducer 120 may concurrently produce a second audio signal for a second loudspeaker for playback in the right ear of a subject. The transducer 120 may play multiple tones or sounds through each loudspeaker. For example, the transducer 120 may superimpose a signal of 500 Hz on a signal of 300 Hz, or the transducer may alternate the playing of the two signals, among other combinations of playback of two or more signals through one loudspeaker.

The application 125 executing on the user device 110 may be a digital therapeutics application and may provide a session (sometimes herein referred to as a therapy session) to address symptoms associated with conditions. The user of the application 125 may be suffering from or at risk of a condition. The condition may include, for example, cancer (e.g., lung cancer, colorectal cancer, skin cancer, breast cancer, ovarian cancer, leukemia, pancreatic cancer, or gastric cancer) or multiple sclerosis (e.g., with or without mobility limitations). A condition may have symptoms, such as pain, fear, or fatigue, as a result of attention bias related to the condition of cancer or multiple sclerosis.

The attention bias may include, for example, avoidance of stimuli or an activity related to the symptom; anxiety induced from stimuli associated with the symptom or the condition; or depression (or depressed mood), among others. The user may pay attention to stimuli which relates to symptoms of the condition, such as pain, fatigue, or actions which bring on symptoms, such as certain movements or behaviors. For example, the user may increase sensitivity to pain by refraining from movements that could cause pain, thereby further restricting the user and causing anxiety around the movement thought to cause pain. Other behaviors may cause or be related to a condition of the user. The application 125 may be used to present stimuli prompting the user to perform actions to reduce a bias towards negative stimulus associated with the condition of the user. The actions may be presented to the user as a result of sending a request to begin a session, detected measurements of the user received from the client device, or a scheduled time or period, among others.

The user may be at least partially concurrently taking an effective amount of a medication to address the condition, at least partially concurrent with the sessions through the application 125. The medication may be at least orally administered, intravenously administered, or topically applied. For example, for multiple sclerosis, the user may be taking beta interferons, glatiramer, cladribine, dimethyl fumarate, diroximel fumarate, fingolimod, monomethyl fumarate, ofatumumab, ozanimod, ponesimod, siponimod, teriflunomide, alemtuzumab, mitoxantrone, or natalizumab, among others. For cancer, the user may have been administered chemotherapy, radiation therapy, or immunotherapy, among others. The user suffering from cancer may also be on pain medication, such as acetaminophen, aspirin, ibuprofen, naproxen, and non-steroid anti-inflammatory drugs, among others. The application 125 may increase the efficacy of the medication that the user is taking to address the condition.

The application 125 can include, present, or otherwise provide a user interface 130 including the one or more UI elements 135 to a user of the user device 110 in accordance with a configuration on the application 125. The UI elements 135 may correspond to visual components of the user interface 130, such as a command button, a text box, a check box, a radio button, a menu item, and a slider, among others. In some embodiments, the application 125 may be a digital therapeutics application and may provide a session (sometimes referred to herein as a therapy session) via the user interface 130 for addressing a bias towards negative stimuli associated with the condition.

The application 125 can receive an instruction for presentation of the visual stimuli 175 or the auditory stimuli 180 to the user. The visual stimuli 175 can be or include a stimulus or action to be presented textually, as an image, as a video, or other visual presentation to the user and can include instructions for the user to perform the action to address symptoms associated with the condition. The auditory stimuli 180 can be or include a stimulus or action to be presented auditorily via a transducer 120 coupled with a loudspeaker.

An action related to the visual stimuli 175 can include interacting or not interacting with the user device 110. For example, the action can include tilting the user interface 130 or tapping a visual stimulus 175 presented by the user device 110. In some embodiments, tilting the user device can change the presentation of the visual stimuli 175. For example, tilting the mobile device to the left may cause a first visual stimulus to become more legible than or stand out over a second visual stimulus. In a similar manner, tilting the mobile device to the right may cause a second visual stimulus to become more legible or stand out from the first visual stimulus. The action can include instructions for the user to address the condition. For example, the action can include a message with instructions which describe the attention bias towards negative stimuli to be reduced. The action can include an interactive interface, through the user interface 130, to engage the user in one or more therapies designed to reduce or mitigate a bias towards negative stimuli associated with the condition. For example, the user may play a game on the user device 110 presented by the application 125 which incorporates one or more therapies to address the bias.

The database 165 may store and maintain various resources and data associated with the session management service 105 and the application 125. The database 165 may include a database management system (DBMS) to arrange and organize the data maintained thereon. The database 165 may be in communication with the session management service 105 and the one or more user devices 110 via the network 115. While running various operations, the session management service 105 and the application 125 may access the database 165 to retrieve identified data therefrom. The session management service 105 and the application 125 may also write data onto the database 165 from running such operations.

Such operations may include the maintenance of the user profile 170 (sometimes herein referred to as a subject profile). The user profile 170 can include information pertaining to a condition of a user, as described herein. For example, the user profile 170 may include information related to the severity of the condition, occurrences of the condition (such as occurrences of symptom associated with an attention bias due to the condition affecting the cognitive function of the user), medications or treatments the user takes for the condition, and/or a duration of the condition, among others. The user profile 170 can be updated responsive to a schedule, periodically (e.g., daily, weekly), responsive to a change in user information (e.g., input by the user via the user interface 130 or learned from the user device 110), or responsive to a clinician (e.g., a doctor or nurse) addressing the user's condition, among others.

The user profile 170 can store and maintain information related to a user of the application 125 through user device 110. Each user profile 170 may be associated with or correspond to a respective subject or user of the application 125. The user profile 170 may contain or store information for each session performed by the user. The information for a session may include various parameters, actions, visual stimuli 175, auditory stimuli 180, or tasks of previous sessions performed by the user, and may initially be null. The user profile 170 can enable streamlined communications to the user by presenting a task to the user which, based on at least the user profile 170, is most likely to aid the user in addressing symptoms of her condition or reducing the bias towards negative stimuli. This directed approach can reduce the need for multiple communications with the user, thereby reducing bandwidth and increasing the benefit of the user-computer interaction.

In some embodiments, the user profile 170 may identify or include information on a treatment regimen undertaken by the user, such as a type of treatment (e.g., therapy, pharmaceutical, or psychotherapy), duration (e.g., days, weeks, or years), and frequency (e.g., daily, weekly, quarterly, annually), among others. The user profile 170 may be stored and maintained in the database 165 using one or more files (e.g., extensible markup language (XML), comma-separated values (CSV) delimited text files, or a structured query language (SQL) file). The user profile 170 may be iteratively updated as the user provides responses and performs actions related to the visual stimuli 175, the auditory stimuli 180, or the session.

The visual stimuli 175 can be or include a stimulus or action to be presented textually, as an image, video, or other visual presentation to the user. For example, the visual stimuli 175 can include an animation to be presented via the user interface 130 of the user device 110. The visual stimuli 175 can include images such as photographs, digital images, art, diagrams, shapes, or other images. The visual stimuli 175 can include live, pre-recorded, or generated videos or animations, such as video recordings, animated shorts, or animated images (e.g., Graphics Interchange Format (GIF)). The visual stimuli 175 can include 3-dimensional (3D) visual presentations, such as holograms, projections, or other 3D visual media. The visual stimuli 175 can be in any size or orientation executable by the user interface 130. The visual stimuli 175 can include text, such as a word or sentence to be presented to the user via the user interface 130. The visual stimuli 175 can include instructions for the user to perform an action to address symptoms associated with the condition. For example, the visual stimuli 175 can include text or graphics which depict an action for the user to take or perform in relation to the visual stimulus 175.

The visual stimuli 175 can include two or more text-based or image-based stimuli. In some embodiments, the two or more stimuli can be superimposed over each other such that the user can focus and comprehend one stimulus at a time. For example, two visual stimuli including text can be presented via the user interface 130. The two visual stimuli can overlap in such a manner that the user is able to read both visual stimuli. The user may be able to focus on one visual stimulus of the two visual stimuli to bring the one visual stimulus into greater focus. In some embodiments, one or more visual stimuli 175 may have a positive or neutral connotation and one or more other visual stimuli 175 may have a negative connotation. For example, a first visual stimulus can be a word or image with a positive association, such as "love" or an image of a smiling baby. A second visual stimulus can be a word or image with a negative association, such as "pain" or an image of a crying baby. In some cases, the second visual stimulus can be associated with the condition of the user. For example, the second visual stimulus can include a word associated with the condition, such as "pain" or an image or video associated with the condition, such as an image of someone in pain.

The auditory stimuli 180 can be or include a stimulus to be presented auditorily to the user via a transducer 120 coupled with the user device 110. The auditory stimuli 180 can include pre-recorded sounds such as recordings of voices, instruments, nature sounds (e.g., rain or whale songs), or other such recordings. The auditory stimuli 180 can include computer-generated sounds such as MIDI tones, white noise, brown noise, or computer-generated voices, among other computer-generated sounds. The auditory stimuli 180 can include live sounds, such as from a broadcasting radio station. The auditory stimuli 180 can be divided into portions. The auditory stimuli 180 can include or be divided into portions, where each portion is a subset of the auditory stimuli. For example, a portion can be a tone over a period of time, multiple tones over a period of time, or a frequency of a tone of a first auditory stimulus, among others. The auditory stimulus 180 can be each portion played together, or different portions at different times, such as in a musical pattern. In some embodiments, the stimuli may include music, words including varying color and font options, word choices, binaural beats including alpha or beta wave lengths (e.g., wavelengths associated with a relaxed or rested state of mind) and vibrations at various frequencies other than music.

In addition, identifications of the visual stimuli 175 and the auditory stimuli 180 may be stored and maintained on the database 165. For example, the database 165 may maintain the visual stimuli 175 using one or more data structures or files (e.g., extensible markup language (XML), comma-separated values (CSV) delimited text files, joint photographic experts group (JPEG), or a structured query language (SQL) file). Each of the visual stimuli 175 may prompt the user via the application 125 to perform an action via the application 125. For example, the application 125 may receive instructions to present an activity to be performed as a part of the visual stimuli 175. The visual stimuli 175 may be used to provide therapies to reduce the bias towards a negative stimulus associated with the condition, symptoms of the condition, or other cognitive or behavioral effects of the condition. The visual stimuli 175 may be presented as games, activities, or actions to be performed by the user via the user interface 130. For example, one or more of the visual stimuli 175 may be presented as overlapping text prompting the user to choose the visual stimulus 175 which does not have a negative association.

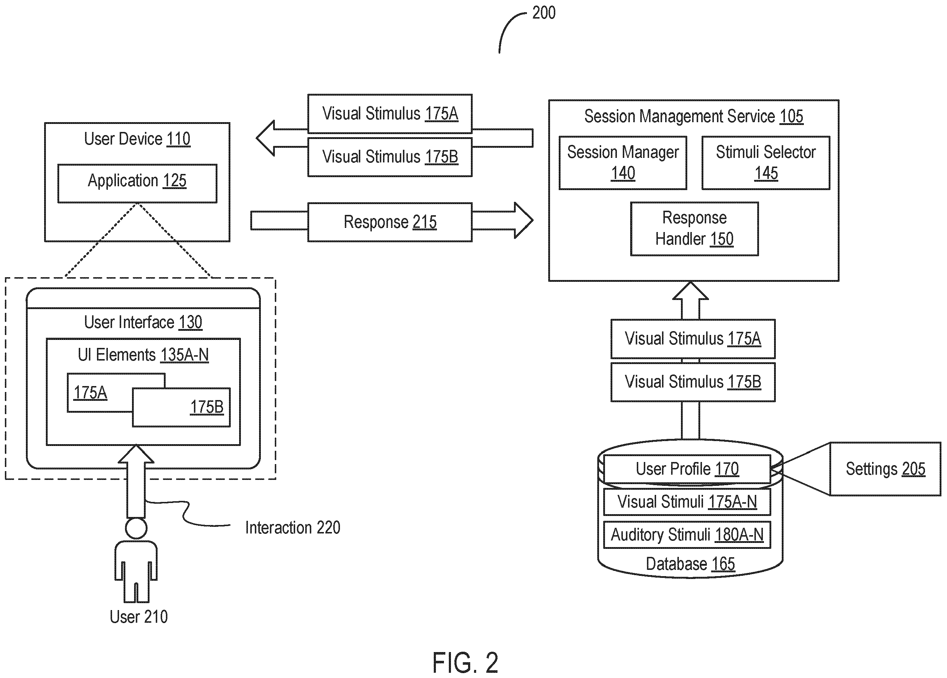

Referring now to FIG. 2, depicted is a block diagram for a process 200 to present the stimuli and detect a response identifying an interaction associated with the stimuli. The process 200 may include or correspond to operations performed in the system 100 to present multimodal stimuli to address symptoms associated with conditions. Under the process 200, the session manager 140 executing on the session management service 105 may access the database 165 to retrieve, fetch, or otherwise identify the user profile 170 for a user 210 (sometimes herein referred to as a subject, patient, or person) of the application 125 on the user device 110. The user profile 170 may identify or define information associated with the user 210, the instance of the application 125 on the user device 110, and the user device 110, among others. For example, the user profile 170 may identify that user 210 has a certain bias towards negative stimuli, symptoms associated with a condition, or other cognitive or behavioral results from the condition. The user profile 170 may identify taking of medication by the user 210 to address the condition or associated symptoms of the condition in the user 210, among others.

The session manager 140 may determine or identify a session for the user 210 for addressing symptoms associated with the condition. The session may correspond to, include, or define a set of stimuli to be presented to the user 210 via the application 125, such as the visual stimuli 175 or the auditory stimuli 180. Each stimulus may be an audio, visual, or haptic stimulus to address the condition of the user. The session manager 140 can identify the session to address a condition of the user 210 associated with the user profile 170.

The user profile 170 may include information on the visual stimuli 175, the auditory stimuli 180, prior sessions (such as previous visual stimuli 175 identified for the user 210), a performance associated with the visual stimuli 175 already identified for the user 210, or a taking of medication by the user 210 to address the condition of the user, among others. The user profile 170 may also identify or include information on recorded performance of the bias, such as a number of occurrences of negative bias, symptoms associated with the condition, a number of occurrences of engaging in a bias towards negative, positive, or neutral stimuli associated with the condition, durations of prior occurrences, and taking of medication, among others. The user profile 170 may initially lack information about prior sessions and may build information as the user 210 engages in the session via the application 125. The user profile 170 can be used to select the one or more stimuli 175 to provide via the application 125 to the user 210 in the session.

The session manager 140 may initiate the session responsive to receiving a request from the user 210 via the application 125. The user 210 may provide, via the user interface 130 to execute through the application 125, a request to start a session. The request may include information related to the onset of the user's condition. The request can include attributes associated with the condition, such as an identification of the user 210 or the user profile 170, symptoms associated with the condition of the user 210, a time of the request, or a severity of the condition, among others. The application 125 operating on the user device 110 can generate the request to start the session to send to the session management service 105 in response to an interaction by the user 210 with the application 125. In some embodiments, the session manager 140 may initiate the session responsive to a scheduled session time, responsive to a receipt of settings 205, or based on the user 210 taking a prescribed medication to address the condition, among others.

The session manager 140 can initiate the session responsive to the receipt of a preference for operation of the session. The session manager 140 can identify one or more stimuli for presentation to the user 210 for addressing the condition based on input including the settings 205 (also herein referred to as the user settings 205) from the user 210. The user 210 can provide the settings 205 before, during, or subsequent to the session provided by the session manager 140. In some embodiments, the user 210 can provide the settings 205 with the request to initiate the session. The user 210 can provide the settings 205 via the user interface 130. The user 210 can interact with the user interface 130 via the UI elements 135 to provide an input of the settings 205 for performance of the session, identification of the visual stimuli 175, a duration available for the session, or symptoms or conditions to be addressed during the session, among others.

The stimuli selector 145 executing on the session management service 105 may select or identify a set of visual stimuli 175 for presentation to the user 210 for the session. The stimuli selector 145 may select the visual stimuli 175 from the stimuli identified by the session manager 140. The stimuli selector 145 may select the visual stimuli 175 as a part of a session to perform attention bias modification training (ABMT) for the user 210 experiencing the condition. The set of visual stimuli 175 can include at least one visual stimulus 175A associated with the condition. As a part of the ABMT session, the stimuli selector 145 may select a first visual stimulus 175A and a second visual stimulus 175B for the user 210. Each visual stimulus 175 may correspond to or be associated with an action to be performed by the user 210 through the application 125 or the user device 110 itself. For instance, the first visual stimulus 175A can be associated with a leftward tilt of the user device 110 and the second visual stimulus 175B can be associated with a rightward tilt of the user device 110.

The first visual stimulus 175A can be a visual stimulus associated with the condition (e.g., threat-related, condition-related, pain-related, or otherwise negatively associated). Conversely, the second visual stimulus 175B can be a visual stimulus not associated with the condition (e.g., neutral or positively associated). In some cases, the first visual stimulus 175A can be a negative stimulus associated with the condition. For example, the first visual stimulus 175A can include text containing a negative word associated with the condition, such as "pain," "ache," "fear," or "tired." The first visual stimulus 175A can include an image associated with the condition. For example, the first visual stimulus 175A can include an image of a sad or frowning face, an image of a stormy raincloud, or an image of a snarling dog, among others. In some cases, the second visual stimulus 175B can be a positive or neutral stimulus. The second visual stimulus 175B may not be associated with the condition. For example, the second visual stimulus 175B may include positive text containing one or more words such as "happy," "good," "smile," or "love." The second visual stimulus 175B can include neutral text containing one or more words such as "beach," "puppy," or "dinner." The second visual stimulus 175B can include positive or neutral images. For example, the second visual stimulus 175B can be a picture of a tree, a baby, or a bicycle.

In some embodiments, the stimuli selector 145 may select the visual stimuli 175 based on the user profile 170. The user profile 170 may include historical information related to the user's condition, such as occurrences or types of symptoms, time of symptom occurrences, the intensity of the bias towards negative stimuli associated with the condition, demographic information, prescription information, location information, among others. For example, the session manager 140 may identify a visual stimulus 175 which has historically been positively associated by the user 210 towards improving the user's bias towards negative stimuli.

In some embodiments, the stimuli selector 145 may identify the set of visual stimuli 175 based on the user settings 205. The user settings 205 can indicate user-preferred stimuli, such as preferred colors, font type, or type of visual stimuli 175 (e.g., images or videos), among others. The user settings 205 can identify images, text, or other visual stimuli 175 which the user 210 associates with the condition. The user settings 205 can identify visual stimuli 175 which the user 210 associates positively or disassociates from the condition. For example, the user 210 can select types of visual stimuli 175 or provide guidance or criteria for the visual stimuli 175. The user settings 205 can indicate preferred auditory stimuli 180. For example, the user settings 205 can identify sounds which the user 210 associates positively and sounds which the user 210 associates negatively. In this manner, the stimuli selector 145 can select auditory stimuli 180 which can act as a positive reinforcement or negative punishment to the user 210.

The user can provide the user settings 205 to the session manager 140, or the session manager 140 can retrieve the user settings 205. The user 210 can provide the user settings 205, or the session manager 140 can retrieve the settings 205 from an external computing system, clinician, or library of pre-generated visual or auditory stimuli. The user settings 205 can include the settings 205 as a file, such as a comma-separated file (CSV), word document (DOC), standard MIDI file (SMF), or MP3, among others. The user settings 205 can be provided via input into the application 125 operating on the user device 110.

The user settings 205 can include a list identifying a set of visual stimuli. For example, the user settings 205 can include a list of words selected from an interface provided before the start of any session or as part an initiation process in signing into the application 125. The visual stimuli 175 can be selected from the set or list of words provided in the settings 205. The user 210 can provide, via the interface 130, a first set of words related to the condition (or symptoms) and a second set of words not related to the condition (or symptoms). The user 210 can provide, via the interface 130, a first set of words with a negative association and a second set of words with a positive or neutral association. The stimuli selector 145 can select the first visual stimulus 175A from first set of words and the second visual stimulus 175B from second set of words.

The user settings 205 can include a property for an auditory stimulus. The properties can include attributes associated with the auditory stimulus such as volume, pitch, speed, tempo, compression, pan, bit rate, distortion, or timbre. For example, the user settings 205 can include a desired, maximum, or minimum volume for playback of the auditory stimulus 180. The user settings 205 can include preferred or unpreferred instruments, tempos, or pitches. The property can include a desired sound selected by the user 210. For example, the user 210 may select a preferred song or a preferred genre of songs for playback as a part of the auditory stimulus 180. The user 210 may select disliked sounds or songs as a part of the auditory stimulus 180.

In some embodiments, the stimuli selector 145 may identify the visual stimuli 175 or the auditory stimuli 180 based on a predefined schedule of stimuli. For example, the stimuli selector 145 may identify the first visual stimulus 175A to be a visual stimulus associated with the condition in accordance with the predefined schedule of tasks. In this illustrative example, the stimuli selector 145 can identify a second visual stimulus 175B based on the subsequent stimulus of the predefined schedule. For example, a predefined schedule of stimuli may include a first and second stimuli corresponding to visual stimuli, a third stimulus corresponding to an auditory stimulus, or a fourth stimulus corresponding to a haptic stimulus. The session manager 140 may define a schedule or time at which the stimuli selector 145 may identify the stimuli or at which to mark the stimuli for presentation. In some embodiments, the stimuli selector 145 can identify the visual stimuli 175 or the auditory stimuli 180 based on a set of rules. The rules may be configured to provide a visual stimulus 175 or set of visual stimuli 175 to target the underlying causes or alleviate the symptoms of the condition in the user 210 in a systematic, objective, and therapeutically effective manner. The rules may be based around time of completion of an action associated with the visual stimulus 175, the user profile 170, a playback of the auditory stimulus 180, or other attributes of the system 100.

In some embodiments, the stimuli selector 145 may assign a type of interaction to each of the visual stimuli 175. The stimuli selector 145 may assign a first type of interaction to the first visual stimulus 175A and a second type of interaction to the second visual stimulus 175B. The type of the interaction may be or include a manipulation of the user device 110, touching or pressing the user interface 130, or speaking into the user device 110, among others. For example, the interaction can include the user 210 tilting the user device 110. For example, the interaction can include the user 210 pressing a button (of the UI elements 135) presented by the application 125. In some embodiments, the first type of interaction associated with the first visual stimulus 175A can be related to the condition of the user 210. For example, the first type of interaction can include selecting a UI element 135 button corresponding to the first visual stimulus 175A when the user interface 130 presents the word "pain." In some embodiments, the second type of interaction associated with the second visual stimulus 175B may include disassociating the user from the condition. For example, the second type of interaction can include not pressing a button associated with the word "pain," or pressing a button associated with a positive second visual stimulus 175B, such as the word "happy."

Upon identification, the session manager 140 may provide, send, or otherwise transmit the set of visual stimuli 175 to the user device 110. In some embodiments, the session manager 140 may send an instruction for presentation of the visual stimuli 175 via the user interface 130 for the application 125 on the user device 110. The instruction may include, for example, a specification as to which UI elements 135 are to be used and may identify content to be displayed on the UI elements 135 of the user interface 130. The instructions can further identify or include the visual stimuli 175. The instructions may be code, data packets, or a control to present the visual stimuli 175 to the user 210 via the application 125 running on the user device 110.

Continuing on, the instructions may include processing instructions for display of the visual stimulus 175 on the application 125. The instruction may include processing instruction for playing audio associated with the auditory stimulus 180, or instructions for actuating a haptic system of the user device 110. The instructions may also include instructions for the user 210 to perform in relation to their session. For example, the instructions may display a message instructing the user 210 to take a medication associated with their session. The visual stimulus 175 may include a text, image, or video presented by the user device 110 via the application 125. For example, the visual stimulus 175 may include presentation of an image instructing the user 210 to interact with the application 125 via the user interface 130.

The application 125 on the user device 110 may render, display, or otherwise present the set of visual stimuli 175. The visual stimuli 175 may be presented via the one or more UI elements 135 of the user interface 130 of the application 125 on the user device 110. The presentation of the UI elements 135 can be in accordance with the instructions provided by the session manager 140 for presentation of the visual stimuli 175 to the user 210 via the application 125. In some embodiments, the application 125 can render, display, or otherwise present the visual stimuli 175 independently of the session management service 105. The application 125 may share or have the same functionalities as the session manager 140, the stimuli selector 145, or other components of the session management service 105 as discussed above. For example, the application 125 may maintain a timer to keep track of time elapsed since presentation of a previous visual stimulus 175. The application 125 may compare the elapsed time with a time limit for the visual stimulus 175. When the elapsed time exceeds the time limit, the application 125 may determine to present the visual stimulus 175. The application 125 may also use a schedule to determine when to present the one or more visual stimuli 175. The application 125 may present the visual stimulus 175 for display through the user interface 130 on the user device 110.

The application 125 can display, render, or otherwise present the visual stimuli 175A and 175B at an at least partially concurrent position of the user interface 130 of the user device 110. The concurrent position can refer to a position on the display (e.g., the user interface 130) which presents at least a portion of the visual stimulus 175A as overlapped with at least a portion of the visual stimulus 175B. For example, the visual stimuli 175A and 175B can overlap in entirety or in part. The visual stimuli 175A and 175B can overlap with varying opacity. The opacity can refer to the transparency of a visual stimulus 175A. In some cases, the opacity of the first visual stimulus 175A may be greater than the second visual stimulus 175B such that the first visual stimulus 175A covers the second visual stimulus 175B in entirety for the overlapping portion of each visual stimulus 175A and 175B. In some cases, the opacity of the first visual stimulus 175A may be equal or similar enough to the opacity of the second visual stimulus 175B such that both the first visual stimulus 175A and the second visual stimulus 175B are visible or legible while presented on the user interface 130 by the application 125. The presentation of the overlapping visual stimuli 175A and 175B can change in opacity. For example, during a first time period, the first visual stimulus 175A may have greater opacity than the second visual stimulus 175B, and the first visual stimulus 175A may have lower opacity than the second visual stimulus 175B during a second time period.

In some embodiments, the application 125 may display, render, or otherwise present the visual stimuli 175A and 175B for different time periods. The application 125 may present the first visual stimulus 175A for a first time period and the second visual stimulus 175B for a second time period. For example, the application 125 may present the first visual stimulus 175A during the first time period and then present the second visual stimulus 175B during the second time period. In some cases, the application 125 may delay the presentation of the second visual stimulus 175B after displaying the first visual stimulus 175A. The application may present the visual stimuli 175A and 175B concurrently. Presenting the visual stimuli 175A and 175B concurrently can refer to displaying the visual stimuli 175 during a concurrent time period, or with a concurrent display position on the user interface 130. A concurrent time period can refer to the first time period and the second time period overlapping in entirety or in part. For example, the presentation of the first stimulus 175A can overlap in duration with the presentation of the second stimulus 175B. The application 125 may present the visual stimuli 175A and 175B for the same period of time or different periods of time. For example, the first time period associated with the first visual stimulus 175A can be the same as or equivalent to the second time period associated with the second visual stimulus 175B.

In some embodiments, the application 125 may present the visual stimuli 175 concurrently with another type of stimuli. For example, the application 125 may display the visual stimuli 175A and 175B concurrently with an auditory stimulus 180. The application 125 may display one or more of the visual stimuli 175 for a first period of time without the auditory stimulus 180. The application 125 may display one or more of the visual stimuli 175 concurrently with one or more of the auditory stimuli 180 for a second period of time. The application 125 may present the auditory stimulus 180 in whole or in part, such as the application 125 presenting portions of the auditory stimulus 180 as described herein. In other cases, the application 125 may present the visual stimuli 175 concurrently with another type of stimulus, such as a haptic stimulus. For example, the application 125 may present the first visual stimulus 175A while actuating the user device 110 to create a vibration, buzz, or other haptic stimulus for the user 210.

The application 125 may display the visual stimuli 175A and 175B to direct the user 210 to interact with the display. The visual stimuli 175A and 175B may include prompts for the user 210 to perform an action associated with the stimuli 175A and 175B. The action may be or include an interaction 220 from the user 210 related to the presentation of the visual stimuli 175. The interaction 220 can include actions such as a physical manipulation of the user device 110, actuation of the UI elements 135, or viewing of a video or image of the visual stimuli 175, among others. The interaction 220 can include an action of moving, tapping, sliding, nodding, shaking, tilting, or orienting the user device 110 towards a side of the user device 110. A side of the user device 110 can refer to a boundary created by the user interface 130 associated with the user device 110, such as a side of a rectangular screen. One or more sides of the client can be associated with the first visual stimulus 175A. In some embodiments, the user 210 can perform the interaction 220 to tilt the user device 110 towards a side of the user device 110 which corresponds to the first visual stimulus 175A.

The interaction 220 can include an action such as touching, pressing, or otherwise actuating a UI element 135 of the user interface 130 associated with at least one of the first stimulus 175A or the second stimulus 175B. For example, the user 210 can provide one or more interactions 220 through the application 125 running on the user device 110 by actuating one or more of the UI elements 135 as described herein. The user 210 can provide the interaction 220 by pressing a button associated with the application 125 and displayed via the user interface 130. In some embodiments, one or more first UI elements 135A can be associated with the first visual stimulus 175A and one or more second UI elements 135B can be associated with the second visual stimulus 175B. In this illustrative example, the user 210 can provide the interaction 220 associated with the first visual stimulus 175A by touching, tilting, nodding, swiping, tapping, or otherwise engaging with the first UI elements 135A. Similarly, the user 210 can provide the interaction 220 associated with the second visual stimulus 175B by engaging with the second UI elements 135B.

The interaction 220 can include a series of actions performed sequentially or concurrently. For example, the interaction 220 can include a manipulation of the user device 110 and a pressing of a UI element 135. The manipulation of the user device 110 and the pressing of the UI element 135 can be performed concurrently as a part of the same interaction 220, or sequentially as a part of the same interaction 220. For example, the user 210 can tilt the user device 110 and press the UI element 135 at the same time, or the user 210 can tilt the user device 110 and then press the UI element 135. The application 125 may present one or more visual stimuli 175 via the user interface 130 to direct the user 210 to perform the interaction 220.

Presenting the stimuli via the user interface 130 can include presenting the first visual stimulus 175A with a first indicator to identify the first type of the interaction 220 and presenting the second visual stimulus 175B with a second indicator to identify the second type of the interaction 220. The indicators can include a visual presentation, such as text or a color. For example, the indicator can include a green highlight over the first visual stimulus 175A and a red highlight over the second visual stimulus 175B to indicate to the user 210 to select the first visual stimulus 175A. As another example, the indicators can include text directing the user 210 as to the type of interaction 220 to perform, such as text denoting "Tilt towards the positive stimulus" or "Do not press the negative stimulus." Each visual stimulus presented by the application 125 can include an indicator. For example, the first visual stimulus 175A can include an indicator stating "Pick Me!" and the second visual stimulus 175B can include an indicator stating "Don't Pick Me!"

In some embodiments, the indicator can include a timer. For example, the application 125 can present a timer as an indicator for a presentation of a visual stimulus 175 or to count down to the performance of an interaction 220. In some embodiments, the indicator can produce an audio tone to prompt the user 210 to perform a specified interaction with the visual stimuli 175. For example, the first indicator may include a beep, emitted from the user device 110 via the loudspeaker and transducer 120, to indicate to the user 210 that they should interact with the first visual stimulus 175A. The second indicator may include a different (e.g., in tone, duration, pitch, volume) beep, emitted from the user device 110 via the loudspeaker and the transducer 120, to indicate to the user 210 that they should interact with the second visual stimulus 175B.

The application 125 may monitor for at least one interaction 220 with the visual stimuli 175. The application 125 can monitor during the session responsive to presentation of the visual stimuli 175 or responsive to receiving the interaction 220. The application 125 can monitor for receipt of the interaction 220. The application 125 can monitor for the interaction 220 through the user interface 130 or through sensors associated with the user device 110, among others. The application 125 can receive multiple interactions 220 during a session. For example, the application 125 can monitor for a series of interactions 220 provided by the user 210 during the session. The application 125 may monitor and record information related to the received interactions 220. For example, the application 125 may monitor and record a time of an interaction 220, a duration of an interaction 220, a sequence of interactions 220, the visual stimulus 175 associated with the interaction 220, and/or the delay time between the presentation of the visual stimulus 175 and the interaction 220, among others.

Upon the user 210 providing the interaction 220, the application 125 may generate at least one response 215. The response 215 can identify the interaction 220. The response 215 can include the information about the interaction 220, such as a duration of the interaction 220, a time of the interaction 220, the visual stimulus 175 associated with the interaction 220, and/or a delay time between the presentation of the visual stimulus 175 and the interaction 220, among others. The application 125 can generate the response 215 for transmittal to the session management service 105. The response 215 can be in a format readable by the session management service, such as an electronic file readable by the session management service or data packets readable by the session management service 105, among others.

The response handler 150 can receive, identify, or otherwise detect a response 215 identifying the interaction 220. The response handler 150 can receive the response 215 from the application 125. The response handler 150 can receive the response 215 at scheduled time intervals or as the interactions 220 occur during the session. The response handler 150 can query or ping the application 125 for the response 215. The response handler 150 can receive multiple responses 215 during a time period. For example, the response handler 150 can receive a first response 215 indicating a first interaction 220 and a second response 215 indicating a second interaction 220.

The response handler 150 can store the response 215 including the interaction 220 in the database 165. The response handler 150 can store information related to the response 215, including a time of the response 215, actions associated with the interaction 220, the user profile 170 associated with the response 215, and the visual stimuli 175 associated with the response 215, among others. The response 215 may include or identify the interaction 220 by the user 210 with the visual stimulus 175A or 175B. The response 215 may include a time for task completion. For example, the response 215 may include that the user spent 4 minutes to perform the action associated with the presentation of the visual stimuli 175. The response 215 can include a total time for completion of the session and may also include a time of initiation of the session and a time of completion of the session. The response 215 may include the UI elements 135 interacted with during the duration of the presentation of the visual stimuli 175. For example, the response 215 may include a listing of buttons, toggles, or other UI elements 135 selected by the user 210 at specified times during the presentation of the visual stimuli 175. The response 215 may include other information, such as a location of the user 210 while performing the session, such as a geolocation, IP address, GPS location, or triangulation by cellular towers, among others. The response 215 may include measurements such as measurements of time, location, or user data, among others.

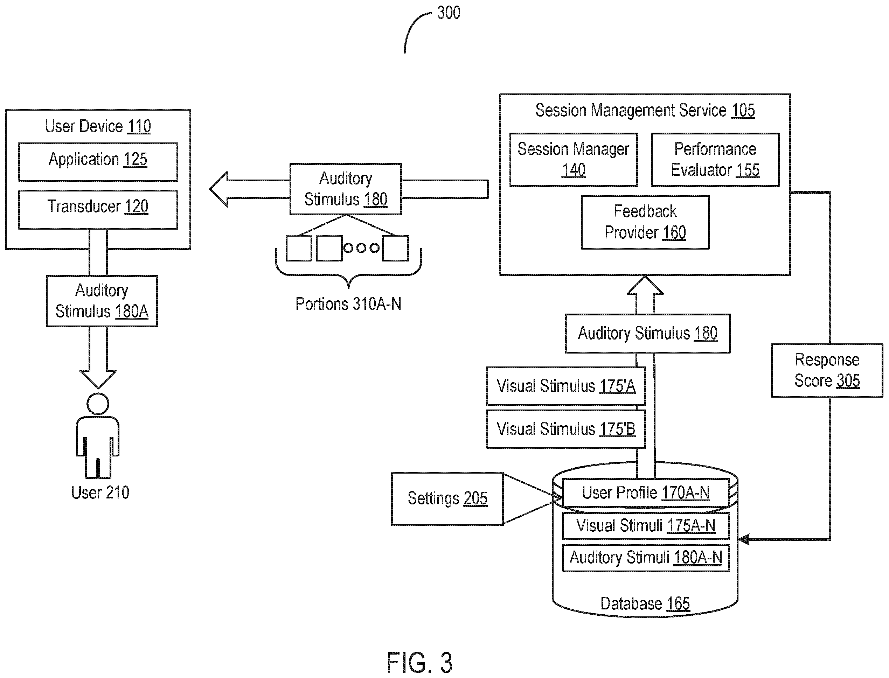

Referring now to FIG. 3, depicted is a block diagram for a process 300 to generate an auditory stimulus and to play the auditory stimulus to provide feedback. The process 300 may include or correspond to operations performed in the system 100 or the process 200. Under the process 300, the performance evaluator 155 may evaluate the response 215 to generate a response score 305. The feedback provider 160 may generate or select a stimulus in accordance with the response score 305 to provide feedback. The session manager 140 may transmit the feedback to the application 125 for presentation to the user 210. The presentation of the visual stimuli 175 or the auditory stimuli 180 may be based on the prior visual stimuli 175, the user profile 170, the response 215, or a determination by the performance evaluator 155.

The performance evaluator 155 can calculate, generate, or otherwise determine the response score 305 associated with the response 215 based on the interaction 220 with the visual stimuli 175. The response score 305 can indicate a level of correctness or conversely a level of error associated with the response 215. A high response score 305 can correlate with a high level of correctness in selecting the visual stimulus 175 which does not relate to the bias towards the condition. A low response score 305 can correlate with a low level of correctness (e.g., high level of error) in selecting the visual stimulus 175 which does not relate to the bias towards the condition. For example, a low response score 305 can indicate that the user 210 is more likely to select a first visual stimulus 175A associated with a negative stimulus or associated with the user's condition.

In determining the response score 305, the performance evaluator 155 may evaluate the response 215 based on the interaction 220. The response 215 may be correct, incorrect, or undeterminable. In some embodiments, the second visual stimulus 175B can be or include a stimulus to disassociate the user 210 from the condition. The user 210 may provide an interaction 220 related to disassociating the user 210 from the condition. For example, the user 210 may select the second visual stimulus 175B by the application 125 using the UI elements 135. The user 210 may click, select, touch, or otherwise indicate a preference or selection for the second stimulus 175B through the interaction 220. The interaction 220 may indicate the selection or preference for the second visual stimulus 175B.

The performance evaluator 155 can identify or determine the response 215 by the user as correct or incorrect based on the selected visual stimulus 175. The response 215 may be correct if the interaction 220 corresponding to the response 215 is associated with the second visual stimulus 175B. The performance evaluator 155 can determine the response 215 to be correct if the response 215 is associated with the interaction 220 corresponding to the visual stimulus 175 disassociating from the condition. The performance evaluator 155 may identify the response 215 including the interaction 220 as correct.

The performance evaluator 155 may identify the response 215 as correct if the interaction 220 indicates a bias towards a positive or neutral stimulus. In some embodiments, the interaction 220 can be associated with a positive or neutral visual stimulus 175B. For example, the interaction 220 can include selecting the positive or neutral visual stimulus 175B. The positive or neutral stimulus 175B can include positive or neutral imagery, text, or videos, among others, which is not related to the condition of the user 210 or to negative stimuli.

Conversely, the performance evaluator 155 may identify the response 215 as incorrect if the interaction 220 associated with the response 215 is with the first stimulus 175A associated with the condition. In some embodiments, the interaction 220 can be associated with a negative stimulus, or a stimulus associated with the user's condition. For example, the interaction 220 can include selecting, orienting, sliding, or tilting the user device 110 towards the negative visual stimulus 175A. The negative visual stimulus 175A can include negative imagery, text, or videos, among others. The negative visual stimulus 175A can relate to the condition of the user 210. For example, the negative visual stimulus 175A can include symptoms or emotions associated with the conditions presented as text, such as "pain" or "grief."

Based on whether the response 215 is correct or incorrect, the performance evaluator 155 may calculate, generate, or otherwise evaluate the response score 305 for the user 210 based on the interaction 220 associated with the response 215. For example, the performance evaluator 155 can set the response score 305 for a given response 215 as "1" when correct and "-1" when incorrect. In some embodiments, the performance evaluator 155 may identify a reaction time or a correctness of the user 210 in selecting a visual stimulus 175. For example, the performance evaluator 155 may determine, from the response 215, that the user 210 is not performing one or more actions indicated by the visual stimuli 175, or that the user 210 is not performing the actions within a threshold time. The threshold time may correspond or define an amount of time in which the user 210 is expected to make an interaction with one of the visual stimuli 175 and can range from 5 seconds to 10 minutes. With the determination, the performance evaluator 155 can modify or adjust the response score 305 using the response time compared to the threshold time.

In some embodiments, the performance evaluator 155 can determine a degree of compliance of the user 210. The degree of compliance of the user 210 with the session can be based on a response time of the user 210 to a presentation of the visual stimuli 175 exceeding or being under a threshold time. For example, if the user 210 takes too long to provide the interaction 220, then the user 210 may not be fully participating or compliant with the session. The degree of compliance of the user 210 can be based on the responses 215 provided by the user via the UI elements 135 while engaging in the session. For example, if the user 210 presses a button presented by the application 125 during the session at an incorrect time, or at a time not indicated for pressing the button, the user 210 may have a low degree of compliance. Conversely, if the user 210 provides the correct interaction 220 within a threshold time period and performs a threshold number of the actions according to the presented visual stimuli 175, then the user 210 may have a high degree of compliance.

In some embodiments, the performance evaluator 155 can calculate, generate, or otherwise determine the response score 305 related to the likelihood of overcoming the bias towards negative stimuli. The likelihood of overcoming the bias towards negative stimuli can refer to, include, or be related to a probability that the user 210 will cease to pay mind to stimuli associated with the condition. For example, if the user 210 succeeds in ignoring negative stimuli associated with the condition each time negative stimuli are presented to the user 210 via the application 125, the user 210 can be said to have a 100% rate of overcoming the bias towards negative stimuli. The likelihood of overcoming the bias towards negative stimuli may include a threshold number of occurrences of the bias. For example, the performance evaluator 155 may not determine the likelihood until a threshold number of occurrences of the negative stimuli has arisen, or until a threshold number of interactions 220 have been provided by the user 210. The performance evaluator 155 may determine the likelihood of overcoming the bias towards negative stimuli based at least on selections of the UI elements 135 during the session, the user profile 170, or a time of the session, among others.

In some embodiments, the performance evaluator 155 can calculate, generate, or otherwise determine the response score 305 related to an increase in mental acuity or a decrease in fatigue for the user 210. The performance evaluator 155 can determine the response score 305 based on delay times between the presentation of the visual stimuli 175 and the receipt of the interaction 220. For example, the delay time between subsequent presentations of the visual stimuli 175 and the receipt of the interaction 220 may decrease. This decrease in delay time can indicate an increase in mental acuity or a decrease in fatigue. The performance evaluator 155 can query the user 210, via the application 125, for an indication of the user's mental state. For example, the performance evaluator 155 can present a question, via the user interface 130, asking the user 210 about his perceived level of mental acuity or fatigue. The performance evaluator 155 can generate the response score 305 related to mental acuity or fatigue based on the user 210 providing a response to the query.

In conjunction, the feedback provider 160 may produce, output, or otherwise generate feedback for the user 210 to receive via the application 125 operating on the user interface 130. The feedback provider 160 may generate feedback based on at least the response score 305, the user profile 170, the response 215, or the historic stimuli presentation. The feedback may include text, video, or audio to present to the user 210 via the application 125 displaying through the user interface 130. The feedback can include one or more visual stimuli 175 or one or more auditory stimuli 180. The feedback may include a presentation of the response score 305. The feedback may display a message, such as a motivating message, suggestions to improve performance, a congratulatory message, a consoling message, among others. In some embodiments, the feedback provider may generate the feedback during the session being performed by the user 210.

Based on the response 215, the feedback provider 160 can generate the auditory stimulus 180A to include one or more portions 310A-N (hereinafter generally referred to as the portion 310) for indicating feedback based on the response 215. The feedback provider 160 can generate the auditory stimulus 180 including the portions 310 based on the response score 305, or the settings 205, among others. In generating, the feedback provider may generate the portions 310 of audio for the auditory stimulus 180. The portions 310 can be segments, divisions, tones, or other such subsets of the auditory stimulus 180. In some embodiments, a first portion 310A of the auditory stimulus 180 can be the audio over a first time period. For example, the first portion 310A can be or include the first 2 seconds of the auditory stimulus 180. In some embodiments, the first portion 310A of the auditory stimulus 180 can be a tone of the auditory stimulus 180. For example, the first portion 310A can be or include the musical pitch B-flat. In some embodiments, the first portion 310A of the auditory stimulus 180 can be or include a musical part, such as denoted by a musical score. For example, the first portion 310A can be or include a violin score, or a soprano score, while the entirety of the auditory stimulus 180 includes a full score, such as a violin score, soprano score, and piano score.

In generating the portions 310 of the auditory stimulus 180, the feedback provider 160 can identify or access recorded sounds or music. The feedback provider 160 can draw from, retrieve, or access a library of prerecorded sounds or music. The feedback provider 160 can generate recorded sounds through a microphone of the user device 110. The feedback provider 160 can generate the auditory stimulus 180 from the pre-recorded or recorded sounds or music. The feedback provider 160 can generate the auditory stimulus 180 by generating tones through the user device 110, such as by a MIDI file associated with the auditory stimulus 180. The feedback provider 160 can generate an electronic sound file or electronic file used to generate sound, such as MIDI, MP3, or WAV, among others.

Based on whether the response 215 is correct or incorrect, the feedback provider 160 can generate the auditory stimulus 180 to indicate feedback. The feedback can provide positive reinforcement or negative punishment for the user 210 depending on the responses 215 from the user 210. When the response 215 is determined to be correct, the feedback provider 160 can generate the auditory stimulus 180 to provide positive reinforcement. To provide positive reinforcement, the feedback provider 160 can insert, supplement, or otherwise add at least one portion 310 to the auditory stimulus 180. In some embodiments, the feedback provider 160 can generate or select the first portion 310A indicating positive feedback to provide to the user 210 based on the response score 305 being at or above a threshold score. For example, if the response score 305 associated with the user 210 for a session is above the threshold score, the feedback provider 160 can generate the first portion 310A for playback to the user to encourage the user or provide positive reinforcement. In some embodiments, the feedback provider 160 can change, adjust, or otherwise modify (e.g., increase) a volume of the auditory stimulus 180 to provide positive reinforcement.

Conversely, when the response 215 is determined to be incorrect, the feedback provider 160 can generate the auditory stimulus 180 to provide negative punishment. To provide negative punishment, the feedback provider 160 can delete, subtract, or otherwise remove at least one portion 310 from the auditory stimulus 180. In some embodiments, the feedback provider 160 can generate or select the second portion 310B indicating negative feedback to provide to the user 210 if the response score 305 is below the threshold score. In some embodiments, the feedback provider 160 can change, adjust, or otherwise modify (e.g., decrease) a volume of the auditory stimulus 180 to provide negative punishment. The generation of positive reinforcement or negative punishment can be used in conjunction with the ABMT session to reduce the user's bias towards negative stimuli associated with his condition.

In some embodiments, the feedback provider 160 can provide a preferred portion or additional portion if the response 215 is correct, or associated with a correct interaction 220, to provide positive reinforcement for the user 210 in correcting the bias for negative stimuli associated with the condition. The user 210 can select or indicate preferred portions 310 or auditory stimuli 180 as one or more of the settings 205. The feedback provider 160 may provide a preferred portion 310A responsive to receiving the response 215 indicating that the user 210 performed a correct interaction 220 (e.g., an interaction 220 associated with a positive visual stimulus 175B or not associated with the condition). The feedback provider 160 may provide a preferred portion 310A responsive to the response score 305 being at or above the threshold score.

In some embodiments, the feedback provider 160 can provide a non-preferred portion for inclusion in the auditory stimulus 180 if the response 215 is incorrect or associated with an incorrect interaction 220. The feedback provider 160 may provide the non-preferred portion to provide negative punishment for the user 210 in correcting the bias for negative stimuli associated with the condition. The user 210 can select or indicate non-preferred portions 310 or auditory stimuli 180 as one or more of the settings 205. The feedback provider 160 may provide a non-preferred portion 310B responsive to receiving the response 215 indicating that the user 210 performed an incorrect interaction 220 (e.g., an interaction 220 associated with a negative visual stimulus 175A or the condition). In this manner, the feedback provider 160 can provide negative punishment to the user 210 upon providing an incorrect response or having a response score 305 below the score threshold.

With successive responses, the feedback provider 160 can send, convey, or otherwise provide feedback to the user 210 by adding or removing different portions 310 of the auditory stimulus 180. Upon receipt of an incorrect response, the feedback provider 160 may remove the preferred portion 310A from playback to the user 210. Likewise, responsive to a correct response 215 or the response score 305 being at or above the score threshold, the feedback provider 160 may provide the preferred portion 310A, remove the non-preferred portion 310B, or a combination thereof. As the user 210 provides more correct responses 215, or as the response score 305 increases, the feedback provider 160 may provide additional preferred portions 310 for playback to the user 210. The additional preferred portions 310 may be played concurrently. The concurrent playback of the preferred portions 310 can create a song, such as a preferred song indicated by the user 210 in the settings 205. In this manner, the feedback provider 160 can provide an auditory stimulus 180A including preferred portions for positive reinforcement. Similarly, the feedback provider 160 can add non-preferred portions 310 to the auditory stimulus 180A responsive to incorrect responses 215 or the response score 305 being below the threshold score to provide negative punishment. In this manner, the user 210 may be presented with a non-preferred song based on the responses 215 to provide negative punishment.

In some embodiments, the feedback provider 160 can generate the auditory stimulus 180 for playback to left and right ears of the user 210. The auditory stimulus 180 may include stereo audio, with different portions 310 to be provide to the left and right ears. The feedback provider 160 can identify or select different portions 310 for playback to each ear of the user 210. In some embodiments, the portions 310 may be selected by the feedback provider 160 to provide binaural perception by the user 210. For example, the feedback provider 160 can generate a first portion 310A including a tone at a frequency of 415 Hz and a second portion 310B including a tone at a frequency of 400 Hz to provide a binaural beat. The binaural beat may be provided through the auditory stimulus 180, to address fatigue associated with the condition (e.g., multiple sclerosis or cancer) in the user 210. The feedback provider 160 can provide the first portion 310A to the left ear of the user 210 using a first loudspeaker and the second portion 310B to the right ear of the user 210 using a second loudspeaker.

In some embodiments, the feedback provider 160 can generate auditory stimuli 180 based on the user profile 170. The user profile 170 can include historical associations between prior-presented auditory stimuli 180 and prior responses 215. For example, the user profile 170 can indicate that the auditory stimulus 180A has historically produced an increased response score 305 for the user 210 during the session. For example, the user profile 170 can indicate that the auditory stimulus 180A has historically been correlated with incorrect responses 215.

In some embodiments, the feedback provider 160 can generate the auditory stimulus 180 based on the user settings 205. The user settings 205 can include properties of the auditory stimulus 180. The user 210 can provide, via the application 125, preferred or non-preferred auditory stimuli 180. For example, the user 210 can indicate a preferred song or a non-preferred musical instrument. The feedback provider 160 can generate the auditory stimulus 180 for positive reinforcement feedback from the preferred auditory stimuli 180 provided by the user 210 in the settings 205. Likewise, the feedback provider 160 can generate the auditory stimulus 180 for negative punishment feedback from the non-preferred auditory stimuli 180 provided by the user 210 in the settings 205.

In some embodiments, the feedback provider 160 can provide one or more of the portions 310 in at least partial concurrence with presentation of the visual stimulus 175 by the stimuli selector 145. The feedback provider 160 can provide one or more of the portions 310 in at least partial concurrence with a presentation of the visual stimulus 175. For example, the feedback provider 160 can provide the first portion 310A via a loudspeaker coupled with the user device 110 while the application 125 presents the visual stimulus 175. The feedback provider 160 can provide one or more portions 310 in association with a visual stimulus 175. For example, a first visual stimulus 175A can correspond to a first auditory stimulus 180.

Upon generation, the feedback provider 160 can send, transmit, or otherwise provide the auditory stimulus 180 to the application 125. The auditory stimulus 180 may be provided in the form of an audio file (e.g., MPEG, FLAC, WAV, or WMA formats) or as part of an audio stream (e.g., as an MP3, AAC, or OGG format) to the application 125 on the user device 110. In some embodiments, the feedback provider 160 may send, transmit, or otherwise present visual feedback for presentation via the application 125 during the performance of the session or subsequent to the receipt of a response 215. For example, the response score 305 may indicate that the user 210 performing in the session is below a threshold correctness. The feedback provider 160 may generate feedback related to the low response score 305, such as a motivating message including the response score 305. The feedback provider 160 can transmit the auditory stimulus 180 as a part of the feedback. The feedback provider 160 can present the feedback including the auditory stimulus 180 via the application 125 operating on the user device 110.

The application 125 can present, produce, or otherwise play the auditory stimulus 180 via the one or more transducers 120 on the user device 110. The application 125 can generate a signal, packet, or electronic file, among others, for playback via one or more loudspeakers coupled with the user device 110. The application 125 can transmit the signal including instructions for playback of the auditory stimulus 180 to the transducer 120. The application 125 can play the auditory stimulus 180 via the transducer 120. The transducer 120 can include or be coupled with one or more loudspeakers. In some embodiments, the application 125 can provide the auditory stimulus 180 to the transducer 120 for playback via the one or more loudspeakers by generating a signal including the auditory stimulus 180 for the transducer 120 to receive. The transducer 120 can convert the signal to mechanical vibrations via the loudspeakers to produce the sound of the auditory stimulus 180 for playback to the user 210.

The application 125 can provide stereo audio for the auditory stimulus 180 to different ears of the user 210. For example, the transducer 120 can provide a first portion 310A of the auditory stimulus 180 to a first ear and a second portion 310B of the auditory stimulus 180 to a second ear via two loudspeakers. The transducer 120 can be coupled with or configured to actuate one or more loudspeakers. The signal including the auditory stimulus 180 can indicate to the transducer 120 to play the first portion 310A to a first loudspeaker and to play the second portion 310B to a second loudspeaker.

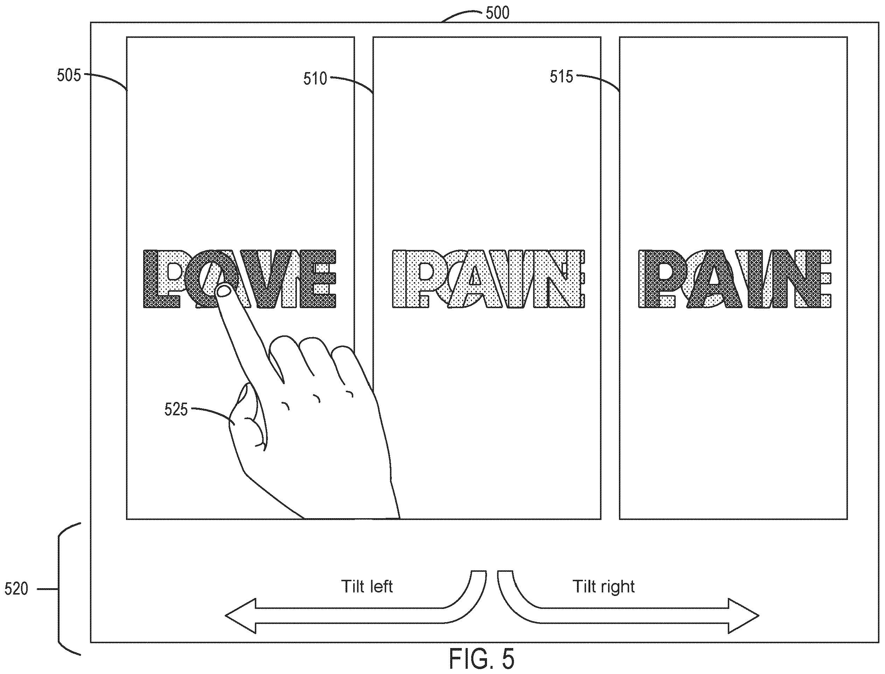

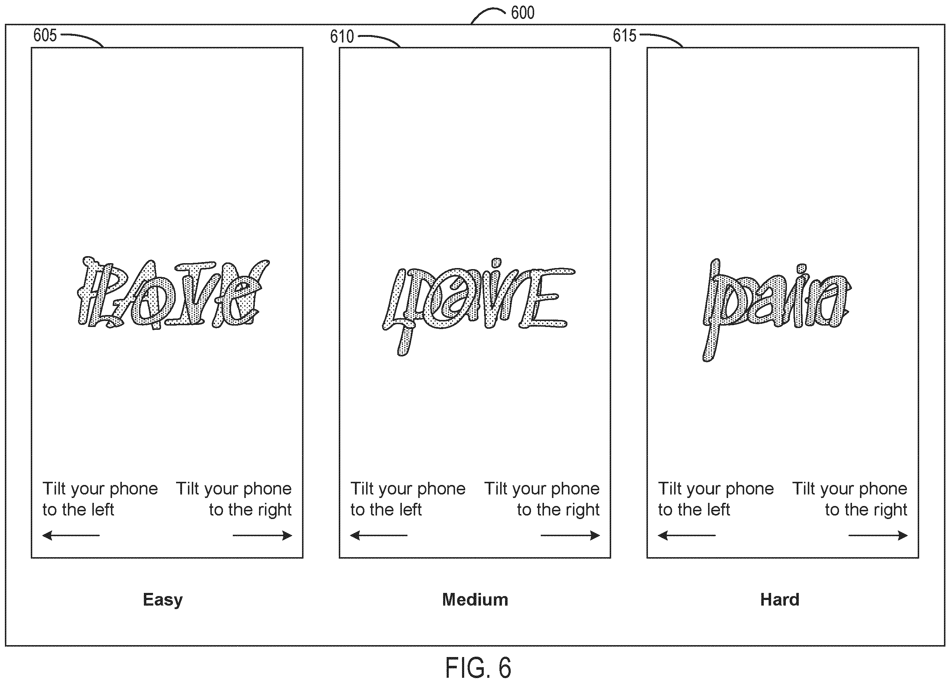

For the next provision of stimuli (e.g., in a subsequent round or session), the session manager 140 can identify or select a difficulty level to associate with the visual stimuli 175'A and 175'B (herein referred to as visual stimuli 175'). The level can be a level, score, or descriptor assigned to determine the presentation of the visual stimulus 175' by the stimuli selector 145. For example, the level can correspond to "easy," "medium," or "difficult," or the level can correspond to a numeric value, a title, or a time period, among others. The level can correspond to a time period. For example, a first level can include a first time period and a second level can include a second time period. The level can define one or more characteristics for presentation of the visual stimuli 175'. The characteristics can instruct the presentation of the stimuli 175'. For example, the characteristics can be visual characteristics defined by the level which affect the display of a visual stimulus or which affect the selection of a visual stimulus. For example, a first visual stimulus 175'A can correspond to an "easy" level, and thereby have a different presentation than the first visual stimulus 175'A corresponding to a "difficult" level. The presentation of the visual stimuli 175' can change depending on the assigned level. The set of visual stimuli in the interfaces depicted in FIG. 5 may correspond to the easier level, whereas the set of visual stimuli in the interfaces depicted in FIG. 6 may correspond to a more difficult level.

To identify or select the level for the visual stimulus 175', the session manager 140 may use a reaction time, response score 305, or correctness of a response 215, among others. The reaction time can correspond to the delay between the presentation of the visual stimuli 175' and the receipt of the response 215, or the performance of the interaction 220. For example, the session manager 140 can select a higher or more difficult level corresponding to a reaction time below a threshold level. The session manager 140 may select the level to include more or less stimuli 175' if the correctness of the prior response 215 is below a threshold value or a specified correctness.

In some embodiments, the session manager 140 can select the level to associate with the visual stimuli 175' based on the response score 305. For example, the session manager 140 may select a more difficult level for association with the visual stimuli 175' if the response score 305 is above a threshold. For example, the session manager 140 may select an easier level for association with the visual stimuli 175' if the response score 305 is below a threshold. In some embodiments, the performance evaluator 155 may select the level based on the reaction time and the correctness of any prior responses 215. In this manner, the user 210 can be presented with more difficult visual stimuli 175' to further the user's progress in the ABMT session.

In some embodiments, the session manager 140 may select the level based on the degree of compliance of the user 210. The session manager 140 may select a higher level for subsequent visual stimuli than associated with the previously presented visual stimuli if the user 210 has a high degree of compliance for the session. Conversely, the session manager 140 may select the same level for the subsequent visual stimuli if the user 210 exhibited a low degree of compliance for that level in the previously presented visual stimuli 175' during the session. The session manager 140 may use any combination of the visual stimuli 175', the user profile 170, the responses 215, the response score 305, or the interactions 220 to determine to select or modify a level for the stimulus 175'.

In some embodiments, the session manager 140 can select the level based on previous presentations of the visual stimuli 175'. The session manager 140 can select the level based on previous presentations of visual, auditory, or haptic stimuli, among others. For example, the session manager 140 can select the level based on the user failing to supply a correct interaction 220 for the previously presented visual stimuli 175'. The session manager 140 can select the level based on the response score 305. For example, if the response score 305 associated with the session is below a threshold score, the session manager 140 may assign an easier level for the subsequent presentation of the visual stimuli 175'.

With the determination of the level, the stimuli selector 145 may modify the presentation of the visual stimuli 175' based on the level for the visual stimuli 175'. The stimuli selector 145 may modify the presentation of the first stimulus 175'A, the second stimulus 175'B, a subsequent visual stimulus, or a combination thereof. The stimuli selector 145 can provide instructions to the application 125 for display of the visual stimuli 175'. The stimuli selector 145 or the application 125 may modify the presentation of the visual stimuli 175' during the presentation of the visual stimuli 175' or subsequent to the presentation of the visual stimuli 175'. For example, the stimuli selector 145 can modify the presentation of a first visual stimuli 175'A as it is presented on the user interface 130 by the application 125. For example, the stimuli selector 145 can modify the presentation of subsequent visual stimuli 175' during the same session or a subsequent session.

The stimuli selector 145 may modify the visual stimulus 175' using one or more visual characteristics associated with the assigned level. For example, a first level of "easy" may include a first characteristic indicating an opacity for a first visual stimulus 175'A. A second level of "difficult" may include a second characteristic indicating an opacity for a second visual stimulus 175'B. In some embodiments, the opacity associated with the second level may be less legible or discernable by the user 210 than the opacity associated with the first level. That is to say, the first stimulus 175'A with the "easy" level may be more legible, easier to read, or more easily discernable than the second stimulus 175'B with the "difficult" level. In this manner, one or more overlapping visual stimuli associated with an "easy" level may be easier to differentiate for the user 210 than one or more overlapping visual stimuli associated with a "difficult" level.

The interaction 220 with the visual stimulus 175' can iteratively cause the stimuli selector 145 to modify the presentation of the visual stimulus 175' based on the response score 305, and the levels determined by the session manager 140. As an illustrative example, the user 210 can provide an interaction 220 of tilting the user device 110 responsive to the visual stimuli 175'. As the user 210 tilts the user device 110 towards a word associated with the first visual stimulus 175'A and away from a word associated with the second visual stimulus 175'B, the word associated with the first visual stimulus 175'A can become more prominent or opaque and the word associated with the second visual stimulus 175'B can become less prominent or visible. The visual stimuli 175' can change in other manners. For example, responsive to a change in a characteristic of a level by the session manager 140, images or words associated with a visual stimulus 175' can change color, size, font, opacity, location, or alignment, among others. In this manner, depending on the response 215 provided by the user 210, the presentation of the visual stimuli 175' can iteratively change.

The session management service 105 may repeat the functionalities described above (e.g., processes 200 and 300) over multiple sessions. The number of sessions may be over a set number of days, weeks, or even years, or may be without a definite end point. By iteratively providing visual and auditory stimuli based at least on the response score 305, user profile 170, responses 215, or the settings 205, the user 210 may be able to receive content to help alleviate the bias towards stimuli associated with the condition. This may alleviate symptoms faced by the user 210, even when suffering from a condition which could otherwise inhibit the user from seeking treatment or even physically accessing the user device 110. Furthermore, from participating in the session when presented through the user interface 130 of the application 125, the quality of a human computer interactions (HCI) between the user 210 and the user device 110 may be improved.

Since the visual stimuli 175' are more related to the user's condition (e.g., cancer, MS, other chronic conditions) and associated with symptoms arising from attention basis due to the condition, the user 210 may be more likely to participate in the session when presented via the user device 110. This may reduce unnecessary consumption of computational resources (e.g., processing and memory) of the service and the user device 110 and lower the usage of the network bandwidth, relative to sending otherwise ineffectual or irrelevant visual stimuli 175'. Furthermore, in the context of a digital therapeutics application, the individualized selection of the visual stimuli 175' may result in the delivery of user-specific interventions to improve subject's adherence to the treatment. This may result in not only higher adherence to the therapeutic interventions but also lead to potential improvements to the user's condition.

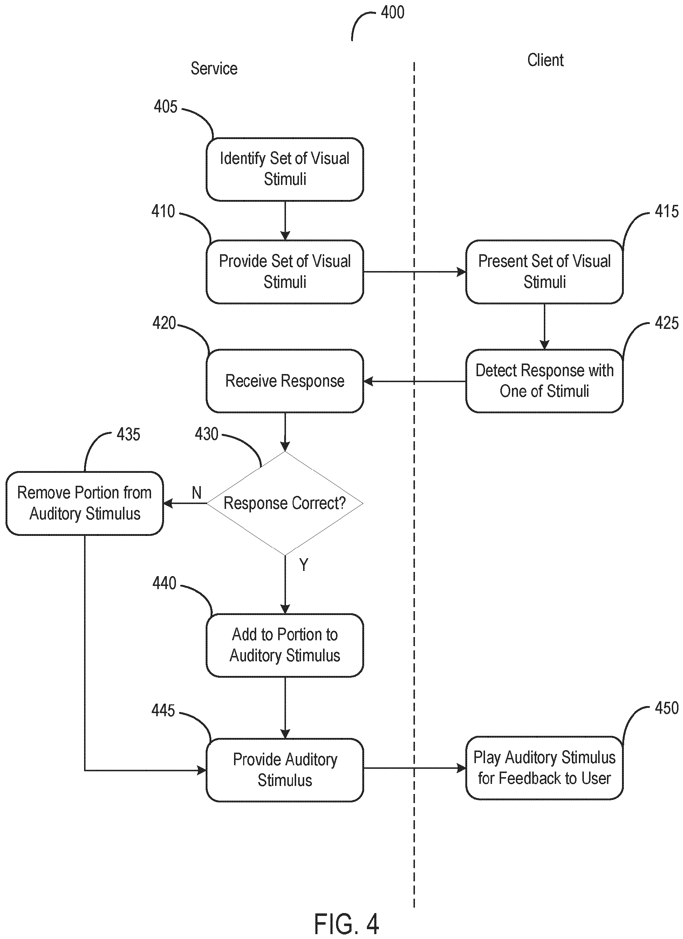

Referring now to FIG. 4, depicted is a flow diagram of a method 400 for presenting multimodal stimuli to address symptoms associated with condition. The method 400 may be implemented or performed using any of the components detailed herein, such as the session management service 105 and the user device 110, or any combination thereof. Under method 400, a service (e.g., the session management service 105) may identify a set of visual stimuli (405). The service may provide the set of visual stimuli to the client (e.g., the user device 110) (410). The client may present the set of visual stimuli using a display device of the client (415). Upon presentation of the stimuli, the application operating on the client may detect a response with one or more of the sets of visual stimuli (425). The service may receive the response (420). Upon receipt of the response, the service may determine whether the response is correct (430). If the response is not correct, the service may remove a portion from an auditory stimulus (435). If the response is correct, the service may add a portion to the auditory stimulus (440). The service may provide the auditory stimulus (445). The client may play the auditory stimulus for feedback to the user (450).

Referring now to FIG. 5, depicted is a screenshot of a sample set 500 of interfaces for presenting multimodal stimuli to address symptoms associated with conditions. The set 500 can include example user interfaces 505, 510, or 515 and a set of instructions 520. The set 500 can include the application 125 installed thereon. The application 125 may present on a user interface or display such as the user interface 130 of the user device 110. In some embodiments, the application 125 may present as a game, image, video, or interactive application 125 on the user device 110. A user (e.g., the user 210) may interact with the user interfaces 505-515. The user interfaces 505-515 may include one or more user elements, such as the UI elements 135, to accept input by the user 210 using at least one hand 525. The user 210 may press on a screen of the user device 110 to enter input, may manipulate or rotate the user device 110 to register (via a detection by an accelerometer, gyroscope, or other sensor associated with the user device 110) a movement of the user device 110.

The user 210 may manipulate the user device 110 according to the instructions 520. The instructions 520 can be presented on the user device 110 as a part of the application 125 operating thereon. Through manipulation of the user device 110 by the user 210 according to the instructions 520, each user interface 505-515 may display to the user 210 via the user device 110. For example, by tilting the user device 110 to the user's left, the user interface 505 may display, wherein the word "love" is more prominent than the word "pain." Conversely, the user 210 tilting the user device 110 to the user's right may cause the application 125 to more prominently display the word "pain," over "love." By maintaining the user device 110 in a neutral position, the client device 110 may display the user interface 510, in which the words "love" and "pain" are equally opaque. In this manner, through the user 210 providing an interaction 220 of tilting the phone, the different stimuli "love" and "pain" may present with different visual characteristics.

Referring now to FIG. 6, depicted is a screenshot of a sample set 600 of user interfaces for presenting multimodal stimuli to address symptoms associated with conditions. The set 600 can include example user interfaces 605, 610, or 615. The set 600 can include the application 125 installed thereon. The application 125 may present on a user interface or display such as the user interface 130 of the user device 110. In some embodiments, the application 125 may present as a game, image, video, or interactive application 125 on the user device 110. A user (e.g., the user 210) may interact with the user interfaces 605-615. The user interfaces 605-615 may include one or more user elements, such as the UI elements 135, to accept input by the user 210. The user 210 may press on a screen of the user device 110 to enter input, may manipulate or rotate the user device 110 to register (via a detection by an accelerometer, gyroscope, or other sensor associated with the user device 110) a movement of the user device 110.

The user interfaces 605-615 may correspond with a difficulty level. The user interface 605 may correspond with an "easy" difficulty level. The user interface 610 may correspond with a "medium" difficulty level. The user interface 615 may correspond with a "hard" difficulty level. The different difficulty levels associated with the user interfaces 605-615 may be determined by the session manager 140 in accordance with the systems and methods described herein. In this illustrative example, the increasing difficulty levels are associated with visual stimuli that may be more difficult to interpret by the user 210. For example, the user interface 615 depicts two stimuli ("love" and "pain") with similar fonts, coloration, and sizing, thereby rendering it more difficult or harder for the user 210 to distinguish between the two visual stimuli. Likewise, the user interface 605 depicts the same two visual stimuli, but in different fonts, sizes, and colors, thereby enabling an easier differentiation of the stimuli for the user 210.

Since the application operates on the subject's mobile device, or at least a mobile device that she can access easily and reliably, e.g., according to the predetermined frequency (e.g., once per day), the application provides real-time support to the subject. For example, upon receiving a request from the user to initiate a session, the application initiates in real-time, i.e., within at least a few milliseconds from receiving the request, a session. Such prompt guidance cannot be achieved via in-person visits, phone calls, video conferences or even text messages between the user and health care providers examining the user for multiple sclerosis or cancer. In this manner, the application is able to provide and customize tasks for the user based on the performance of the user. This can create an iteratively improving service for the user wherein overall bandwidth and data communications are minimized due to the increasing usefulness of each task.

### B. Method of Reducing Symptoms Associated with Attention Bias of Users Suffering from Conditions Affecting Cognitive Function

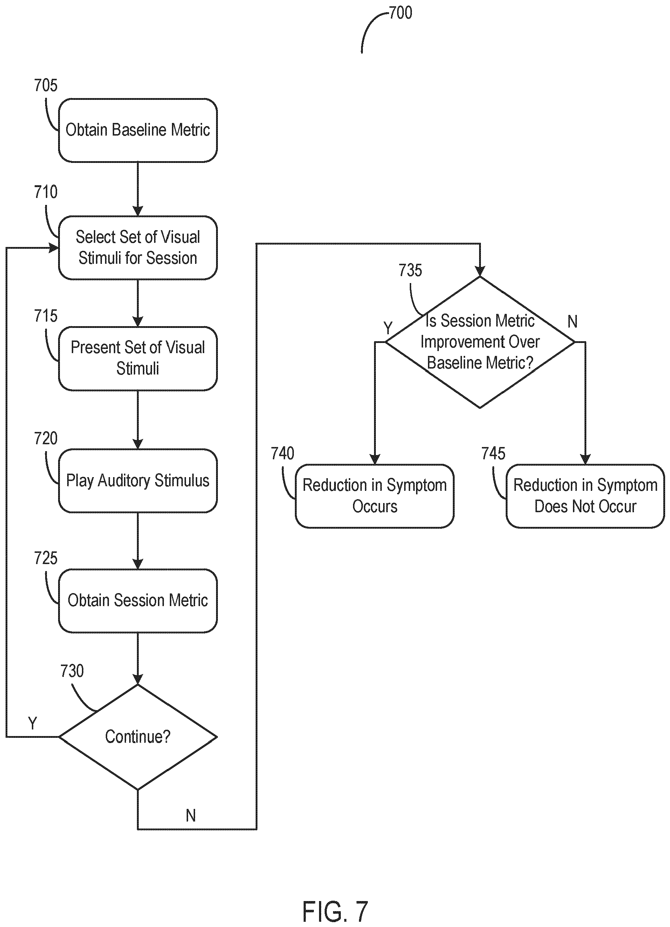

Referring now to FIG. 7, depicted is a flow diagram of a method 700 of reducing symptoms associated with attention bias of users suffering from conditions affecting cognitive function in need thereof. The method 700 may be performed by any components or actors described herein, such as the session management service 105 and the user device 110, among others. The method 700 may be used in conjunction with any of the functionalities or actions described herein in Section A, in Example 1 in Section B, or Appendix A. In brief overview, the method 700 may include obtaining a baseline metric (705). The method 700 may include selecting a visual stimulus for a session (710). The method 700 may include presenting the set of visual stimuli (715). The method 700 may include playing an auditory stimulus (720). The method 700 may include obtaining a session metric (725). The method 700 may include determining whether to continue (730). The method 700 may include determining whether the session metric is an improvement over the baseline metric (735). The method 700 may include determining that a reduction in a symptom has occurred, when the session metric is determined to have improved over the baseline metric (740). The method 700 may include determining that no reduction in the symptom has occurred, when the session metric is determined to have not improved over the baseline metric (745).

In further detail, the method 700 may include determining, identifying, or otherwise obtaining a baseline metric prior to any session (705). The baseline metric may be associated with a user (e.g., the user 210) at risk of, diagnosed with, or otherwise suffering from a condition. In some cases, the condition of the user may include multiple sclerosis (MS), with or without mobility limitations, due to muscle weakness or spasticity, fatigue, or loss of balance. The user with multiple sclerosis may be in relapse and steroid-free for at least a period of time (e.g., ranging between 3 days to 3 weeks) prior to the sessions. In other cases, the condition of the user may include cancer, such as lung cancer, colorectal cancer, skin cancer, breast cancer, ovarian cancer, leukemia, pancreatic cancer, or gastric cancer, among others. In other cases, the condition may include a psychogenic chronic pain condition, including but not limited to, fibromyalgia, rheumatoid arthritis and IBS.

"Cancer", which is also referred to herein as "tumor", is a known medically as an uncontrolled division of abnormal cells in a part of the body, benign or malignant. In one embodiment, cancer refers to a malignant neoplasm, a broad group of diseases involving unregulated cell division and growth, and invasion to nearby parts of the body. Non-limiting examples of cancers include carcinomas, sarcomas, leukemia and lymphoma, e.g., colon cancer, colorectal cancer, rectal cancer, gastric cancer, esophageal cancer, head and neck cancer, breast cancer, brain cancer, lung cancer, stomach cancer, liver cancer, gall bladder cancer, or pancreatic cancer. In one embodiment, the term "cancer" refers to a solid tumor, which is an abnormal mass of tissue that usually does not contain cysts or liquid areas, including but not limited to, sarcomas, carcinomas, and certain lymphomas (such as Non-Hodgkin's lymphoma). In another embodiment, the term "cancer" refers to a liquid cancer, which is a cancer presenting in body fluids (such as, the blood and bone marrow), for example, leukemias (cancers of the blood) and certain lymphomas.

Additionally or alternatively, a cancer may refer to a local cancer (which is an invasive malignant cancer confined entirely to the organ or tissue where the cancer began), a metastatic cancer (referring to a cancer that spreads from its site of origin to another part of the body), a non-metastatic cancer, a primary cancer (a term used describing an initial cancer a subject experiences), a secondary cancer (referring to a metastasis from primary cancer or second cancer unrelated to the original cancer), an advanced cancer, an unresectable cancer, or a recurrent cancer. As used herein, an advanced cancer refers to a cancer that had progressed after receiving one or more of: the first line therapy, the second line therapy, or the third line therapy.

The user may be on a medication to address the condition, at least in partial concurrence with the sessions. For multiple sclerosis, the medication may include beta interferons, glatiramer, cladribine, dimethyl fumarate, diroximel fumarate, fingolimod, monomethyl fumarate, ofatumumab, ozanimod, ponesimod, Siponimod, teriflunomide, alemtuzumab, mitoxantrone, or natalizumab, among others. For cancer, the user may have completed chemotherapy, radiation therapy, or immunotherapy for at least a period of time (e.g., ranging between 3 days to 18 months) prior to the sessions. The user may be of any demographic or trait, such as by age (e.g., an adult (above age of 18), late adolescent (between ages of 18-24)) or gender (e.g., male, female, or non-binary), among others.

The user may have one or more symptoms associated with an attention bias due to the condition affecting the user's cognitive function. The symptoms relevant to the condition may include chronic pain, fatigue, and emotion (e.g., depressed mood), among others. The cognitive function affected by the condition may include chronic nervous disease and cognitive impairment for multiple sclerosis and cognitive impairment resulting from cancer or the treatment of cancer, among others. The attention bias may include, for example, avoidance of stimuli or an activity related to the symptom; anxiety induced from stimuli associated with the symptom, multiple sclerosis, or cancer; or depression, among others.

The baseline measure may be obtained (e.g., by a computing system such the user device 110 or the session management service 105 or a clinician separately from the computing system) prior to the user being provided with any of the sessions via a digital therapeutics application (e.g., the application 125 or the Study App described herein). The baseline measure may identify or indicate a degree of severity of the symptom associated with an attention bias due to the condition affecting the user's cognitive function. Certain types of metrics may be used for both multiple sclerosis and cancer. For both conditions, the baseline metric may include, for example, a Patient Reported Outcomes Measurement Information System (PROMIS) value (e.g., PROMIS-29), brief pain inventory (BPI) value, a pain catastrophizing scale (PCS) value, a global rating of change (GRC) value, a user experience questionnaire value, and computerized assessment values, among others. Certain types of metrics may be used for one of multiple sclerosis or cancer. The baseline metric for multiple sclerosis may include a Symbol Digit Modalities Test (SDMT) value, and the baseline metric for cancer may include functional assessment of cancer therapy-cognitive function (FACT-Cog) value.

The method 700 may include identifying or selecting a set of visual stimuli (e.g., the visual stimuli 175) for a session (710). The computing system may select the set of visual stimuli based on settings (e.g., the setting 205 identifying a list of words to identify the visual stimuli from) of a user profile (e.g., the user profile 170) for the user and prior sessions if any have been previously provided to the user. The visual stimuli may include text, images, or video, and may be selected in accordance with attention bias modification training (ABMT). The set of visual stimuli may include at least one visual stimulus associated with the condition (or the symptom associated with the condition) and at least one other visual stimulus. The first visual stimulus (e.g., the first visual stimulus 175A) may be, for example, a pain-related visual stimulus, a threat-related visual stimulus, a condition-related visual stimulus, or otherwise negatively related visual stimulus, among others. The second visual stimulus (e.g., the second visual stimulus 175B) may be a neutral visual stimulus or a positive visual stimulus, among others.

The method 700 may include displaying, rendering, or otherwise presenting the set of visual stimuli (715). With the selection of the set of visual stimuli, the computing system may present the first visual stimulus at least partially overlapped with the second visual stimulus on a display. The user may be prompted or directed (e.g., via the display) to perform at least one interaction (e.g., the interaction 220) with one of the first visual stimulus or the second visual stimulus presented to the user. For instance, the computing system may display an indicator identifying which side of the display toward which to orient the device in order to select the visual stimulus. The computing system may monitor for the interaction with one of the visual stimuli. The interaction may include an orientation (e.g., tilting or moving) for the display of the device toward one side corresponding to one of the visual stimuli or a touch (e.g., a touch or click event) with one of the visual stimuli, among others. Upon detection, the computing system may identify (e.g., from the response 215) the visual stimulus of the set with which the user performed the interaction.

The method 700 may include presenting, outputting, or otherwise playing an auditory stimulus (e.g., the auditory stimulus 180) (720). The computing system may generate the auditory stimulus to provide feedback to the user based on the response. The computing system may determine whether the response is correct based on which visual stimulus the user performed the interaction. When the response identifies the interaction was with the first visual stimulus associated with the condition, the computing system may determine that the response is incorrect. The computing system may produce the auditory stimulus to provide negative punishment (e.g., by removing one or more portions 310) when the response is incorrect. When the response identifies the interaction was with the second visual stimulus, the computing system may determine that the response is correct. The computing system may produce the auditory stimulus to provide positive reinforcement (e.g., by adding one or more portions 310) when the response is correct. In some embodiments, the computing system may provide the auditory stimulus at least in partial concurrence with the presentation of the set of visual stimuli. The auditory stimulus may contain stereo audio, with audio played to one ear of the user different from the audio played to the other ear of the same user to effectuate binaural perception. The user may experience an improvement in fatigue symptoms associated with the condition, in response to binaural beats in the auditory stimulus.

The method 700 may include determining, identifying, or otherwise obtaining a session metric (725). The session metric may be obtained (e.g., by the computing system such the user device 110 or the session management service 105 or a clinician separately from the computing system) subsequent to the user being provided with at least one of the sessions via the digital therapeutics application. The session metric may identify or indicate a degree of severity of the symptom associated with an attention bias due to the condition affecting the user's cognitive function. The session metric may be of the same type of measurement as the baseline metric. Certain types of metrics may be used for both multiple sclerosis and cancer. For both conditions, the baseline metric may include, for example, a Patient Reported Outcomes Measurement Information System (PROMIS) value (e.g., PROMIS-29), brief pain inventory (BPI) value, a pain catastrophizing scale (PCS) value, a global rating of change (GRC) value, a user experience questionnaire value, and computerized assessment values, among others. Certain types of metrics may be used for one of multiple sclerosis or cancer. The baseline metric for multiple sclerosis may include a Symbol Digit Modalities Test (SDMT) value, and the baseline metric for cancer may include functional assessment of cancer therapy-cognitive function (FACT-Cog) value.

The method 700 may include determining whether to continue (730). The determination may be based on the set length (e.g., days, weeks, or years) of the trial or a set number of sessions to be provided to the user. For example, the set number of time instances may range between 2 to 8 weeks, relative to the obtaining of the baseline metric. When the amount of time from the obtaining of the baseline metric exceeds the set length, the determination may be to stop providing additional sessions. The method 700 may repeat from step 710, with the selection of the set of visual stimuli for the next session. The presentation of visual stimuli for the subsequent session may be altered, changed, or otherwise modified based on the response in the current session. In some embodiments, the computing system may determine, identify, or otherwise select a difficulty level based on the response from the user in the current or prior sessions. With the selection, the computing system may modify a visual characteristic (e.g., opacity or degree of overlaps) of the set of visual stimuli based on the difficulty level for the next session.

The method 700 may include identifying or determining whether the session metric is an improvement over the baseline metric (735). The improvement may correspond to an amelioration or a reduction in the symptoms associated with an attention bias due to the condition affecting the user's cognitive function. The improvement may be determined (e.g., by the computing system or a clinician examining the user) to have occurred when the session metric is increased compared to the baseline metric by a first predetermined margin or when the session metric is decreased compared to the baseline metric by a second predetermined margin. The margin may identify or define a difference in value between the baseline and session metrics at which to determine that the user shows reduction in the symptom or severity thereof. Whether the improvement is shown by increase or decrease may depend on the type of metric used to measure the user with respect to the condition or the symptom. The margin may also depend on the type of metric used, and may in general correspond to the difference in value showing noticeable difference to the clinician or user with respect to the symptom or showing a statistically significant result in the difference in the values between the baseline and session metrics.

The method 700 may include determining that a reduction in a symptom has occurred, when the session metric is determined to have improved over the baseline metric by the first or second predetermined margin (740). In some embodiments, the reduction in the symptom may occur when the session PROMIS value is increased from the baseline PROMIS value by the first predetermined margin. In some embodiments, the reduction in the symptom may occur when the session BPI value is decreased from the baseline BPI by the first predetermined margin. In some embodiments, the reduction in the symptom may occur when the session PCS value is decreased from the baseline PCS by the first predetermined margin. In some embodiments, the reduction in the symptom may occur when the session metric value is increased from the baseline metric value by the second predetermined margin, for a computerized cognitive assessment value. In some embodiments, the reduction in the symptom may occur when the session SDMT value is increased from the baseline SDMT value by the second predetermined margin, for the user with multiple sclerosis. In some embodiments, the reduction in the symptom may occur when the session FACT-Cog value is increased from the baseline FACT-Cog value by the second predetermined margin, for the user with cancer. In some embodiments, the reduction in the symptom may occur following a session of FACIT-Fatigue, FACT-G and/or HADS.

The method 700 may include determining that no reduction in the symptom has occurred, when the session metric is determined to have not improved over the baseline metric by the first or second predetermined margin (745). In some embodiments, the reduction in the symptom may not occur when the session PROMIS value is not increased from the baseline PROMIS value by the first predetermined margin. In some embodiments, the reduction in the symptom may not occur when the session BPI value is not decreased from the baseline BPI by the first predetermined margin. In some embodiments, the reduction in the symptom may not occur when the session PCS value is not decreased from the baseline PCS by the first predetermined margin. In some embodiments, the reduction in the symptom may not occur when the session metric value is not increased from the baseline metric value by the second predetermined margin, for a computerized cognitive assessment value. In some embodiments, the reduction in the symptom may not occur when the session SDMT value is not increased from the baseline SDMT value by the second predetermined margin, for the user with multiple sclerosis. In some embodiments, the reduction in the symptom may not occur when the session FACT-Cog value is not increased from the baseline FACT-Cog value by the second predetermined margin, for the user with cancer.

### Example 1: A Randomized, Controlled, Exploratory Basket Study to Evaluate Multimodal Multistable Modification of Biases for the Normalization of Attentional Biases and Improving Cognition in Adults with Multiple Sclerosis, Breast Cancer, and Lung Cancer

### 1. Synopsis

Indications: Multiple Sclerosis (MS); Breast Cancer and Lung Cancer

Introduction and Study Rationale: CT-100 (e.g., the application 125) is a platform that provides interactive, software based therapeutic components that may be used as part of a multimodal treatment in future software-based prescription digital therapeutics. One class of CT-100 components are Digital Neuro-activation and Modulation (DiNaMo^{™}) components. DiNaMo components target key neural systems (including but not limited to systems related to sensory-, perceptual-, affective-, pain-, attention-, cognitive control, social- and self-processing) to optimally improve a participant's health.

Multimodal Multistable Modification of Biases (or multimodal, multistable bias modification or MMBM) is a targeted cognitive training using Multimodal NeuroEnrichment Task (M-NET) principles in order to reduce symptoms relevant to multiple sclerosis (MS) and cancer. Biases to direct attention towards or away from certain stimuli can exacerbate a variety of symptoms in many different indications. For example, in chronic pain conditions, patients are more attentive to pain-related stimuli, which can lead to hypersensitization. Similarly, hypersensitivity to threat-related stimuli can exacerbate anxiety.

In Multimodal Multistable Modification of Biases, users are trained to re-orient attention towards positive stimuli, instead of stimuli that may cause them distress, such as pain-or threat-related stimuli. This is done in a novel format, tapping into multistable perception. Visual multistable perception, in the form of monocular rivalry, is used to implicitly retrain attentional biases. At the same time, auditory multistable perception, in the form of binaural beats,are used to both target fatigue symptoms and to work synergistically to enhance the attentional bias component.

Objectives: To evaluate trends in effect in comparison to Digital Control arm in pain interference, cognition and related outcomes (attentional bias and general quality of life [QoL]).

### Criteria for Evaluation

### Exploratory Endpoints

- Change from baseline to Week 4 in quality of life as measured by PROMIS-29+2 (Patient Reported Outcomes Measurement Information System)
- Change from baseline to Week 3 in pain severity and pain interference as measured by the Brief Pain Inventory (BPI)
- Change from baseline to Week 4 Pain Catastrophizing Scale (PCS)
- Change from baseline to Week 4 in indication-specific measures: Symbol Digit Modalities Test (SDMT) for MS; Functional Assessment of Cancer Therapy-Cognitive Function (FACT-Cog) for cancer
- Change from baseline to Week 4 in computerized cognitive assessment
- Proportion of participants with an improvement as measured by the Global Rating of Change (GRC) score at Week 4
- Engagement with the Study App as measured by metrics such as daily App usage and daily time in the Study App
- Experience with the Study App as assessed by the user experience survey and potential optional qualitative interviews after the treatment period

### Study Design

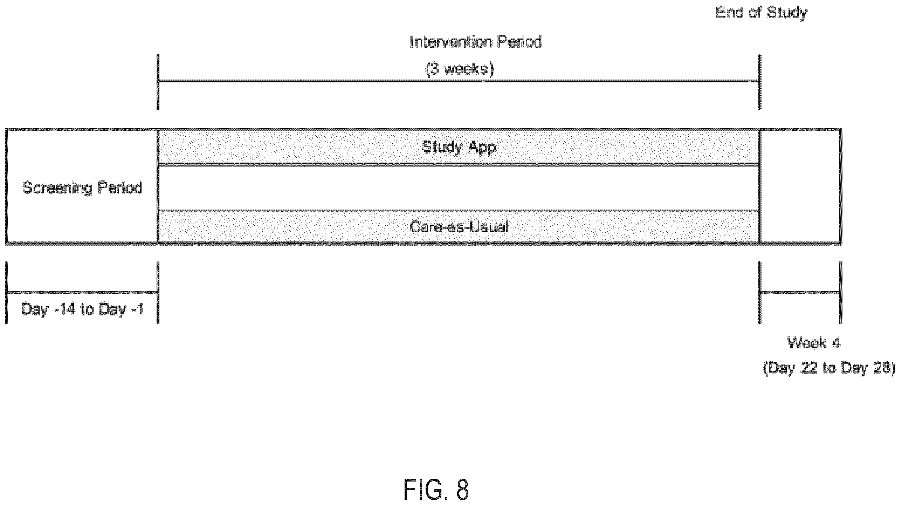

Referring now to FIG. 8, depicted is a timeline of the randomized, controlled, exploratory basket study to evaluate multimodal multistable modification of biases for the normalization of attentional biases and improving cognition in adults with multiple sclerosis, breast cancer, and lung cancer.

### Screening Period (Day -14 to Day -1)

- Participants were screened for up to 14 days, including electronic informed consent. Assessments during this period were performed remotely according to the Schedule of Activities and Assessments (SoA).
- Participants were considered eligible if they meet all inclusion criteria and no exclusion criteria, based on investigator assessment.
- Screening and the Baseline Visit may have occurred on the same day.

### Baseline (Day 1)

- Participants were contacted for a Baseline Visit to review and confirm eligibility and perform identity verification. Assessments were performed according to the SoA.
- Eligible participants were randomized during a virtual study visit on Day 1. Participants will be randomized 1: 1 (Study App:Control) within the following 4 cohorts: MS patients without mobility limitation; MS patients with mobility limitation; breast cancer patients; lung cancer patients. Participants randomized to the Study App treatment group downloaded and activated the app onto their personal primary smart phone.

### Intervention Period (Day 1 to Day 28)

- Study App group participants utilized an app-based daily brain exercise for 7 minutes a day for 4 weeks.
- Control group participants received the Digital Control and did not receive any study intervention.
- Assessments occurred every week throughout the intervention period for both groups according to the SoA.
- Participants assigned to the Study App group completed the user experience questionnaire at the end of the Intervention Period.

### Follow-up Period (Up to 1 Week)

- Participants completed follow-up assessments according to the SoA.
- Participants did not perform any activities within the Study App.
- A subset of participants completed an optional qualitative interview between Day 29 and Day 35.

### Planned Number of Participants: 189 total participants were enrolled in the study:

- 107 participants total for MS, with at least 53 in the DiNaMo 4 arm and at least 54 in the control group,
- 82 participants for BC, with at least 41 in the DiNaMo 4 arm and at least 41 in the control group.

### Study Entry Criteria

Inclusion Criteria: A participant was eligible for entry into the study if all of the following criteria were met:
1. Fluent in written and spoken English, confirmed by ability to read, understand, and sign the informed consent form
2. Lives in the United States
3. Adult between 22 and 65 years old
4. Meets indication-specific including criteria, as reported by the study participant with adequate clinical documentation (to be provided to the study team upon request)
5. Has an active email address and is willing and able to receive and respond to email messages
6. Has access to an internet connection during the study duration
7. Has an active bank account to receive study compensation, or is willing to create one
8. Willing and able to comply with study protocol and assessments
9. Is the sole user of smart phone for the duration of the study
10. Is willing and able to receive Short Message Service (SMS) text messages and notifications on their smartphone
11. Has access to operating headphones

Exclusion Criteria: A participant was not eligible for study entry if any of the following criteria were met:
1. Visual, dexterity or cognitive deficit so severe that it precludes the use of an App-based reaction time-based activity per investigator judgment
2. Severe psychiatric disorder involving a history of psychosis (e.g., schizophrenia, bipolar disorder, severe personality disorders)
3. Psychiatric hospitalization in the past 6 months
4. Participation in any research study (including studies on psychotherapy, mindfulness, cognitive training or pharmacological treatment) during the past 3 months
5. Initiation or change in primary disease-specific medication within 30 days prior to entering the study
6. Planning the introduction of new therapies (including studies on psychotherapy, mindfulness, cognitive training or pharmacological treatment) during the study duration (6 weeks)
7. Anticipating a change in current pharmacological or psychotherapy treatment regimen during the study period (6 weeks)
8. Self-reported substance use disorder within the past 1 year
9. Severe neurological disorders impairing brain function (e.g., previous stroke, dementia, primary brain function, brain metastases, Alzheimer's disease, Parkinson's disease, history of significant head trauma followed by persistent neurologic deficits or known structural brain abnormalities)
10. Mild, moderate, severe or profound unilateral or bilateral hearing loss

### Test Product and Mode of Administration DiNaMo 004

Eligible participants will download and install the Study App onto their own smartphone at the Baseline Visit (Day 1).

### Treatment Regimen: Study App: Daily use for 3 weeks (7 minutes per day)

Study Duration: Participation in the study will last for up to 6 weeks:
- Screening Period: Up to 2 weeks
- Intervention Period: 3 weeks
- Follow-up: Up to 1 week

Sample Size: There was 107 participants for MS, at least 53 DiNaMo 4 arm and at least 54 in a control group, and at least 80 participants for BC, at least 41 in the DiNaMo 4 arm and at least 41 in the control group. These sample sizes were sufficient to measure the changes with relative precision.

Statistical Analysis: Change from baseline to Week 3 for all the PROMIS-29+2 domains, BPI pain severity and BPI pain interference, PCS subcategories and total score, as well as the computerized cognitive assessment, were summarized using descriptive statistics. An Analysis of Covariance (ANCOVA) model was implemented to assess the difference between the 2 treatment arms. The model included the change from baseline as the dependent variable, and include treatment arm, indication and baseline value as covariates in the model. A 95% confidence interval (CI) and 2-sided p-value for the null hypothesis of no difference between the 2 arms were provided. Least-squares (LS) means for the treatment effect was provided. In addition, LS means, 95% CI and p-value for the null hypothesis of no change from baseline, were provided for each treatment arm.

For the SDMT and FACT-Cog, which are indication specific, changes from baseline were summarized using descriptive statistics. In addition, for the change from baseline to Week 3 within each treatment arm, a 95% CI and a 2-sided p-value for the null hypothesis of no difference between the treatment arms were provided. The CI and the p-value were based on t-distribution for 2 samples.

A 95% CI for the change from baseline to Week 3 within each arm was provided, as well as a 2-sided p-value for the null hypothesis of no change from baseline using t-distribution for one sample (pre-post).

The number and percentage with an improvement as measured in the GRC (improvement is a score greater than 1) was summarized by treatment arm. Comparison between the treatment arms was conducted using Cochran-Mantel-Haenszel stratified by indication.

For the remaining exploratory endpoints as well as for the safety parameters, only descriptive statistics were used.

### 2. Individual Indications

### A. Multiple Sclerosis Without Mobility Limitations

### Indication: Multiple Sclerosis (MS)

### Criteria for Evaluation

### Additional disease-specific scales:

- Symbol Digit Modalities Test (SDMT)

### Study Entry Criteria

Inclusion Criteria:
1. Self-reported clinical diagnosis of multiple sclerosis with adequate clinical documentation (to be provided to study team upon request)
2. Relapse and steroid-free for 30 days prior to screening
3. Self-reported pain for at least the last 3 months, which started following MS diagnosis.

Exclusion Criteria:
1. Self-reported history of cancer diagnosis
2. Unable to walk 300 meters (328 yards) without aid

### B. Multiple Sclerosis With Mobility Limitations

### Indication: Multiple Sclerosis (MS)

### Criteria for Evaluation

Additional disease-specific scales:
- Symbol Digit Modalities Test (SDMT)

### Study Entry Criteria

Inclusion Criteria:
1. Self-reported clinical diagnosis of multiple sclerosis with adequate clinical documentation (provided to study team upon request)
2. Relapse and steroid-free for 30 days prior to screening
3. Unable to walk 300 meters (328 yards) without aid
4. Self-reported pain for at least the last 3 months, which started following MS diagnosis

### Exclusion Criteria

### 1. Self-reported history of cancer diagnosis

### C. Breast Cancer

### Indication: Breast Cancer

### Criteria for Evaluation:

Additional disease-specific scales:
- Functional Assessment of Cancer Therapy-Cognitive Function (FACT-Cog)

### Study Entry Criteria Inclusion Criteria

1. Has a history of breast cancer (any stage) as reported by study participant with adequate clinical documentation (to be provided to study team upon request)
2. Has completed cancer chemotherapy treatment for at least 3 months, within the 9 months to 5 years prior to screening
3. Self-reported pain for at least the last 3 months, which started following cancer therapy

### Exclusion Criteria

### 1. Self-reported history of clinical diagnosis of multiple sclerosis

### D. Lung Cancer

### Indication: Lung Cancer

### Criteria for Evaluation

Additional disease-specific scales:
- Functional Assessment of Cancer Therapy-Cognitive Function (FACT-Cog)

### Study Entry Criteria Inclusion Criteria

1. Has a history of lung cancer (any stage) as reported by study participant with adequate clinical documentation (to be provided to study team upon request)
2. Has completed cancer chemotherapy treatment for at least 3 months, within the 9 months to 5 years prior to screening
3. Self-reported pain for at least the last 3 months, which started following cancer therapy

### Exclusion Criteria

1. Self-reported history of clinical diagnosis of multiple sclerosis *3. Schedule of Activities and Assessments*

| **Schedule of Activities and Assessments** | | | | | | |
|---|---|---|---|---|---|---|
| **Study Day and Visit Window** | **Screening Period** | **Intervention Period** | | | | **Follow-Up** |
| | **Screening** | **Baseline** | **Week 1** | **Week 2** | **Week 3/EOS/ET** | **Week 4** |
| | **Day -14 to Day -1** | **Day 1** - **3 days** | **Day 7 + 3 days** | **Day 14 + 3 days** | **Day 21** + **3 days** | **Day 22 to Day 28** |
| **Study Visit Number** | **n/a** | **1** | **2** | **3** | **4** | **5** |
| **Remote Visit** | | **X** | | | | |
| Informed Consent | X | | | | | |
| Demographics | X | | | | | |
| ID Verification | | X | | | | |
| Inclusion/Exclusion Criteria | X | X | | | | |
| Randomization | | X | | | | |
| Study App Installation and Activation^{a} | | X | | | | |
| Computerized Assessments (Millisecond) | | X | | | X | |
| PROMIS-29+2 | | X | | | X | |
| Brief Pain Inventory (BPI) | | X | X | X | X | |
| Pain Catastrophizing Scale (PCS) | | X | | | X | |
| Global Rating of Change (GRC) | | | | | X | |
| Additional Indication-specific Assessments | | X | | | X | |
| User Treatment Experience (quantitative survey)^{a} | | | | | X | |
| Optional User Interview^{a} | | | | | | X |
| Study App Engagement | X | | | | | |
| Study App Deactivation^{a} | | | | | X^{a,b} | |
| Concomitant Medications^{c} | X | X | X | X | X | X |
| **Study Visit Number** | **n/a** | **1** | **2** | **3** | **4** | **5** |
| Adverse Events (AEs) Review | X | X | X | X | X | X |

| | | | | | | |
|---|---|---|---|---|---|---|
| Abbreviations: AE = adverse event; app = application; BPI = Brief Pain Inventory; EOS = End of Study; ET = early termination; GRC = Global Rating of Change; PCS = Pain Catastrophizing Scale, PROMIS-29+2 = Patient Reported Outcomes Measurement Information System a. Only for participants in a treatment group b. After App-based assessments c. All medications that are related to the primary indication or are psychoactive | | | | | | |

### 4. Introduction

### A. Multimodal Multistable Modification of Biases

Multimodal Multistable Modification of Biases (multimodal, multistable bias modification or MMBM) is a targeted cognitive training using Multimodal NeuroEnrichment Task (M-NET) principles in order to reduce symptoms relevant to multiple sclerosis (MS) and cancer, including fatigue, pain, and mood-related symptoms. In Multimodal Multistable Modification of Biases, users are trained to re-orient attention towards positive stimuli, instead of things that may cause them distress, such as pain- or threat-related stimuli, through a multistable perception phenomenon called monocular rivalry. Simultaneously, binaural beats are utilized to target fatigue symptoms and work synergistically by providing a second modality of multistable perception.

### B. Attentional Biases

Individuals with a variety of indications, such as MS or various types of cancer, tend to present with problems in both pain and mood. These problems can be exacerbated by attentional biases related to pain- or threat-related stimuli. Individuals with chronic pain may be more attentive to pain-related stimuli, which can worsen the condition via fear-avoidance. Individuals may avoid stimuli or activities that are perceived to potentially cause pain, which can decondition the body and lead to further disability exacerbating the condition. With threat-related stimuli, individuals with anxiety often automatically seek out any signs of threat. This threat causes a feedback loop that perpetuates feelings of anxiety. This can include indication-related stimuli: for example, cancer survivors with a fear of cancer recurrence can be biased towards cancer-related stimuli. Likewise, as pain and anxiety are highly comorbid and share similar neurocircuitry alterations, targeting biases related to both are likely to have a synergistic effect. Finally, individuals with depression often display an attentional bias towards negative emotional information, which can contribute to rumination and thereby exacerbate symptoms. Together, these various attentional biases compounded can become quite debilitating.

Common symptomology for MS includes pain and disturbances of mood, which are both targeted by this treatment. There is also a significant relationship between high levels of pain and anxiety, for which Multimodal Multistable Modification of Biases could be particularly beneficial.

Individuals with cancer and cancer survivors both demonstrate an attentional bias for cancer-related words. Studies have also found evidence of biases towards threat and negative emotions. Attempts to change these automatic associations have demonstrated some success. As cancer patients also experience pain and mood symptoms, Multimodal Multistable Modification of Biases is expected to be highly relevant for this patient population.

### C. Cognitive Impairment

Cognitive impairments are common in several physical and mental disorders, either due to primary symptoms associated with a disease/disorder or associated with treatment for specific diseases (e.g., chemotherapy in cancer) or both. Cognitive impairments are also commonly related to problems in emotion regulation and mood problems. The combination of cognitive deficits and mood symptoms is distressing and disabling for patients, can negatively affect their quality of life and can result in increased health-care utilization.

For example, in multiple sclerosis (MS), a chronic central nervous disease, cognitive impairments are very common, with up to 70% of MS patients experiencing cognitive deficits. Deficits can affect several cognitive domains, with attention and processing speed being commonly affected. Patients living with MS experience these cognitive deficits as disabling and stressful, and there is currently a lack of treatment options to improve these challenges. Computerized cognitive training has been repeatedly demonstrated to be effective at correcting cognitive impairment in MS. This training can have widespread benefits outside of its cognitive domain. For example, cognitive training has shown beneficial effects on memory, semantic fluency, processing speed, and executive functioning. Benefits can also extend outside of cognitive improvements as well. For example, other studies have found beneficial effects on depression, daily life activities, expanded disability status scale score, and fatigue.

In cancer patients, tumor activity and related inflammatory processes and/or cancer-related treatments can impact cognitive functioning. Cancer-related cognitive impairment occurs in up to 75% of cancer patients, and for up to 35% of those experiencing impairment, this can last for months or years post-treatment. With the rising numbers of cancer survivors, lasting cognitive impairments due to cancer and cancer treatment will further increase. Similar to MS, computerized cognitive training has shown efficacy in general cancer patients, breast cancer patients, and cancer survivors.

Fatigue is also a debilitating symptom to both indications and can heavily contribute to problems with cognition. Up to 97% of MS patients have significant fatigue, and up to 40% describe it as the most disabling symptom of their disease; in fact, the presenting symptom for 1 in 3 patients diagnosed was fatigue. Fatigue is one of the most common cancer symptoms, also often being the presenting symptom, and this can result both from treatment, such as chemotherapy (with a prevalence of 75-100%), or from the disease itself (especially in the final stages). One study even found that 37% of cancer survivors still did not have their energy return to a satisfying level, even years later.

### D. Binaural Beats

In binaural beats, a single tone frequency is played in each ear, and the frequency difference between the two that can be detected is called the "beat". The theory behind binaural beats is that rhythmic sensory stimulation could entrain neural oscillations, similar to noninvasive techniques like transcranial magnetic stimulation and transcranial direct current stimulation. Studies have found beneficial effects of binaural beats on depression and anxiety symptoms, memory, attention, and cognition, fatigue and pain perception.

### E. The Study Application (App)

The Multimodal Multistable Modification of Biases CT-100 Digital Neuro-activation and Modulation (DiNaMo) component (e.g., the application 125) uses implicit training to redirect and strengthen attention processes. This intervention can help improve cognitive capabilities as well as redirect attentional biases to make participants less sensitive to distressing stimuli. Preliminary studies have indicated success in computerized cognitive training of MS and cancer, as well as success in retraining attentional biases in pain, threat, and mood. It is likely that Multimodal Multistable Modification of Biases may improve both cognitive capabilities as well as attentional biases present in multiple sclerosis and cancer.

### F. Digital Control

The Digital Control group downloaded the Digital Control App and completed a version of the task designed to be inert in the Study Application. This included only selecting between a set of neutral words, based on personal preference, and did not include any attention-bias-capturing stimuli. Additionally, all instances of multistable perception were removed, including the removal of all auditory elements.

### G. Study Rationale

Multimodal Multistable Modification of Biases is a novel intervention using Multimodal NeuroEnrichment Task (M-NET) principles in order to reduce symptoms relevant to MS and cancer. Attentional biases to a variety of attention-capturing stimuli can exacerbate a variety of symptoms in many different indications. For example, in conditions, patients are more attentive to pain-related stimuli, which can lead to hypersensitization. Similarly, hypersensitivity to threat-related stimuli can exacerbate anxiety.

In Multimodal Multistable Modification of Biases, users are trained to re-orient attention towards positive stimuli, instead of stimuli that may cause them distress, such as pain-or threat-related stimuli. This is done in a novel format, tapping into multistable perception. Visual multistable perception, in the form of monocular rivalry, is used to implicitly retrain attentional biases. At the same time, auditory multistable perception, in the form of binaural beats, will be used to both target fatigue symptoms and to work synergistically to enhance the attentional bias component.

The purpose of the proposed study is to evaluate initial effects of the DiNaMo component (the Study App) on measures of attentional bias, attentional impairment and related outcomes (general quality of life [QoL]) in a variety of conditions, such as MS, breast cancer and lung cancer. Results derived from this research could be used as components within future digital therapeutics.

### H. CT-100 Platform

The CT-100 platform provides interactive, software based therapeutic components that may be used as part of a multimodal treatment in future software-based prescription digital therapeutics. One class of CT-100 components are DiNaMo components. DiNaMo components target key neural systems (including but not limited to systems related to sensory-, perceptual-, affective-, pain-, attention-, cognitive control, social- and self-processing) to optimally improve a participant's health.

### 3. Objectives And Endpoints

The study objectives were:
- To evaluate trends in effect in comparison to the Digital Control arm in pain interference, cognition, and related outcomes (attentional bias and general QoL)
- To explore feasibility of an at-home digital Multimodal Multistable Modification of Biases visual search task (Study App), including engagement and experience with the Study App in participants
- To evaluate the safety of the Study App

The study endpoints to support these objectives are listed in Table 1.

**Table 1: Study Endpoints**

| **Exploratory Endpoints** | |
|---|---|
| ● | Change from baseline to Week 3 in quality of life as measured by PROMIS-29+2 (Patient Reported Outcomes Measurement Information System) |
| ● | Change from baseline to Week 3 in pain severity and pain interference as measured by the Brief Pain Inventory (BPI) |
| ● | Change from baseline to Week 3 Pain Catastrophizing Scale (PCS) |
| ● | Change from baseline to Week 3 in indication-specific measures: Symbol Digit Modalities Test (SDMT) for MS; Functional Assessment of Cancer Therapy-Cognitive Function (FACT-Cog) for cancer |
| ● | Change from baseline to Week 3 in computerized cognitive assessment |
| ● | Proportion of participants with an improvement as measured by the Global Rating of Change (GRC) score at Week 3 |
| ● | Engagement with the Study App as measured by metrics such as daily App usage and daily time in the Study App |
| ● | Experience with the Study App as assessed by the user experience survey and potential optional qualitative interviews after the treatment period |

### 4. Study Design

### A. Scientific Rationale for Study Design

This study was designed to evaluate the initial effects of the Multimodal Multistable Modification of Biases CT-100 component (the Study App) on attentional biases and cognitive deficits in attention as compared to Digital Control.

Participants were assessed based on validated standard participant-rated outcomes. Participant engagement with the Study App was evaluated based on participant usage data captured within the Study App. Participants were be evaluated for safety throughout the duration of the study. The scales and assessments are described herein.

### B. End of Study Definition

The end of the study is defined as the date of the last contact, or the date of final contact attempt, for the last participant completing or withdrawing from the study. For the purposes of this study, participants who complete the trial assessments at Day 21 (+3) (Week 3) were defined as trial completers.

### 6. Study Intervention(s) and Concomitant Therapy

### A. Study Intervention(s) Administered

The CT-100-D-004 Study App (e.g., the application 125) was administered to participants with the study intervention.

### B. CT-100

The study intervention under evaluation is the Multimodal Multistable Modification of Biases CT-100 component, a digital mobile application. Participants randomized to this group downloaded and installed the Study App onto their own smartphone at the Baseline Visit (Day 1) and use the Study App daily for Multimodal Multistable Modification of Biases training over the 3-week intervention period.

### C. Control

The Digital Control group downloaded the Digital Control App and completed a version of the task designed to be inert in the Study Application. This included only selecting between a set of neutral words, based on personal preference, and did not include any attention-bias-capturing stimuli. Additionally, all instances of multistable perception were removed, including the removal of all auditory elements.

### D. Preparation/Handling/Storage/Accountability

Detailed instructions for the download, installation, activation, use of, and disposition of the Study App were described in the CT-100-D-004 Study App Site Instructions.

Generally:
1. Only participants enrolled in the study received study intervention.
2. The site personnel must confirm download and activation of the Study App.
3. The Study App automatically became inert after the completion of Week 3. Site
personnel informed participants who complete the study or early terminate to uninstall the Study App.

### E. Concomitant Therapy

Participants continued to use their prescribed therapies while enrolled in this study. Participants self-reported any changes to all concomitant medications that are related to the primary indication or are psychoactive through the end of the Follow-up Period.

### 7. Study Assessments and Procedures

Study assessments and procedures, including their timing, are summarized in the SoA. Adherence to the study design requirements, including those specified in the SoA, was essential and required for study conduct. Protocol waivers or exemptions are not allowed. Every effort should be made to ensure that the protocol required assessments and procedures are completed as described. All the clinician-administered scales, if applicable, were administered by individuals who have been appropriately trained. Study assessments are described below.

The following efficacy assessment scales were used in this trial at the times as provided in the SoA. A description of the scales and the respective scoring algorithms for all endpoints will be provided in the Statistical Analysis Plan (SAP).

### A. Patient Reported Outcomes Measurement Information System-29+2 (PROMIS-29+2) Profile v2.1

PROMIS-29 is part of the Patient Reported Outcomes Measurement Information System (PROMIS). PROMIS-29 is a short form assessment that contains 4 items from each of 7 PROMIS domains (Anxiety, Physical Function, Pain Interference, Fatigue, Sleep Disturbance, Depression and Ability to Participate in Social Roles and Activities) plus one pain intensity question (0-10 numeric rating scale) and 2 cognitive functioning questions. The PROMIS-29 is universal rather than disease-specific (i.e., can assess health from patients regardless of disease or condition) and is intended for adults (ages 18+). Scores were produced for all 7 domains. The domains were assessed over the past 7 days. The PROMIS-29 has been widely administered and validated in a range of populations and settings. This electronic questionnaire was completed by the participant. It takes approximately 7 minutes to complete.

The PROMIS 29+2 also permits the calculation of the PROMIS Preference (PROPr) score. This allows for the calculation of a single Quality of Life summary score using the PROMIS subscales. The calculation lies on a scale from 0 (dead) to 1 (full health).

### B. Brief Pain Inventory (BPI)

The BPI is a self-report measure used for clinical trials. The BPI has 32 items to assess pain severity and interference using Numerical Rating Scale (NRS 0-10), pain location, pain medications, and amount of pain relief in the past 24 hours. This measure has had excellent test-retest reliability and internal consistency in chronic pain studies. This questionnaire was completed electronically by the participant. It took approximately 5 minutes to complete.

The BPI interference subscale has seven items, each item rated using a Numerical Rating Scale (NRS 0-10). The BPI interference subscale aims to assess how much pain impacts daily functions. This measure was used for both acute and chronic pain. This questionnaire was completed electronically by the participant using the standard 24 hours. It took approximately one minute to complete.

### C. Pain Catastrophizing Scale (PCS)

The PCS is a reliable and valid 13-item self-report measure used to assess catastrophic thinking relating to pain and is intended for adults (ages 18-64). The PCS consists of 5-point Likert scales across 3 subscales: Rumination (4 items), Magnification (3 items), and Helplessness (6 items). The subscales can be scored separately, or they can be summed to provide a total score. This questionnaire was a survey completed electronically by the participant. It took approximately 5 minutes to complete.

### D. Rating of Change (GRC)

The GRC is a self-reported, single item 10-point Likert scale used to assess the participant's rating of overall improvement with their indication after the study intervention. This item was completed electronically by the participant.

### E. User Experience Questionnaire and Optional Qualitative Interview

The User Experience Questionnaire is a questionnaire to understand participants' experience with the Study App during the intervention phase. The questionnaire asked questions related to the perceived enjoyment, challenges, and related user experience and does not contain questions related to clinical outcomes. This questionnaire was completed electronically by the participant. It took approximately 7 minutes to complete.

Additionally, a subset of participants may have participated in phone or videoconference-based qualitative user interviews. These interviews gathered additional information about the users' experience with the app, such as favorite app features, usability of the features, challenges related to the interventions, or any other feedback from regularly interacting with the app.

### 8. Statistical Considerations

### A. Statistical Hypotheses

This study was exploratory in nature and no formal statistical hypotheses is specified.

### B. Sample Size Determination

There was at least 100 participants for MS, at least 50 without mobility limitations and at least 50 with mobility limitations, and at least 80 participants for oncology, at least 40 with breast cancer and at least 40 with lung cancer.

### C. Analysis Sets

For the purposes of analysis, the following analysis sets are defined:

| **Analysis Set** | **Description** | |
|---|---|---|
| Enrolled | All participants who signed the ICF. This analysis set was used for disposition. | |
| Intent-To-Treat (ITT) | All randomized participants, based on the assigned intervention in the randomization and recorded in the database, regardless of successful activation or use of the Study App. Participants treated without being randomized were not be considered for the ITT Set. This analysis set was used as a supportive analysis for some of the efficacy endpoints. | |
| Modified Intent-to-Treat (mITT) | All ITT participants, based on the assigned intervention in the randomization and recorded in the database who have evaluable baseline measurements, for whom the Study App was activated and who have used the application at least once, with evaluable baseline measurements. This was the main analysis set for all efficacy analysis and was used to summarize baseline disposition and demography. | |
| Per Protocol (PP) | Participants with the following deviations were excluded from the PP set: | |
| | | 1. Participants who received intervention different from the randomized intervention |

| | | |
|---|---|---|
| | | 2. Participants who do not meet the inclusion/exclusion criteria |
| | | 3. Participants who meet discontinuation criteria but have not discontinued |
| | Additional criteria for exclusion from the PP set may be defined in the SAP. | |
| Safety | All participants who were randomized. Participants were analyzed according to the intervention they received. This analysis set was used for all safety analyses. | |

### D. Statistical Analyses

The SAP was finalized prior to database lock, and it included a more technical and detailed description of the statistical analyses described in this section. This section is a summary of the planned statistical analyses of the most important endpoints.

### E. General Considerations

Descriptive statistics included means and standard deviations, medians, as well as minimum and maximum values for continuous variables. For categorical variables, Ns and percentages were reported for all variables where applicable. Descriptive statistics tabulations were done by pooled treatment arms and by treatment arm within each indication.

All analyses are exploratory in nature. 95% confidence intervals and statistical tests were performed for some of the efficacy endpoints and were detailed as part of the description of planned analysis for each of the endpoints.

Data capture methods were programmed to minimize missing data (e.g., an answer must be given to advance to the next assessment question). The SAP addressed handling of incomplete questionnaires in which there were domain or total scores missing. In case that full questionnaire or a visit are missing, no imputations were done and only observed data will be included in the analysis. No adjustment for multiplicity was planned.

### F. Participant's Disposition

This analysis was done on the Enrolled Set. The number of participants who were enrolled, screen failures, randomized and completed the study was presented. The number of subjects in each of the analysis sets was presented by treatment group within each indication cohort.

### G. Demographics and Baseline Characteristics

In order to assess the comparability of the two arms at baseline, demographic and baseline characteristics data were summarized by treatment group. These summaries were presented for the mITT Set. If there was a difference of more than five participants in the total number of participants between the mITT and the Safety, ITT and PP analysis sets, the summaries were presented for these sets.

### H. Exploratory Endpoints

This was an exploratory study. Certain endpoints apply to all participants: PROMIS-29+2, BPI, PCS, and GRC. The analysis of those endpoints was performed by treatment arm using the mITT Set. The analysis was repeated for the PP Set.

For PROMIS, the absolute value and change from baseline to Week 3 for the 7 core domains, pain intensity question, cognitive functioning domain, and PROPr summary score was summarized descriptively. In addition, the change from baseline to Week 3 were analyzed using Analysis of Covariance (ANCOVA) model to estimate the difference between the 2 treatment arms. The model included the change from baseline as the dependent variable, and include treatment arm, indication and baseline value as covariates to the model. A 95% confidence interval (CI) and 2-sided p-value for the null hypothesis of no difference between the two arms were provided. Least-squares (LS) means for the treatment effect was provided. In addition, LS means, 95% CI and p-value for the null hypothesis of no change from baseline, were provided for each treatment arm.

For BPI, the absolute value and change from baseline to Week 3 for the 4 questions on pain severity and 7 questions on pain interference were summarized descriptively for all visits. In addition, CI and tests within a treatment arm and between treatment arms were done using the same approach as described for PROMIS. For PCS, the absolute value and change from baseline to Week 3 for the 3 domains (helplessness, magnification, rumination) as well as their total were summarized descriptively for all visits. CI and tests within a treatment arm and between treatment arm were done using the same approach as described for PROMIS.

Graphs of mean (standard error [SE]) over time for change from baseline by treatment arm and within indication were generated for the above endpoints and using the mITT Set. For GRC, the number and proportion of subjects with an improvement (a score greater than 1) were summarized by treatment arm. Comparison between the treatment arms was conducted using Cochran-Mantel-Haenszel stratified by indication.

### I. Other Exploratory Endpoints

The endpoints considered in this section are either indication-specific or endpoints completed only by participants in the Study App arm. For the former (SDMT and FACT-Cog), changes from baseline will be summarized using descriptive statistics. The change from baseline to Week 3 within each treatment arm and a 95% CI and a 2-sided p-value for the null hypothesis of no difference between the treatment arms was provided. The CI and the p-value was based on t-distribution for 2 samples. A 95% CI for the change from baseline to Week 3 within each arm, was provided as well as a 2-sided p-value for the null hypothesis of no change from baseline using t-distribution for one sample (pre-post).

For endpoints completed only by the Study App group, the analysis was stratified by indication and overall (for MS and cancer patients combined). The analysis of those endpoints was performed using the mITT Set and will include only descriptive statistics.

### 9. Results

Applicant conducted a remote, randomized, single-blinded exploratory basket study to evaluate the feasibility and acceptability of the smartphone app-based multimodal multistable bias modification (MMBM) intervention in Multiple Sclerosis (MS) and Breast Cancer (BC). The results of the study exhibit strong evidence to support progressing MMBM in BC, especially for fatigue and mood, as well as progressing MMBM in MS, especially for anxiety and pain catastrophizing.

### I. Patient Reported Outcomes: PROMIS

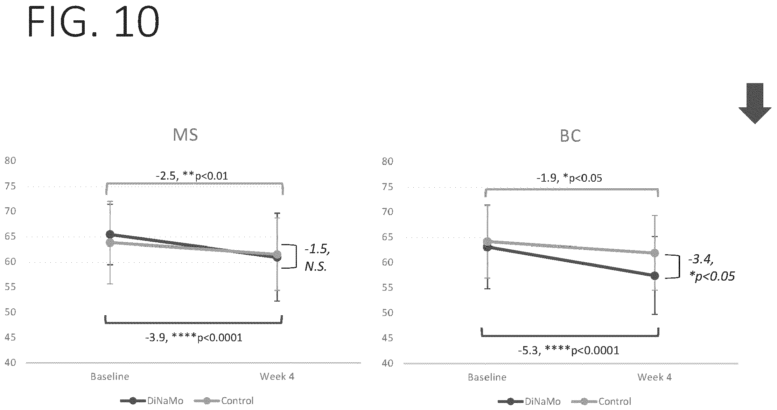

Patients were evaluated through Patient Reported Outcomes Measurement Information Systems (PROMIS). Anxiety, depression, fatigue, sleep disturbance, and pain interference improvements are indicated with lower scores. Cognitive functioning, physical functioning, and socialization improvements are indicated with higher scores. The results of patient reported outcomes recorded by PROMIS are described below and demonstrated in FIGS. 10-18.

### A. Fatigue

As demonstrated in FIG. 10, Breast cancer shows superiority of the DiNaMo over control. While both DiNaMo and control for BC improve significantly over time, only DiNaMo exceeds the minimal clinically important change threshold. The more stringent criteria of half the standard deviation (5 points) to indicate clinically important change is also met by only DiNaMo in the BC group. Both DiNaMo and control for MS improve significantly over time and exceed the minimal clinically important change threshold.

### B. Depression

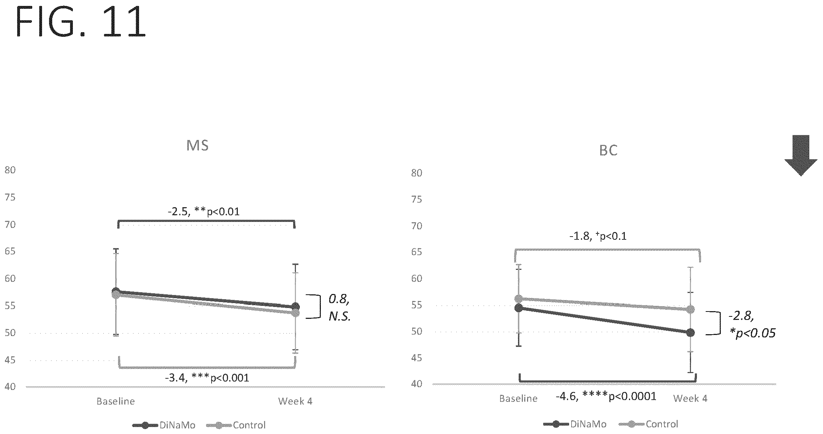

As demonstrated in FIG. 11, BC shows superiority of DiNaMo over control. This difference is above the minimal clinically important change threshold. Both DiNaMo and control for MS significantly improve over time and exceed the minimal clinically important change threshold.

### C. Anxiety

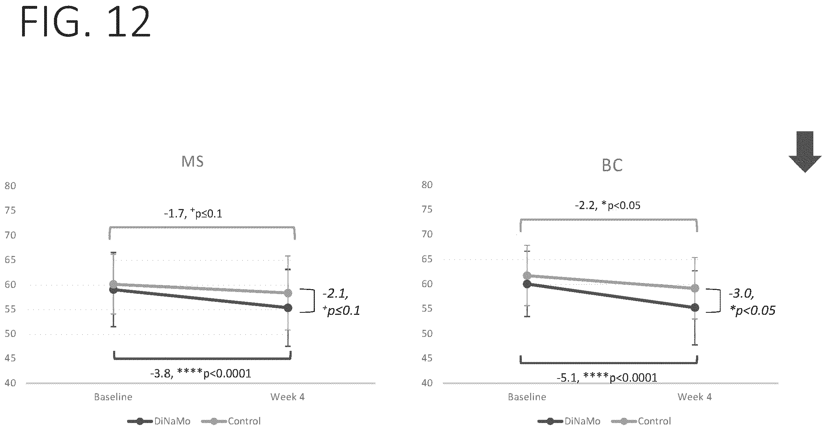

As demonstrated in FIG. 12, BC shows significant superiority of the DiNaMo over control, while MS shows trending superiority. This difference is above the minimal clinically important change threshold. Only DiNaMo for MS improves significantly over time and is above the minimal clinically important change threshold. Both DiNaMo and control for BC improves significantly over time and exceed the minimal clinically important change threshold. The more stringent criteria of half the standard deviation (5 points) to indicate clinically important change is also met by only DiNaMo in the BC group.

### D. Pain Interference

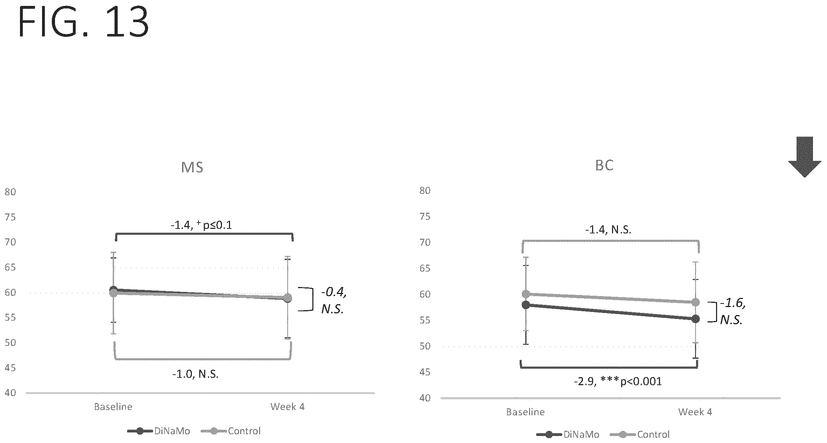

As demonstrated in FIG. 13, only DiNaMo for MS trends towards improvement over time. Only DiNaMo for BC improves significantly over time and exceeds the minimal clinically important change threshold.

### E. Pain Score

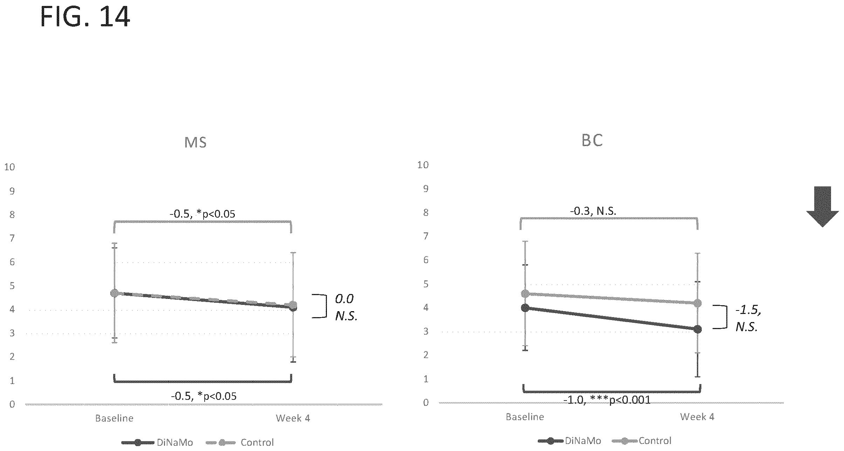

As demonstrated in FIG. 14, both DiNaMo and Control for MS significantly improve over time. Only DiNaMo for BC improves significantly over time.

### F. Sleep Disturbance

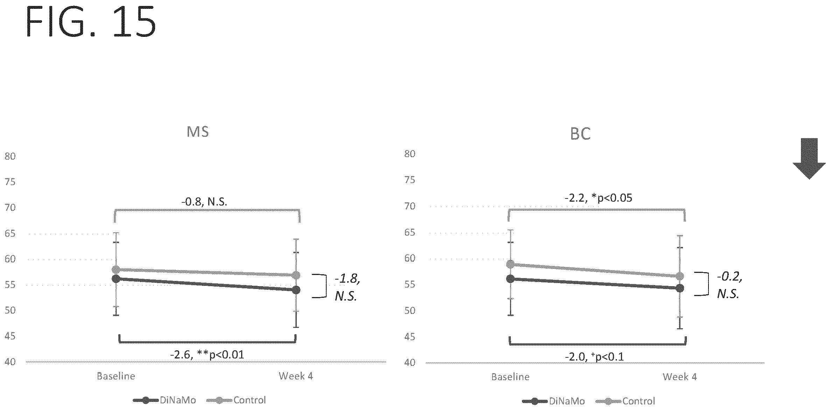

As demonstrated in FIG. 15, only DiNaMo for MS and BC significantly improves over time and exceeds the minimal clinically important difference threshold.

### G. Physical Functioning

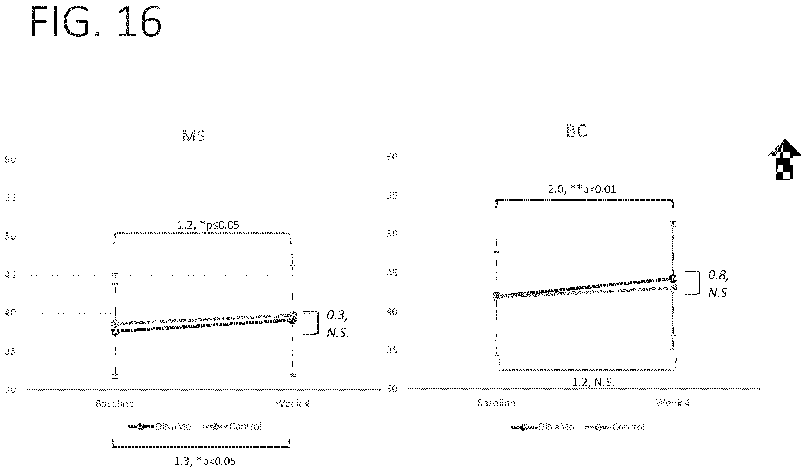

As demonstrated in FIG. 16, Both DiNaMo and Control for MS significantly improve over time. Only DiNaMo for BC significantly improves over time and exceeds the minimal clinically important change threshold.

### H. Cognitive Functioning

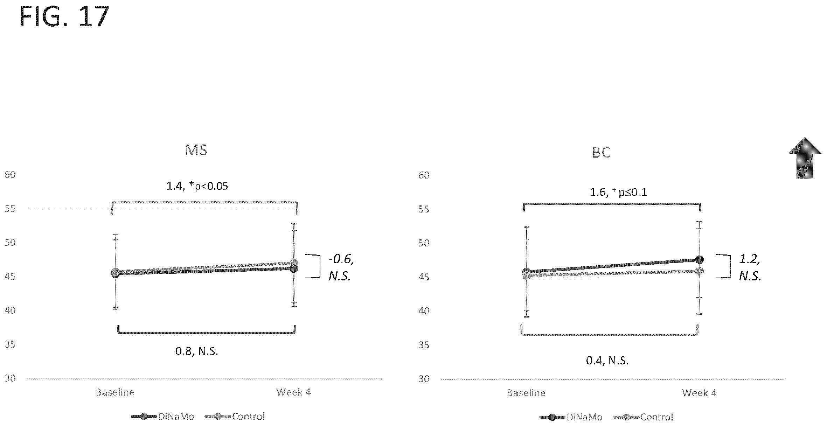

As demonstrated in FIG. 17, only DiNaMo for BC trends towards improvement over time.

### I. Social Functioning

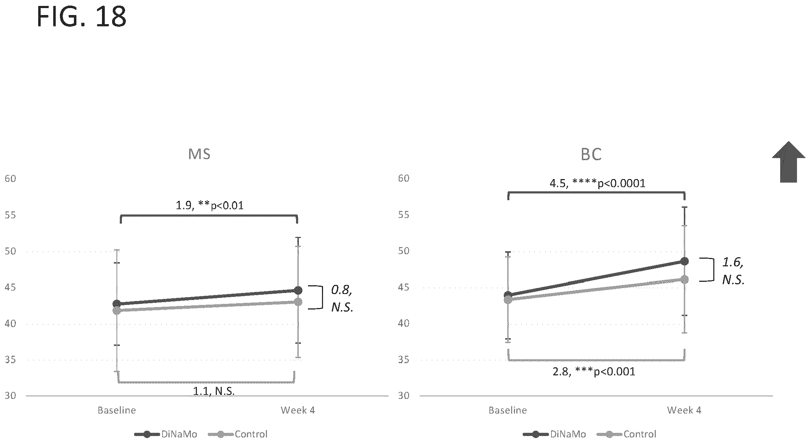

As demonstrated in FIG. 18, only DiNaMo for MS significantly improves over time. Both DiNaMo and Control for BC significantly improve over time and exceed the minimal clinically important change threshold.

### II. Patient Related Outcomes: Pain Related

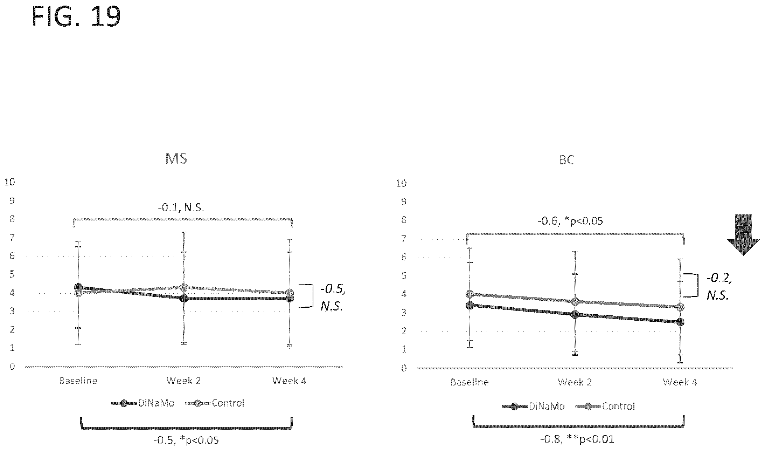

Pain-related outcomes were evaluated through patient reported outcomes. Self-reported evaluations of pain interference and pain severity for MS and BC patients are described below and demonstrated in FIGS. 19-23. Lower scores indicate improvements.

### A. Brief Pain Inventory - Interference

As demonstrated in FIG. 19, DiNaMo for MS improves over time. Both DiNaMo and control for BC improve over time.

### B. Brief Pain Inventory - Worst Pain Severity

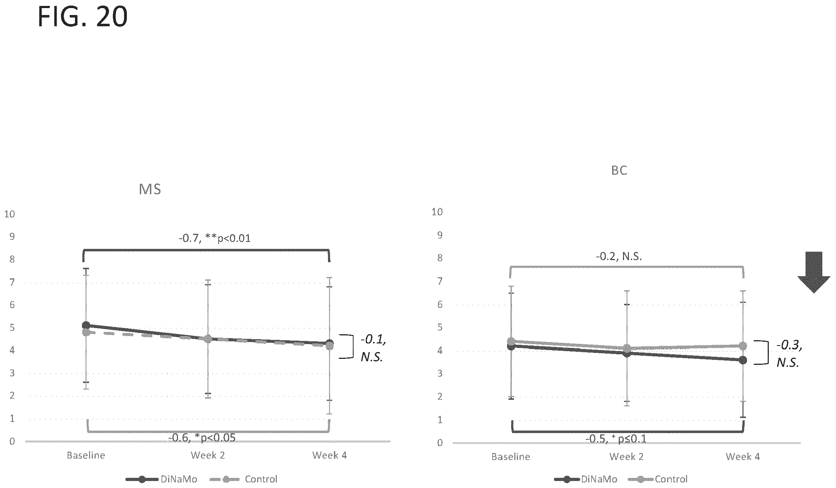

As demonstrated in FIG. 20, both DiNaMo and control for MS improve over time. DiNaMo for BC trends towards improvement over time.

### C. Brief Pain Inventory - Least Pain

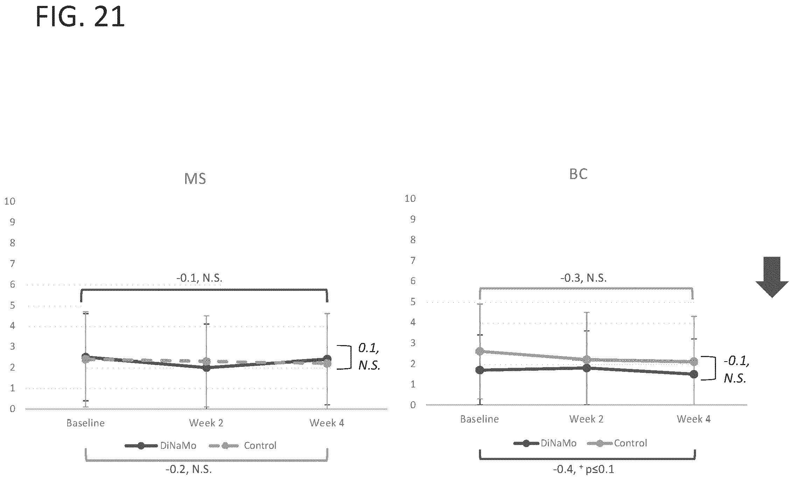

As demonstrated in FIG. 21, DiNaMo for BC trends towards improvement over time.

### D. Brief Pain Inventory - Average Pain Severity

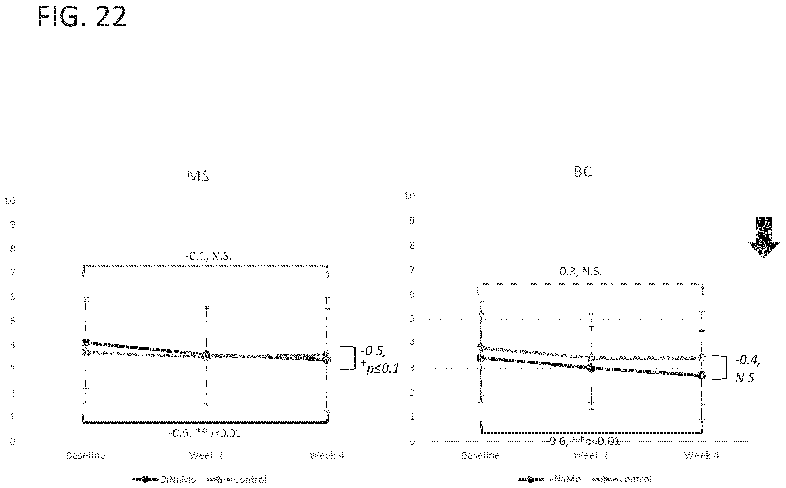

As demonstrated in FIG. 22, DiNaMo for MS trends towards demonstrating superiority over the control group. DiNaMo for MS and BC significantly improves over time.

### E. Brief Pain Inventory - Current Pain Severity

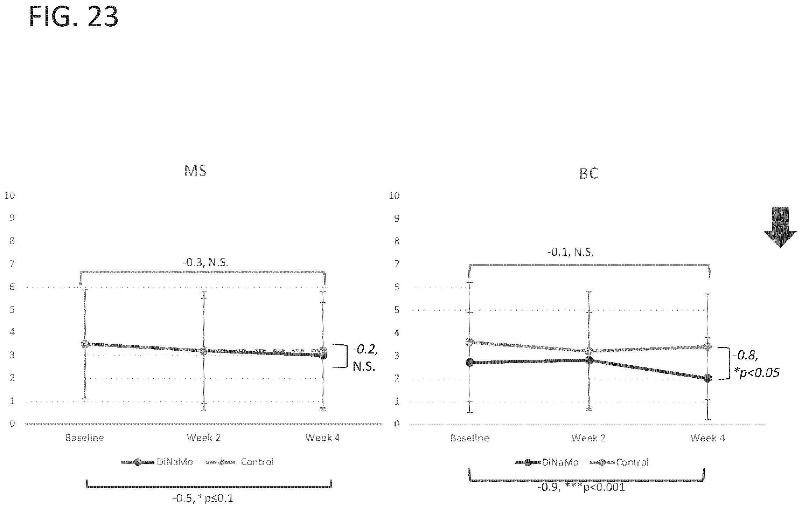

As demonstrated in FIG. 23, DiNaMo for BC demonstrates significant superiority over control. DiNaMo for MS trends towards improvement over time. DiNaMo for BC significantly improves over time.

### F. Pain Catastrophizing Scale

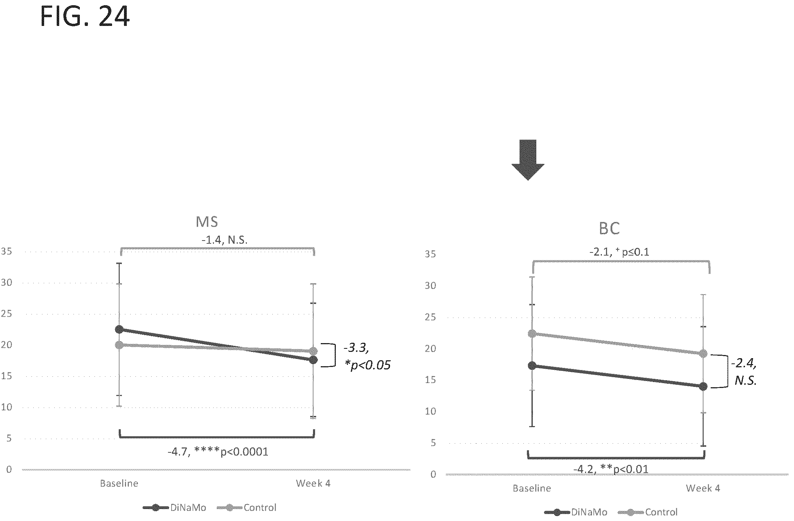

Self-reporting on pain catastrophizing scale demonstrates significant improvements for MS patients over the control group (FIG. 24). DiNaMo also demonstrated significant improvements in BC patients.

### III. Patient Reported Outcomes - Miscellaneous

### A. Cognition

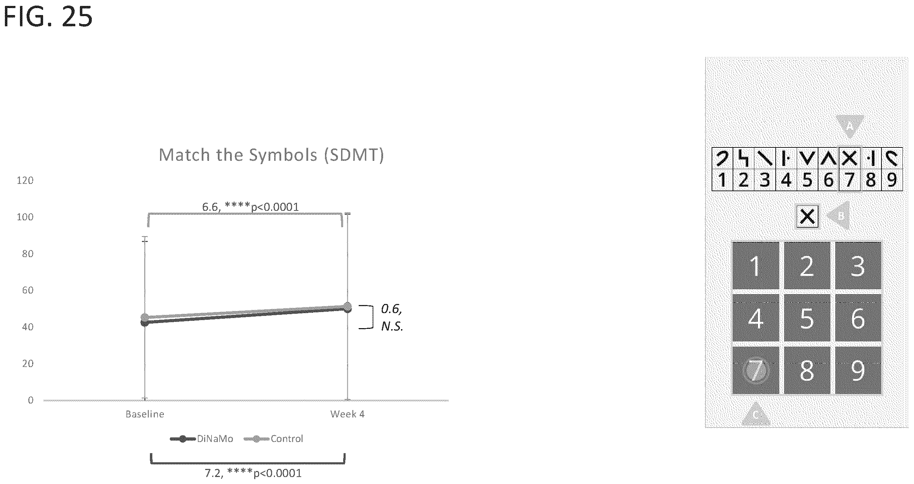

As shown in FIG. 25, cognition in MS demonstrates statistical and clinically meaningful improvement in both DiNaMo and control.

### IV. Global Rating of Change

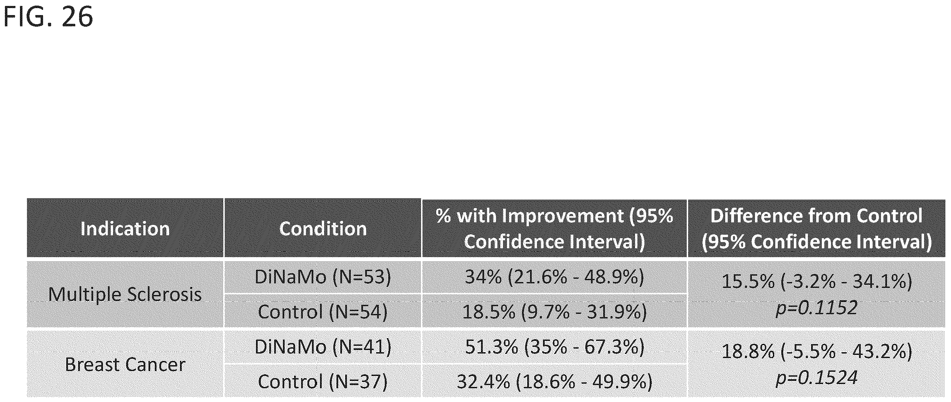

Patients were asked to describe how they felt following the study compared to the beginning of the study. The DiNaMo group tended to have a greater percentage of improvements compared with control (FIG. 26).

### V. Objective Outcomes

Patients evaluated through dot probe evaluations to measure attention bias. The goal of the attention bias index is to approach 0 (no bias) or lower.

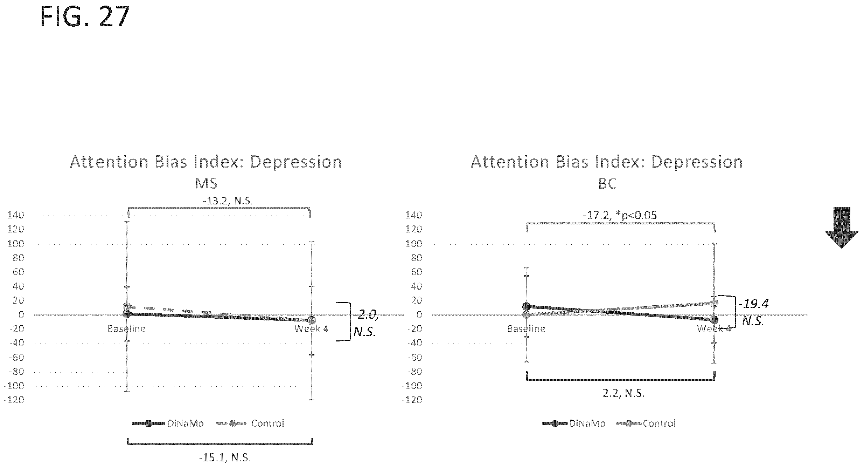

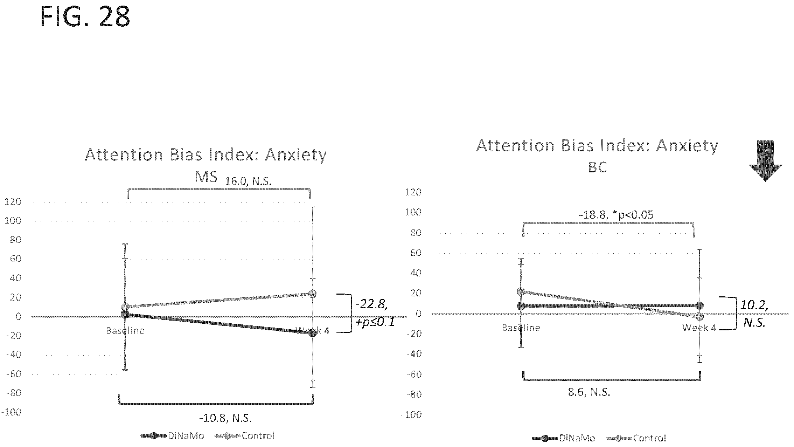

As demonstrated in FIG. 27, BC control significantly worsens in the attention bias depression idex over time, while BC DiNaMo does not. As demonstrated in FIG. 28, MS DiNaMo trends towards superiority in attention bias index for anxiety, fitting well with the superiority of MS DiNaMo over control for the PROMIS-anxiety domain (indication-specific words can trigger anxiety about one's condition).

### VI. Conclusions

The data described above relates to a remote, randomized, single-blinded exploratory basket study to evaluate the feasibility and acceptability of the smartphone app-based multimodal multistable bias modification (MMBM) intervention in Multiple Sclerosis (MS) and Breast Cancer (BC). The intervention was designed to treat fatigue, mood, and pain symptoms.

The results include strong evidence to support progressing MMBM in BC. Fatigue shows statistically and clinically significant superiority of DiNaMo in comparison to control, as measured by the PROMIS-29+2 (Patient Reported Outcomes Measurement Information System). The related PROMIS domain of cognitive functioning also shows trending improvement over time in only DiNaMo. For mood, the PROMIS domains of depression and anxiety show statistical and clinically significant superiority of DiNaMo in comparison to control (PROMIS-29+2). For pain, the PROMIS domains of pain interference and pain intensity show improvement over time in only DiNaMo. All pain severity scores in the Brief Pain Inventory also show either trending or statistically significant improvement in DiNaMo, but not control. In current pain severity score, there is also significant superiority of DiNaMo over control. In the Pain Catastrophizing Scale, only DiNaMo improves significantly over time. Finally, the related PROMIS domain of physical functioning also shows statistical and clinically significant improvement over time.

Further the results exhibit strong evidence to support progressing MMBM in MS. In the PROMIS-29+2, there is statistically trending and clinically significant superiority of DiNaMo in comparison to control group for anxiety. In the objective attention bias assessment, MS shows trending superiority of improvement in DiNaMo over control for the anxiety and indication-related words. For pain, in the Pain Catastrophizing Scale, there is statistically significant superiority of DiNaMo in comparison to control. There is also a trend towards improvement of PROMIS pain interference only in DiNaMo. In the Brief Pain Inventory, the pain interference score shows significant improvement over time in only DiNaMo. The average pain severity score shows a trend towards superiority of DiNaMo over control. Current pain severity score shows a trend for improvement in only DiNaMo.

The DiNaMo 004 intervention (MMBM) demonstrated statistically and clinically significant improvements in breast cancer (BC) patients. This suggests that the treatment's effects may vary across different diseases, with a hypothesis that can be supported by direct EEG evidence. This would further strengthen the already compelling case.

The results described herein indicate that BC (and potentially cancer in general) may be particularly responsive to the MMBM intervention. This could be due to its direct impact on functional frontoparietal connectivity, which has been linked to both chemotherapy-associated cognitive impairment and the development of cancer itself as notably, research has shown that functional brain changes before chemotherapy are a stronger predictor of post-treatment fatigue than the chemotherapy itself.

The faster response observed in breast cancer compared to multiple sclerosis (MS) may further support this hypothesis, because in MS, cognitive impairment and fatigue are primarily caused by structural damage and inflammation, with subsequent maladaptive network recruitment contributing to fatigue. In other words, MMBM in MS targets not root causes but downstream events, potentially explaining a slower or less pronounced effect compared to breast cancer. This mechanism of action (MOA) for MMBM could be further investigated through EEG recordings to assess changes in resting state functional connectivity and neural oscillations within the frontoparietal cortex and network communication.

### Example 2: A Randomized, Controlled, Exploratory Basket Study to Evaluate Multimodal Multistable Modification of Biases for the Normalization of Attentional Biases and Improving Cognition in Adults with Cancer or psychogenic chronic pain.

### Synopsis

Indications: Cancer and psychogenic chronic pain conditions

Objectives: To evaluate trends in effect in comparison to the care-as-usual (CAU) or Digital Control arm in pain interference, cognition and related outcomes (attentional bias and general quality of life [QoL]).

### Criteria for Evaluation

### Exploratory Endpoints

- Change from baseline to Week 2-8 in quality of life as measured by PROMIS-29+2 (Patient Reported Outcomes Measurement Information System)
- Change from baseline to Week 2-8 in pain severity and pain interference as measured by the Brief Pain Inventory (BPI)
- Change from baseline to Week 2-8 Pain Catastrophizing Scale (PCS)
- Change from baseline to Week 2-8 in indication-specific measures: Functional Assessment of Cancer Therapy-Cognitive Function (FACT-Cog) for cancer
- Change from baseline to Week 2-8 in computerized cognitive assessment
- Proportion of participants with an improvement as measured by the Global Rating of Change (GRC) score at Week 2-8
- Engagement with the Study App as measured by metrics such as daily App usage and daily time in the Study App
- Experience with the Study App as assessed by the user experience survey and potential optional qualitative interviews after the treatment period
- Other exploratory endpoints may include the Functional Assessment of Chronic Illness Therapy - Fatigue Scale (FACIT-Fatigue), Functional Assessment of Cancer Therapy - General (FACT-G), and/or Hospital Anxiety and Depression Scale (HADS). The FACIT-Fatigue is a 13 item scale with 5 point Likert-type scale questions. The scores are added together (two of the questions are reverse scored). To account for missing scores, they are multiplied by 13 and divided by the number of questions answered. A higher score indicates a better quality of life. The FACT-G is a 27-item questionnaire with 5 point Likert-type scales assessing health-related quality of life in cancer patients with physical, social, emotional, and functional subscales. Values are added together, with some being reverse scored (all of the social and functional subscales, as well as one of the emotional subscale questions). All subscales are added together to get the total FACT-G score, where a higher score means a better quality of life. HADS is a 14-item questionnaire used to assess depression and anxiety, where a higher score for each subdomain means higher symptom severity.

### Study Design

Referring now to FIG. 8, depicted is a timeline of the randomized, controlled, exploratory basket study to evaluate multimodal multistable modification of biases for the normalization of attentional biases and improving cognition in adults with cancer or psychogenic chronic pain conditions.

### Screening Period (Day -14 to Day -1)

- Participants will be screened for up to 14 days, including electronic informed consent. Assessments during this period will be performed remotely according to the Schedule of Activities and Assessments (SoA).
- Participants will be considered eligible if they meet all inclusion criteria and no exclusion criteria, based on investigator assessment.
- Screening and the Baseline Visit can occur on the same day.

### Baseline (Day 1)

- Participants will be contacted for a Baseline Visit to review and confirm eligibility and perform identity verification. Assessments will be performed according to the SoA.
- Eligible participants will be randomized during a virtual study visit on Day 1. Participants will be randomized 1:1 (Study App:Control) within the cancer and psychogenic chronic pain condition cohorts. Participants randomized to the Study App treatment group will download and activate the app onto their personal primary smart phone.

Intervention Period
- Study App group participants will utilize an app-based daily brain exercise for 1 minute to 30 minutes at least 3 times a week for 2-8 weeks.
- Control group participants will receive care-as-usual (CAU) or a Digital Control and will not receive any study intervention.
- Assessments will occur every week throughout the intervention period for both groups according to the SoA.
- Participants assigned to the Study App group will complete the user experience questionnaire at the end of the Intervention Period.

### Follow-up Period (Up to 1-8 Weeks)

- Participants will complete follow-up assessments according to the SoA.
- Participants will not perform any activities within the Study App.
- A subset of participants will complete an optional qualitative interview between Day 22 and Day 28.

### Planned Number of Participants: At least 40 total participants will be enrolled in the study:

### Study Entry Criteria

Inclusion Criteria: A participant will be eligible for entry into the study if all of the following criteria are met:
1. Fluent in written and spoken English, confirmed by ability to read, understand, and sign the informed consent form
2. Lives in the United States
3. Adult between 22 and 65 years old
4. Meets indication-specific including criteria, as reported by the study participant with adequate clinical documentation (to be provided to the study team upon request)
5. Has an active email address and is willing and able to receive and respond to email messages
6. Has access to an internet connection during the study duration
7. Has an active bank account to receive study compensation, or is willing to create one
8. Willing and able to comply with study protocol and assessments
9. Is the sole user of a smart phone for the duration of the study
10. Is willing and able to receive Short Message Service (SMS) text messages and notifications on their smartphone
11. Has access to operating headphones

Exclusion Criteria: A participant will not be eligible for study entry if any of the following criteria are met:
1. Visual, dexterity or cognitive deficit so severe that it precludes the use of an App-based reaction time-based activity per investigator judgment
2. Severe psychiatric disorder involving a history of psychosis (e.g., schizophrenia, bipolar disorder, severe personality disorders)
3. Psychiatric hospitalization in the past 6 months
4. Participation in any research study (including studies on psychotherapy, mindfulness, cognitive training or pharmacological treatment) during the past 3 months
5. Initiation or change in primary disease-specific medication within 30 days prior to entering the study
6. Planning the introduction of new therapies (including studies on psychotherapy, mindfulness, cognitive training or pharmacological treatment) during the study duration (1-12 weeks)
7. Anticipating a change in current pharmacological or psychotherapy treatment regimen during the study period (1-12 weeks)
8. Self-reported substance use disorder within the past 1 year
9. Severe neurological disorders impairing brain function (e.g., previous stroke, dementia, primary brain function, brain metastases, Alzheimer's disease, Parkinson's disease, history of significant head trauma followed by persistent neurologic deficits or known structural brain abnormalities)
10. Mild, moderate, severe or profound unilateral or bilateral hearing loss

Test Product and Mode of Administration DiNaMo 004

Eligible participants will download and install the Study App onto their own smartphone at the Baseline Visit (Day 1).

### Treatment Regimen: Study App: at least 3 times a week use for at least 2 weeks (1 minute to 30 minutes per day)

Study Duration: Participation in the study will last for up to 6 weeks:
- Screening Period: Up to 2 weeks
- Intervention Period: 2-8 weeks
- Follow-up: 1-3 weeks

Sample Size: There will be at least 40 cancer subjects and/or 40 psychogenic chronic pain conditions subjects. These sample sizes should be sufficient to measure the changes with relative precision.

Statistical Analysis: Change from baseline to Week 3 for all the PROMIS-29+2 domains, BPI pain severity and BPI pain interference, PCS subcategories and total score, as well as the computerized cognitive assessment, will be summarized using descriptive statistics. An Analysis of Covariance (ANCOVA) model will be implemented to assess the difference between the 2 treatment arms. The model will include the change from baseline as the dependent variable, and include treatment arm, indication and baseline value as covariates in the model. A 95% confidence interval (CI) and 2-sided p-value for the null hypothesis of no difference between the 2 arms will be provided. Least-squares (LS) means for the treatment effect will be provided. In addition, LS means, 95% CI and p-value for the null hypothesis of no change from baseline, will be provided for each treatment arm.

For the SDMT and FACT-Cog, which are indication specific, changes from baseline will be summarized using descriptive statistics. In addition, for the change from baseline to Week 3 within each treatment arm, a 95% CI and a 2-sided p-value for the null hypothesis of no difference between the treatment arms will be provided. The CI and the p-value will be based on t-distribution for 2 samples.

A 95% CI for the change from baseline to Week 3 within each arm will be provided, as well as a 2-sided p-value for the null hypothesis of no change from baseline using t-distribution for one sample (pre-post).

The number and percentage with an improvement as measured in the GRC (improvement is a score greater than 1) will be summarized by treatment arm. Comparison between the treatment arms will be conducted using Cochran-Mantel-Haenszel stratified by indication.

For the remaining exploratory endpoints as well as for the safety parameters, only descriptive statistics will be used.

### 2. Individual Indications - Cancer and psychogenic chronic pain conditions

### Criteria for Evaluation

Additional disease-specific scales:
- Functional Assessment of Cancer Therapy-Cognitive Function (FACT-Cog)

### Study Entry Criteria Inclusion Criteria

1. Has a history of cancer (any stage) as reported by study participant with adequate clinical documentation (to be provided to study team upon request)
2. Has completed cancer chemotherapy treatment for at least 3 months, within the 9 months to 5 years prior to screening
3. Self-reported pain for at least the last 3 months, which started following cancer therapy

### Exclusion Criteria

1. Self-reported history of clinical diagnosis of multiple sclerosis or breast cancer.

### 3. Schedule of Activities and Assessments

The schedule of activities may be found as described in example 1.

### 4. Introduction

### A. Multimodal Multistable Modification of Biases

Multimodal Multistable Modification of Biases (multimodal, multistable bias modification or MMBM) will be utilized as described above in Example 1.

### B. Attentional Biases

Individuals with a variety of indications, such as various types of cancer, tend to present with problems in both pain and mood. These problems can be exacerbated by attentional biases related to pain- or threat-related stimuli. Individuals with chronic pain may be more attentive to pain-related stimuli, which can worsen the condition via fear-avoidance. Individuals may avoid stimuli or activities that are perceived to potentially cause pain, which can decondition the body and lead to further disability exacerbating the condition. With threat-related stimuli, individuals with anxiety often automatically seek out any signs of threat. This threat causes a feedback loop that perpetuates feelings of anxiety. This can include indication-related stimuli: for example, cancer survivors with a fear of cancer recurrence can be biased towards cancer-related stimuli. Likewise, as pain and anxiety are highly comorbid and share similar neurocircuitry alterations, targeting biases related to both are likely to have a synergistic effect. Finally, individuals with depression often display an attentional bias towards negative emotional information, which can contribute to rumination and thereby exacerbate symptoms. Together, these various attentional biases compounded can become quite debilitating.

Individuals with cancer and cancer survivors both demonstrate an attentional bias for cancer-related words. Studies have also found evidence of biases towards threat and negative emotions. Attempts to change these automatic associations have demonstrated some success. As cancer patients also experience pain and mood symptoms, Multimodal Multistable Modification of Biases is expected to be highly relevant for this patient population.

### C. Cognitive Impairment

Cognitive impairments are common in several physical and mental disorders, either due to primary symptoms associated with a disease/disorder or associated with treatment for specific diseases (e.g., chemotherapy in cancer) or both. Cognitive impairments are also commonly related to problems in emotion regulation and mood problems. The combination of cognitive deficits and mood symptoms is distressing and disabling for patients, can negatively affect their quality of life and can result in increased health-care utilization.

In cancer patients, tumor activity and related inflammatory processes and/or cancer-related treatments can impact cognitive functioning. Cancer-related cognitive impairment occurs in up to 75% of cancer patients, and for up to 35% of those experiencing impairment, this can last for months or years post-treatment. With the rising numbers of cancer survivors, lasting cognitive impairments due to cancer and cancer treatment will further increase.

Fatigue is also a debilitating symptom to both indications and can heavily contribute to problems with cognition. Fatigue is one of the most common cancer symptoms, also often being the presenting symptom, and this can result both from treatment, such as chemotherapy (with a prevalence of 75-100%), or from the disease itself (especially in the final stages). One study even found that 37% of cancer survivors still did not have their energy return to a satisfying level, even years later.

### D. Binaural Beats

In binaural beats, a single tone frequency is played in each ear, and the frequency difference between the two that can be detected is called the "beat". The theory behind binaural beats is that rhythmic sensory stimulation could entrain neural oscillations, similar to noninvasive techniques like transcranial magnetic stimulation and transcranial direct current stimulation. Studies have found beneficial effects of binaural beats on depression and anxiety symptoms, memory, attention, and cognition, fatigue and pain perception.

### E. The Study Application (App)

The Multimodal Multistable Modification of Biases CT-100 Digital Neuro-activation and Modulation (DiNaMo) component (e.g., the application 125) uses implicit training to redirect and strengthen attention processes. This intervention can help improve cognitive capabilities as well as redirect attentional biases to make participants less sensitive to distressing stimuli. Preliminary studies have indicated success in computerized cognitive training of cancer, as well as success in retraining attentional biases in pain, threat, and mood. It is likely that Multimodal Multistable Modification of Biases may improve both cognitive capabilities as well as attentional biases present in cancer.

### F. CAU or Digital Control Control

The care-as-usual (CAU) control group may only complete assessments and will not use the Study App. A Digital Control App group will download the Digital Control App. They will complete a version of the task designed to be inert in the Study Application. This will include only selecting between a set of neutral words, based on personal preference, and will not include any attention-bias-capturing stimuli. Additionally, all instances of multistable perception will be removed, including the removal of all auditory elements.

### G. Study Rationale

Multimodal Multistable Modification of Biases is a novel intervention using Multimodal NeuroEnrichment Task (M-NET) principles in order to reduce symptoms relevant to cancer. Attentional biases to a variety of attention-capturing stimuli can exacerbate a variety of symptoms in many different indications. For example, in conditions, patients are more attentive to pain-related stimuli, which can lead to hypersensitization. Similarly, hypersensitivity to threat-related stimuli can exacerbate anxiety.

In Multimodal Multistable Modification of Biases, users are trained to re-orient attention towards positive stimuli, instead of stimuli that may cause them distress, such as pain-or threat-related stimuli. This is done in a novel format, tapping into multistable perception. Visual multistable perception, in the form of monocular rivalry, is used to implicitly retrain attentional biases. At the same time, auditory multistable perception, in the form of binaural beats, will be used to both target fatigue symptoms and to work synergistically to enhance the attentional bias component.

The purpose of the proposed study is to evaluate initial effects of the DiNaMo component (the Study App) on measures of attentional bias, attentional impairment and related outcomes (general quality of life [QoL]) in a variety of conditions, such as cancer. Results derived from this research could be used as components within future digital therapeutics.

### H. CT-100 Platform

The CT-100 platform provides interactive, software based therapeutic components that may be used as part of a multimodal treatment in future software-based prescription digital therapeutics. One class of CT-100 components are DiNaMo components. DiNaMo components target key neural systems (including but not limited to systems related to sensory-, perceptual-, affective-, pain-, attention-, cognitive control, social- and self-processing) to optimally improve a participant's health.

### 3. Objectives And Endpoints

The study objectives are:
- To evaluate trends in effect in comparison to the care-as-usual (CAU) or Digital Control arm in pain interference, cognition, and related outcomes (attentional bias and general QoL)
- To explore feasibility of an at-home digital Multimodal Multistable Modification of Biases visual search task (Study App), including engagement and experience with the Study App in participants
- To evaluate the safety of the Study App

Study Endpoints correspond to the endpoints provided in Example 1.

### 4. Study Design

### A. Scientific Rationale for Study Design

This study is designed to evaluate the initial effects of the Multimodal Multistable Modification of Biases CT-100 component (the Study App) on attentional biases and cognitive deficits in attention as compared to CAU or the Digital Control.

Participants will be assessed based on validated standard participant-rated outcomes. Participant engagement with the Study App will be evaluated based on participant usage data captured within the Study App. Participants will also be evaluated for safety throughout the duration of the study. The scales and assessments are described herein.

### B. End of Study Definition

The end of the study is defined as the date of the last contact, or the date of final contact attempt, for the last participant completing or withdrawing from the study. For the purposes of this study, participants who complete the trial assessments at Day 21 (+3) (Week 3) will be defined as trial completers.

### 6. Study Intervention(s) and Concomitant Therapy

### A. Study Intervention(s) Administered

The CT-100-D-004 Study App (e.g., the application 125) will administer participants with the study intervention.

### B. CT-100

The study intervention under evaluation is the Multimodal Multistable Modification of Biases CT-100 component, a digital mobile application. Participants randomized to this group will download and install the Study App onto their own smartphone at the Baseline Visit (Day 1) and use the Study App daily for Multimodal Multistable Modification of Biases training over the 2-8-week intervention period.

### C. Control

Participants randomized to the CAU or Digital Control group will complete study assessments only over the 2-8-week intervention period. The Digital Control group will download the Digital Control App. They will complete a version of the task designed to be inert in the Study Application. This will include only selecting between a set of neutral words, based on personal preference, and will not include any attention-bias-capturing stimuli. Additionally, all instances of multistable perception will be removed, including the removal of all auditory elements.

### D. Preparation/Handling/Storage/Accountability

Detailed instructions for the download, installation, activation, use of, and disposition of the Study App is described in the CT-100-D-004 Study App Site Instructions.

Generally:
1. Only participants enrolled in the study may receive study intervention.
2. The site personnel must confirm download and activation of the Study App.
3. The Study App will automatically become inert after the completion of Week 2-8. Site personnel will inform participants who complete the study or early terminate to uninstall the Study App.

### E. Concomitant Therapy

Participants will continue to use their prescribed therapies while enrolled in this study. Participants will self-report any changes to all concomitant medications that are related to the primary indication or are psychoactive through the end of the Follow-up Period.

### 7. Study Assessments and Procedures

Study assessments and procedures, including their timing, are summarized in the SoA and above for Example 1.

### 8. Statistical Considerations

Statistical considerations correspond to the considerations provided above with respect to Example 1.

### 9. Conclusions

Based on the results of Example 1, it is expected that individuals receiving the treatment will show an improvement in at least one-indication-related symptom as measured by the performance measure provided herein. The improvement in the at least one indication-related symptom in individuals receiving the application is expected to be statistically significant relative to any improvement observed in the corresponding indication-related symptom in individuals receiving the Digital Control or CAU.

### C. Network and Computing Environment

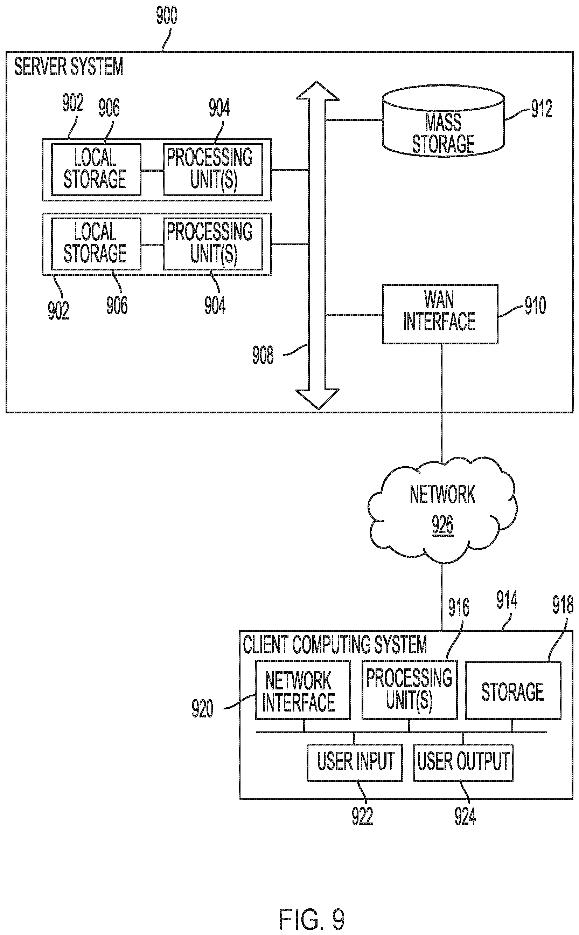

Various operations described herein can be implemented on computer systems. FIG. 9 shows a simplified block diagram of a representative server system 900, client computer system 914, and network 926 usable to implement certain embodiments of the present disclosure. In various embodiments, server system 900 or similar systems can implement services or servers described herein or portions thereof. Client computer system 914 or similar systems can implement clients described herein. The system 100 described herein can be similar to the server system 900. Server system 900 can have a modular design that incorporates a number of modules 902 (e.g., blades in a blade server embodiment); while two modules 902 are shown, any number can be provided. Each module 902 can include processing unit(s) 904 and local storage 906.

Processing unit(s) 904 can include a single processor, which can have one or more cores, or multiple processors. In some embodiments, processing unit(s) 904 can include a general-purpose primary processor as well as one or more special-purpose co-processors such as graphics processors, digital signal processors, or the like. In some embodiments, some or all processing units 904 can be implemented using customized circuits, such as application specific integrated circuits (ASICs) or field programmable gate arrays (FPGAs). In some embodiments, such integrated circuits execute instructions that are stored on the circuit itself In other embodiments, processing unit(s) 904 can execute instructions stored in local storage 906. Any type of processors in any combination can be included in processing unit(s) 904.

Local storage 906 can include volatile storage media (e.g., DRAM, SRAM, SDRAM, or the like) and/or non-volatile storage media (e.g., magnetic or optical disk, flash memory, or the like). Storage media incorporated in local storage 906 can be fixed, removable, or upgradeable as desired. Local storage 906 can be physically or logically divided into various subunits such as a system memory, a read-only memory (ROM), and a permanent storage device. The system memory can be a read-and-write memory device or a volatile read-and-write memory, such as dynamic random-access memory. The system memory can store some or all of the instructions and data that processing unit(s) 904 need at runtime. The ROM can store static data and instructions that are needed by processing unit(s) 904. The permanent storage device can be a non-volatile read-and-write memory device that can store instructions and data even when module 902 is powered down. The term "storage medium" as used herein includes any medium in which data can be stored indefinitely (subject to overwriting, electrical disturbance, power loss, or the like) and does not include carrier waves and transitory electronic signals propagating wirelessly or over wired connections.

In some embodiments, local storage 906 can store one or more software programs to be executed by processing unit(s) 904, such as an operating system and/or programs implementing various server functions such as functions of the system 100 or any other system described herein, or any other server(s) associated with system 100 or any other system described herein.

"Software" refers generally to sequences of instructions that, when executed by processing unit(s) 904, cause server system 900 (or portions thereof) to perform various operations, thus defining one or more specific machine embodiments that execute and perform the operations of the software programs. The instructions can be stored as firmware residing in read-only memory and/or program code stored in non-volatile storage media that can be read into volatile working memory for execution by processing unit(s) 904. Software can be implemented as a single program or a collection of separate programs or program modules that interact as desired. From local storage 906 (or non-local storage described below), processing unit(s) 904 can retrieve program instructions to execute and data to process in order to execute various operations described above.

In some server systems 900, multiple modules 902 can be interconnected via a bus or other interconnect 908, forming a local area network that supports communication between modules 902 and other components of server system 900. Interconnect 908 can be implemented using various technologies, including server racks, hubs, routers, etc.

A wide area network (WAN) interface 910 can provide data communication capability between the local area network (e.g., through the interconnect 908) and the network 926, such as the Internet. Other technologies can be used to communicatively couple the server system with the network 926, including wired (e.g., Ethernet, IEEE 802.3 standards) and/or wireless technologies (e.g., Wi-Fi, IEEE 802.11 standards).

In some embodiments, local storage 906 is intended to provide working memory for processing unit(s) 904, providing fast access to programs and/or data to be processed while reducing traffic on interconnect 908. Storage for larger quantities of data can be provided on the local area network by one or more mass storage subsystems 912 that can be connected to interconnect 908. Mass storage subsystem 912 can be based on magnetic, optical, semiconductor, or other data storage media. Direct attached storage, storage area networks, network-attached storage, and the like can be used. Any data stores or other collections of data described herein as being produced, consumed, or maintained by a service or server can be stored in mass storage subsystem 912. In some embodiments, additional data storage resources may be accessible via WAN interface 910 (potentially with increased latency).

Server system 900 can operate in response to requests received via WAN interface 910. For example, one of modules 902 can implement a supervisory function and assign discrete tasks to other modules 902 in response to received requests. Work allocation techniques can be used. As requests are processed, results can be returned to the requester via WAN interface 910. Such operation can generally be automated. Further, in some embodiments, WAN interface 910 can connect multiple server systems 900 to each other, providing scalable systems capable of managing high volumes of activity. Other techniques for managing server systems and server farms (collections of server systems that cooperate) can be used, including dynamic resource allocation and reallocation.

Server system 900 can interact with various user-owned or user-operated devices via a wide-area network such as the Internet. An example of a user-operated device is shown in FIG. 9 as client computing system 914. Client computing system 914 can be implemented, for example, as a consumer device such as a smartphone, other mobile phone, tablet computer, wearable computing device (e.g., smart watch, eyeglasses), desktop computer, laptop computer, and so on.

For example, client computing system 914 can communicate via WAN interface 910. Client computing system 914 can include computer components such as processing unit(s) 916, storage device 918, network interface 920, user input device 922, and user output device 924. Client computing system 914 can be a computing device implemented in a variety of form factors, such as a desktop computer, laptop computer, tablet computer, smartphone, other mobile computing device, wearable computing device, or the like.

Processing unit 916 and storage device 918 can be similar to processing unit(s) 904 and local storage 906 described above. Suitable devices can be selected based on the demands to be placed on client computing system 914. For example, client computing system 914 can be implemented as a "thin" client with limited processing capability or as a high-powered computing device. Client computing system 914 can be provisioned with program code executable by processing unit(s) 916 to enable various interactions with server system 900.

Network interface 920 can provide a connection to the network 926, such as a wide area network (e.g., the Internet) to which WAN interface 910 of server system 900 is also connected. In various embodiments, network interface 920 can include a wired interface (e.g., Ethernet) and/or a wireless interface implementing various RF data communication standards such as Wi-Fi, Bluetooth, or cellular data network standards (e.g., 3G, 4G, LTE, etc.).

User input device 922 can include any device (or devices) via which a user can provide signals to client computing system 914; client computing system 914 can interpret the signals as indicative of particular user requests or information. In various embodiments, user input device 922 can include any or all of a keyboard, touch pad, touch screen, mouse or other pointing device, scroll wheel, click wheel, dial, button, switch, keypad, microphone, and so on.

User output device 924 can include any device via which client computing system 914 can provide information to a user. For example, user output device 924 can include display-to-display images generated by or delivered to client computing system 914. The display can incorporate various image generation technologies, e.g., a liquid crystal display (LCD), light-emitting diode (LED) display including organic light-emitting diodes (OLED), projection system, cathode ray tube (CRT), or the like, together with supporting electronics (e.g., digital-to-analog or analog-to-digital converters, signal processors, or the like). Some embodiments can include a device such as a touchscreen that function as both input and output device. In some embodiments, other user output devices 924 can be provided in addition to or instead of a display. Examples include indicator lights, speakers, tactile "display" devices, printers, and so on.

Some embodiments include electronic components, such as microprocessors, storage, and memory that store computer program instructions in a computer readable storage medium. Many of the features described in this specification can be implemented as processes that are specified as a set of program instructions encoded on a computer readable storage medium. When these program instructions are executed by one or more processing units, they cause the processing unit(s) to perform various operations indicated in the program instructions. Examples of program instructions or computer code include machine code, such as is produced by a compiler, and files including higher-level code that are executed by a computer, an electronic component, or a microprocessor using an interpreter. Through suitable programming, processing unit(s) 904 and 916 can provide various functionality for server system 900 and client computing system 914, including any of the functionality described herein as being performed by a server or client, or other functionality.

It will be appreciated that server system 900 and client computing system 914 are illustrative and that variations and modifications are possible. Computer systems used in connection with embodiments of the present disclosure can have other capabilities not specifically described here. Further, while server system 900 and client computing system 914 are described with reference to particular blocks, it is to be understood that these blocks are defined for convenience of description and are not intended to imply a particular physical arrangement of component parts. For instance, different blocks can be but need not be located in the same facility, in the same server rack, or on the same motherboard. Further, the blocks need not correspond to physically distinct components. Blocks can be configured to perform various operations, e.g., by programming a processor or providing appropriate control circuitry, and various blocks might or might not be reconfigurable depending on how the initial configuration is obtained. Embodiments of the present disclosure can be realized in a variety of apparatus including electronic devices implemented using any combination of circuitry and software.

While the disclosure has been described with respect to specific embodiments, one skilled in the art will recognize that numerous modifications are possible. Embodiments of the disclosure can be realized using a variety of computer systems and communication technologies, including but not limited to specific examples described herein. Embodiments of the present disclosure can be realized using any combination of dedicated components and/or programmable processors and/or other programmable devices. The various processes described herein can be implemented on the same processor or different processors in any combination. Where components are described as being configured to perform certain operations, such configuration can be accomplished, e.g., by designing electronic circuits to perform the operation, by programming programmable electronic circuits (such as microprocessors) to perform the operation, or any combination thereof. Further, while the embodiments described above may make reference to specific hardware and software components, those skilled in the art will appreciate that different combinations of hardware and/or software components may also be used and that particular operations described as being implemented in hardware might also be implemented in software or vice versa.

Computer programs incorporating various features of the present disclosure may be encoded and stored on various computer readable storage media; suitable media include magnetic disk or tape, optical storage media such as compact disk (CD) or digital versatile disk (DVD), flash memory, and other non-transitory media. Computer readable media encoded with the program code may be packaged with a compatible electronic device, or the program code may be provided separately from electronic devices (e.g., via Internet download or as a separately packaged computer-readable storage medium).

Thus, although the disclosure has been described with respect to specific embodiments, it will be appreciated that the disclosure is intended to cover all modifications and equivalents within the scope of the following claims.

## Claims

1. A method of presenting multimodal stimuli to address symptoms associated with conditions, comprising:
identifying, by a computing system, for addressing a condition of a user, (i) a first visual stimulus associated with the condition and (ii) a second visual stimulus;
presenting, by the computing system, via a display, the first visual stimulus at least partially overlapped with the second visual stimulus, to direct the user to interact with the display;
detecting, by the computing system, a response identifying an interaction associated with one of the first visual stimulus or the second visual stimulus;
generating, by the computing system, an auditory stimulus to include one or more portions indicating feedback based on the response; and
playing, by the computing system, via a transducer, the auditory stimulus to provide the feedback on the response to the user.

2. The method of claim 1, further comprising determining, by the computing system, that the response is correct based on the interaction associated with the second visual stimulus to disassociate the user from the condition, and
wherein generating the auditory stimulus further comprises adding at least one portion to the auditory stimulus to provide positive reinforcement, responsive to determining that the response is correct.

3. The method of claim 1, further comprising determining, by the computing system, that the response is incorrect based on the interaction associated with the first visual stimulus associated with the condition, and
wherein generating the auditory stimulus further comprises removing at least one portion from the auditory stimulus to provide negative punishment, responsive to determining that the response is incorrect.

4. The method of any preceding claim, further comprising assigning, by the computing system, (i) the first visual stimulus to a first type of interaction and (ii) the second visual stimulus to a second type of interaction, and
wherein presenting via the display further comprises presenting (i) the first visual stimulus with a first indicator to identify the first type of interaction and (ii) the second visual stimulus with a second indicator to identify the second type of interaction.

5. The method of any preceding claim, further comprising:
selecting, by the computing system, based on a rate of correct responses from previous presentations of visual stimuli, a difficulty level from a plurality of difficulty levels, each of the plurality of difficulty levels defining one or more visual characteristics; and
modifying, by the computing system, a presentation of the first visual stimulus and the second visual stimulus using the one or more visual characteristics in accordance with the difficulty level.

6. The method of any preceding claim, wherein generating the auditory stimulus further comprises generating the auditory stimulus to include (i) a first element to be played to a first ear of the user and (ii) a second element to be played to a second ear of the user, and
wherein playing via the transducer further comprises providing the first element to the first ear of the user via a first loudspeaker and the second element to the second ear via a second loudspeaker.

7. The method of any preceding claim, wherein the interaction comprises: (i) an orientation of the display toward a side corresponding to one of a first side associated with the first visual stimulus or a second side associated with the second visual stimulus.

8. The method of any preceding claim, further comprising modifying, by the computing system, a visual characteristic of one of the first visual stimulus or the second visual stimulus based on the response.

9. The method of any preceding claim, wherein playing the auditory stimulus further comprises playing at one portion of the one or more portions of the auditory stimulus, at least in partial concurrence with the presentation of the first visual stimulus and the second visual stimulus.

10. The method of any preceding claim, further comprising receiving, by the computing system, via an interface, a selection by the user of at least one property from a plurality of properties for auditory stimuli, and
wherein generating the auditory stimulus further comprises generating the auditory stimulus in accordance with the at least one property.

11. The method of any preceding claim, further comprising receiving, by the computing system, via an interface, a list identifying a plurality of words comprising a first subset of words related to the condition and a second subset of words; and
wherein identifying for addressing the condition further comprises selecting the first visual stimulus from the first subset of words and the second visual stimulus from the second subset of words.

12. The method of any preceding claim, wherein the user is on a medication to address the condition associated with at least one of cancer or multiple sclerosis, at least in partial concurrence with the session.

13. A computer-readable storage medium comprising computer-readable instructions that, when executed by one or more processors, cause the one or more processors to perform the method of any preceding claim.

14. A system for presenting multimodal stimuli to reduce symptoms associated with conditions, comprising:
a computing system having one or more processors coupled with memory, configured to perform the method of any of claims 1 to 12.
